Europäisches Patentamt

European Patent Office

Office européen des brevets

(19)

(11) **EP 1 707 209 A1**

(12) **EUROPEAN PATENT APPLICATION**
published in accordance with Art. 158(3) EPC

(43) Date of publication:
04.10.2006 Bulletin 2006/40

(21) Application number: 04820043.0

(22) Date of filing: 29.11.2004

(51) Int Cl.:
*A61K 35/36* (2006.01)     *A61K 9/20* (2006.01)
*A61K 9/48* (2006.01)     *A61P 35/00* (2006.01)

(86) International application number:
**PCT/CN2004/001367**

(87) International publication number:
**WO 2005/056029 (23.06.2005 Gazette 2005/25)**

(84) Designated Contracting States:
**CH DE FR GB IT LI**

(30) Priority: **10.12.2003  CN 200310109213**

(71) Applicant: **Shanghai Baike Pharmaceutical Co., Ltd**
**Fengxian District,**
**Shanghai 201415 (CN)**

(72) Inventor: **XU, Guifen**
**Fengxian District,**
**Shanghai 201415 (CN)**

(74) Representative: **Schwahn, Hartmut et al**
**Gleiss Grosse Schrell & Partner**
**Patentanwälte Rechtsanwälte**
**Leitzstraße 45**
**70469 Stuttgart (DE)**

(54) **THE TRADITIONAL CHINESE MEDICINE PREPARATION FOR TREATMENT OF TUMOUR AND METHOD OF MAKING AND USING SAME**

(57)    The invention provides a traditional chinese medicine and its' method of making for anti-tumor. The Traditional Chinese Medicine preparation is composed of following materials (by weight portion):SemenHydnocarpil, Momordicae SemenO.8-1.4, Squama Manitis0.5-1.1, Radix et Rhizoma Rhei0.8-1.3, Radix Glycyrrhizael-1.5. The invented preparation possesses good curative effect and low toxicity, and can be widely used to treat tumors such as digestive tract tumor including gastric carcinoma, intestines, liver cancer and esophagus , and tumors of lung, cervix, breast and skin etc. It has remarkable effect on gastric carcinoma and liver cancer.

EP 1 707 209 A1

**Description**

**Field of the Invention**

This invention involves in the fields of Traditional Chinese Medicine, in particularly, involves in a kind of Traditional Chinese Medicine preparation, its preparative methods and applications in the procedure of preparing anti-tumor drugs.

**Background of the Invention**

Cancer is one of the most serious diseases which severely threaten our healths According to the statistical materials, at present there are more than 18 million cancer patients all over the world. It is predicted that the cancer incidence will increase by 50% than that of present level, there will be 15 million new cancer cases by the year 2020 when the average onset age of cancer patient will be about 40, while now it ranges from 50 to 60, in certain cities, onset age of gastric cancer is 35, it is quite frightening.

In the field of cancer therapy, in addition to traditional therapy methods such as surgical excision , chemotherapy and radiotherapy, scientists and medical workers are now trying hard to explore and find new means and methods that can radically cure cancer, for example, cryotherapy under 180 degree below zero, heat therapy using microwave solidification; starve tumor treatment ,that is to say, blocking the blood vessels and cutting the nutrition supply of tumor; adopting biotechnology, necrosis factors, gene therapy and many other methods to treat cancers, but considering the present technology level and medical conditions at home and abroad, all of the new anti-tumor methods mentioned above still can't reach scientific conclusions because of lacking prospective study , while hoping to find new drugs which can treat cancers from Traditional Chinese Medicines, which becomes the focus of international anti-cancer fields.

Traditional Chinese Medicine and Traditional Chinese Medicine theories are one of the most precious cultural heritages, they are basic protections of ancient Chinese living and breeding, at the same time, those abounding experiences and theories are formed in the courses of fighting with diseases which still play important roles in modem civilized society.

The anti-cancer effects of Traditional Chinese Medicine have been approved by Chinese people and western countries, however anti- cancer Traditional Chinese Medicine used in the whole clinical medication only account for 3-5% , meanwhile, there is no anti-cancer Traditional Chinese Medicine used for the treatment of gastric cancer.

The idea of developing and inventing a kind of safe, effective and controllable anti cancer Traditional Chinese Medicine preparation(not prescription) which can be widely used in clinical work is the basic guiding throught of this invention.

**Summary of the Invention**

This invention is based on the Traditional Chinese Medicine theory on cancer: "Cancer is caused by stagnation of vital energy and blood stasis which further give rise to lump and tumor accumulation and should be treated with the strategies of promoting blood flow and removing blood stasis; eliminating toxic material and SanJie" , combined with modem medicine theories, using modem technology, carefully screening the herbs in order to meet three purposes: to supply a kind of anti-tumor traditional Chinese medicine preparation; to supply the preparative method; to supply its application in the preparation of anti-tumor drugs.

The purpose of this invention is realized as follows: A kind of anti-tumor traditional Chinese medicine preparation, its' feature is that this preparation is composed of following parts by weight of materials: 1 parts by weight of hydnocarpus, 0.8-1.4 parts by weight of cochinchina momordica seed, 0.5-1.1 parts by weight of pangolin scales, 0.8-1.3 parts by weight of rhubarb, 1-1.5 parts by weight of licorice root.

Of which, the herb "hydnocarpus" is adopted which can enter liver spleen meridian, its fatty acid glycerolipid that has the functions of eliminating wind, depriving the evil wetness, expelling toxin, relieving sputum and accumulating water serves as principal drug.

The herb "cochinchina momordica seed" is adopted which can enter spleen stomach meridian, its momordic acid that has the functions of detumescence, expelling toxin and promoting tissue regeneration serves as ministerial drug.

The herb "pangolin scales"is adopted which can enter liver stomach meridian, its pangolin scales alkali that has the functions of detumescence, relieving ache, removing the wind, activating collaterals, treating crewels and ulcer and fighting papillary cancer cells serves as adjunctive drug.

The herb "rhubarb" is adopted which can enter stomach intestine meridian, its emodin and rhrum tannic acids that have the functions of removing pyretic toxicity, breaking dyspeptic disease and improving microcirculation serves as adjunctive drug.

The herb "licorice root" is adopted which can enter spleen stomach meridian, its enoxolone that has the functions of anti-cancer, neutralizing poison and coordinating the drug actions of a prescription serves as messenger drug.In this invention, the optimum dosage of each crude herb is 1 parts by weigh.

Preparative methods of anti-tumor Traditional Chinese medicine preparation in this invention include the following steps:

1) Weighing each crude herb, grinding to middle size particles,
2) Adding 62% ethyl alocohol in a w/v of 1:2.5~1:3.5 with crude herbs and soaking thoroughly,
3) Heating and recirculating fully,
4) Filtrating, filter liquor acquired is the active ingredient solution of the Traditional Chinese Medicine preparation mentioned in this invention.

In this invention, to the residues and gruffs gotten in step 4, adding 62% ethyl alocohol in a w/v of 1:0.8~1:1.5, heating again and recirculating thoroughly, filtrating ,filter liquor aquired is combined with filter liquor aquired in the former steps, the combined liquid also is the active ingredient solution of the Traditional Chinese Medicine preparation mentioned in this invention.

The optimum w/v in this method is 1:1.

Heating and recirculating time mentioned in the method is 0.5-1 hour generally.

Adjusting active ingredient solution with ethyl alocohol and water, making ethyl alocohol volume percentage to be 6.0-8.0%, adjusting pH to be 4.0-5.0, then the composition of the Traditional Chinese Medicine preparation mentioned in this invention is completely made.

The best relative density of this composition of the Traditional Chinese Medicine preparation is 1.02-1.08. Or drying the active ingredient solution of the Traditional Chinese Medicine preparation mentioned above and making it into granula, filling granula into blank capsules, then the capsule of the Traditional Chinese Medicine preparation mentioned in this invention is completely made.

Alternatively, drying the active ingredient solution of the Traditional Chinese Medicine preparation mentioned above and compressing into round lamellar shape, the tablets of the Traditional Chinese Medicine preparation in this invention is conpletely made.

Hydnocarpus and cochinchina momordica seed are two poisonous herbs in the composition, the standard dosage and maximum dosage is 15g each day, dosage more than 15g will cause lowering blood pressure, short of breath, accelerating of heart beat, vomit, anepithymia, insomnia, haemolytic anemia, nephritis hyperproteinuria and erythrocyturia and other adverse reactions, on the contrary, if the dosage is less than 15 g each day, the anti-cancer effect will be poor or even ineffective completetly.

The Traditional Chinese Medicine preparation in this invention has many advantages: the herb is well organized, compatibility is reasonable, principal,adjuvant,auxiliary and conductant ingredients are highlighted, dosage is accurate, safe and effective,any herb can not be added or reduced, increasing dosage will enhance toxicity, decreasing dosage will result in poor anti-cancer effects It not only has the functions of eliminating pathogen via promoting blood flow , improving microcirculation and counteracting toxic substance, but also has the functions of strengthening body resistance via rising WBC and promoting tissue regeneration.this highly coincides with the Traditional Chinese Medicine theory of cancer therapy, that is, treating the malignant with poisonous agents, eliminating pathogen to support vital qi, it also coincides with modem medicine theory of enhancing phagocytosis function of macrophage , improving the immune system of the patients and inhibiting and killing cancer cells.

The invention was substantiated by animal experiments and clinic trails, and was proved to be safe, effective and controllable.

The invention surmount other drugs which has simple function such as strengthening body resistance , synergism or attenuation , it possess four founctions(eliminating pathogen , strengthening body resistance, synergism, attenuation)at the same time.

The invention can solely be used as anti-cancer drug, it also can be combined to use with chemotherapy drugs, when combined to use, the Traditional Chinese Medicine preparation of this invention can not only greatly reduce severe adverse reactions of chemotherapy, but also greatly improve its short term therapeutic efficacy and prostecdtive efficacy (outweighing chemotherapy), thus could enhance anti-cancer effects.

The Traditional Chinese Medicine preparation of this invention can be widely used for the treatment of tumor such as digestive tract tumor including gastric cancer, intestines cancer, liver cancer, esophageal cancer , other cancers like lung cancer, uterine cervix cancer, breast cancer, skin cancer etc, particularly, gastric cancer and liver cancer patients have good therapeutic response to the invention.

**Detailed Description of the Invention**

Example 1 Preparation of Traditional Chinese Medicine composition of the invention. 150g of hydnocarpus, 150g of cochinchina momordica seed, 150g of pangolin scales, 150g of rhubarb and 150g of licorice root. Grinding all above five herbs into middle size granula, adding 2500 ml of 62% ethyl alcohol, soaking for 12 hours, heating and recirculating for 1 hour, filtrating, adding 800 ml of 62% ethyl alcohol into gruffs and heating again, recirculating for 1 hour, filtrating and combining all of the filter liquors, decompressing and condensing to 950 ml, adjusting to 1000 ml with ethyl alcohol and water, making the volume of ethyl alcohol to be 6.0~8.0%, adjusting pH to be 4.0-5.5, regulating relative density to be 1.05, mixing evenly, standing still at 6-10˚C for 12 hours, centrifugating and extracting supernatant, bottling and thus getting the composition.

Example 2 Preparation of Traditional Chinese Medicine composition of the invention. 150g of hydnocarpus, 240g of cochinchina momordica seed, 120g of pangolin scales, 120g of rhubarb and 225g of licorice root.Grinding all above five herbs into middle size granula, adding 2600 ml of 62% ethyl alcohol, soaking for 18 hours, heating and recirculating for 1 hour, filtrating, adding 900 ml of 62% ethyl alcohol into gruffs and heating again, recirculating for 1 hour, filtrating and combining all of the filter liquors, decompressing and condensing to 950 ml, adjusting to 1000 ml with ethyl alcohol and water, making the volume of ethyl alcohol to be 6.0~8.0%, adjusting pH to be 4.0-5.5, regulating relative density to be1.02, mixing evenly, standing still at 6-10˚C for 12 hours, centrifugating and extracting supernatant, bottling and thus getting the composition.

Example 3 Preparation of Traditional Chinese Medicine composition of the invention. 80g of hydnocarpus,75g of cochinchina momordica seed, 50g of pangolin scales, 75g of rhubarb and 100g of licorice root.Grinding all above five herbs into middle size granula, adding 950 ml of 62% ethyl alcohol , soaking for 12 hours, heating and recirculating for 1 hour, filtrating, adding 320 ml of 62% ethyl alcohol into gruffs and heating again, recirculating for 1 hour, filtrating and combining all of the filter liquors, decompressing and condensing to 950 ml, adjusting to 1000 ml with ethyl alcohol and water, making the volume of ethyl alcohol to be 6.0~8.0%, adjusting pH to be 4.0-5.5, regulating relative density to be 1.06, mixing evenly, standing still at 6-10˚C for 12 hours, centrifugating and extracting supernatant, bottling and thus getting the composition.

Example 4 Preparation of Traditional Chinese Medicine composition of the invention. 120g of hydnocarpus, 140g of cochinchina momordica seed,100g of pangolin scales, 150g of rhubarb and 180g of licorice root. Grinding all above five herbs into middle size granula, adding 2000 ml of 62% ethyl alcohol , soaking for 12 hours, heating and recirculating for 1 hour, filtrating, adding 700 ml of 62% ethyl alcohol into gruffs and heating again, recirculating for 1 hour, filtrating and combining all of the filter liquors, decompressing and condensing to 950 ml, adjusting to 1000 ml with ethyl alcohol and water, making the volume of ethyl alcohol to be 6.0~8.0%, adjusting pH to be4.0-5.5, regulating relative density to be 1.08, mixing evenly, standing still at 6-10˚C for 12 hours, centrifugating and extracting supernatant, bottling and thus getting the composition.

Example 5 Preparation of Traditional Chinese Medicine capsules of the invention. 180g of hydnocarpus,180g of cochinchina momordica seed,90g of pangolin scales, 150g of rhubarb and 250g of licorice root. Grinding all above five herbs into middle size granula, adding 2500 ml of 62% ethyl alcohol, soaking for 24 hours, heating and recirculating for 1 hour, filtrating, adding 1000 ml of 62% ethyl alcohol into gruffs and heating again, recirculating for 1 hour, filtrating and combining all of the filter liquors, drying and making into uniform granula and filling them into vacant capsules, thus forming hard capsules.

Example 6 Preparation of Traditional Chinese Medicine tablets of the invention. 100g of hydnocarpus, 80g of cochinchina momordica seed,110g of pangolin scales, 130g of rhubarb and 150g of licorice root.Grinding all above five herbs into middle size granula, adding 1500 ml of 62% ethyl alcohol, soaking for 18 hours, heating and recirculating for 0.5 hour, filtrating, adding 750 ml of 62% ethyl alcohol into gruffs and heating again, recirculating for 1 hour, filtrating and combining all of the filter liquors, drying and compressing them into ground lamellar shape, thus getting the tablets.

**Test example 1: Pharmacodynamics research of the Traditional Chinese Medicine preparation of this invention.**

I Tested Drugs

1.Preparation labeled dosage: lmlcontaining 0.55g of crude drug
2.Solvent: 0.5%CMC-Na
3. Preparative methods: stock solution was diluted to demanding concentration with 0.5%CMC-Na, giving 0.5 ml of drug to each mouse each time.

II Animals

1. Name, source, Strain: BALB/c mice or F1 (ICR×BALB/c), mice and Kunming mice, animal group of our institute. C57BL/6 mice and nu BALB/c mice were purchased from Shanghai laboratory animal center.
2. Weight: 19±1g, 6-8weeks old.
3.Sex: male or female, using the same sex for each study.
4.Animal breeding and experiment conditions: Kunming mice, C57BL/6 mice and F1 mice were kept in Clean Animal Laboratory,nu/BALB/c mice were kept in lamina flow framework and raised according to SPF condition, administration was given in the lamina flow framework.
5. The number of animal of each group:tested group , three dosage.Positive group, and blank group.6 nuBALB/c mice each group, 10 Kunming mice, 10 C57HL/6 mice and 10 F1 mice each group.

III Test Method Choice

According to «Guideline for Traditional Chinese Medicine Research», performing study on eliminating pathogen to strengthen body resistance, attenuation and synergism function

Considering that it is a compound preparation, it should adopt the whole animal test.

IV Dosage

po (take orally), 25.0, 12.5 and 6.25ml/kg or 75, 37.5 and 18.75ml/M2 for high dosage, middle dosage, low dosage respectively.

ip (intraperitoneal injection) 10.0, 5.0 and 2.5ml/kg for high dosage, middle dosage, low dosage respectively.

V Administration manner

po×10qd and ip×10qd for studing on eliminating pathogen, strengthening body resistance, attenuation and synergism function, of which, po is the administration manner for clinical medication; Studying on strengthening body resistance function only use po path, implementing 12.5, 6.25, 3.125ml/kg po×10 regimen

VI Controls

Blank: solvent 0.5%CMC-Na.

Positive drug control: Considering that there is no suitable corresponding positive control, it should choose cyclophosphamide as positive control, in order to substantiate authenticity of each test.

VII. Test Main Steps and Results

1. Eliminating pathogen function study

Test on xenogeneic graft mice models of human gastric cancer cell line MKN and human liver cancer cell line QGY: Taking related cancer cells, preparing to homogenate containing 1-2×10$^7$tumor cells/ml,chosing corresponding recipient mice, armpit hypodermic inoculation 0.2 ml or pedis hypodermic inoculation 0.05 ml of tumor cell suspension, randomization, giving therapy according to the administration regimen next day, dissecting each test group tumor after 2 weeks and comparing with control group, calculating inhibition ratio, all the procedure should be performed strictly under sterilized condition, results are shown in table 1-4 Inhibition test on animal-transplanted tumor such as mice colon carcinoma C26 and Lewis lung carcinoma, methods are the same as above, and the results are shown in table 5-10. note: "***"represents P<0.01; "**"represents P<0.05; "*"represents P<0.1.

Table 1. Therapeutic effects test of invented composition on xenogeneic graft mice models of human gastric cancer cell line MKN via po

| sample group | dosage ml/kg | administration ml/M$^2$ | mice number regimen | weight beginning | end | tumor weight beginning | end | inhibition ratio $\bar{x}\pm$SD | % |
|---|---|---|---|---|---|---|---|---|---|
| invented composition | 25 | 75 | po×10qd | 6 | 6 | 17.6 | 17.1 | 0.317±0.12 | 77.83*** |
| invented composition | 12.5 | 37.5 | po×10qd | 6 | 6 | 17.7 | 17.7 | 0.717±0.12 | 49.86*** |
| invented composition | 6.25 | 18.75 | po×10qd | 6 | 6 | 17.8 | 17.4 | 1.12±0.23 | 21.68 |
| positive control | 30mg/kg | | | | | | | | |
| cyclophosphamide | | | ip×7qd | 6 | 6 | 17.7 | 16.6 | 0.17±0.15 | 88.11*** |
| negative control | | solvent | po×10qd | 12 | 12 | 17.5 | 19.9 | 1.43±0.24 | |

table 2. Therapeutic effects test of invented composition on xenogeneic graft mice models of human gastric cancer cell line MKN via peritoneal injection

| sample group | dosage ml/kg | administration ml/M² | mice number regimen | weight beginning | end | tumor weight beginning | end | inhibition ratio $\overline{x}\pm$SD | % |
|---|---|---|---|---|---|---|---|---|---|
| invented composition | 10 | 30 | ip×10qd | 6 | 6 | 18.3 | 17.7 | 0.25±0.10 | 80.47*** |
| invented composition | 5 | 15 | ip×10qd | 6 | 6 | 18.5 | 18.4 | 0.53±0.12 | 58.59*** |
| invented composition | 2.5 | 7.5 | ip×10qd | 6 | 6 | 18.2 | 18.6 | 0.90±0.26 | 26.69 |
| positive control cyclophosphamide | 30mg/kg | | ipx7qd | 6 | 6 | 18.0 | 17.9 | 0.14±0.05 | 89.06*** |
| negative control | solvent | | ip×10qd | 12 | 12 | 18.1 | 20.1 | 1.28±0.26 | |

table 3. Therapeutic effects test of invented composition on xenogeneic graft mice models of human liver cancer cell line QGY via po

| sample group | dosage ml/kg | administration ml/M$^2$ | mice number regimen | weight beginning | end | tumor weight beginning | end | inhibition ratio $\overline{x}\pm$SD | % |
|---|---|---|---|---|---|---|---|---|---|
| invented composition | 25 | 75 | po×10qd | 6 | 6 | 17.4 | 17.9 | 1.13±0.22 | 49.33*** |
| invented composition | 12.5 | 37.5 | po×10qd | 6 | 6 | 17. 7 | 18.0 | 1.48±0.30 | 33.63** |
| invented composition | 6.25 | 18.75 | po×10qd | 6 | 6 | 17.5 | 17.9 | 1.65±0.20 | 26.00 |
| positive control | | 30mg/kg | | | | | | | |
| cyclophosphamide | | | ipx7qd | 6 | 6 | 17.6 | 17.0 | 0.25±0.14 | 88.79*** |
| negative control | | solvent | po×10qd | 12 | 12 | 17.5 | 19.4 | 2.23±0.31 | |

table 4. Therapeutic effects test of invented composition on xenogeneic graft mice models of human liver cancer cell line QGY via peritoneal injection

| sample group | dosage ml/kg | administration ml/M² | mice number regimen | weight beginning | end | tumor weight beginning | end | inhibition ratio $\bar{x}\pm$SD | % |
|---|---|---|---|---|---|---|---|---|---|
| invented composition | 10 | 30 | ip×10qd | 6 | 6 | 17. 3 | 18. | 1.03±0.29 | 48. 76*** |
| invented composition | 5 | 15 | ip×10qd | 6 | 6 | 17.2 | 18.7 | 1.37±0.23 | 31.84** |
| invented composition | 2.5 | 7.5 | ip×10qd | 6 | 6 | 16.8 | 18.4 | 1.60+0.24 | 20.40 |
| positive control cyclophosphamide | | 30mg/kg | ipx7qd | 6 | 6 | 17.0 | 17.6 | 0.22±0.08 | 89.05*** |
| negative control | | solvent | ip×10qd | 12 | 12 | 17.3 | 19.5 | 2.01±0.33 | |

table 5. Therapeutic effects test of invented composition on mice C26 colon solid tumor via po

| sample group | dosage ml/kg | administration ml/M$^2$ | mice number regimen | weight beginning | end | tumor weight beginning | end | inhibition ratio $\overline{x} \pm$SD | % |
|---|---|---|---|---|---|---|---|---|---|
| invented composition | 25 | 75 | po×10qd | 10 | 10 | 21. 2 | 24.7 | 1.38±0.22 | 43.67*** |
| invented composition | 12.5 | 37.5 | po×10qd | 10 | 9 | 21. 4 | 25.2 | 1.71±0.36 | 30.2** |
| invented composition | 6.25 | 18.75 | po×10qd | 10 | 10 | 21. 3 | 25.7 | 2.24±0.25 | 8.6 |
| positive control cyclophosphamide | 30mg/kg | | ipx7qd | 10 | 10 | 21.3 | 22.6 | 0.22±0.06 | 91.02*** |
| negative control | | solvent | po×10qd | 20 | 20 | 21. 3 | 26.2 | 2.45±0.57 | |

table 6. Therapeutic effects test of invented composition on mice C-26 colon solid tumor via peritoneal injection

| sample group | dosage ml/kg | administration ml/M$^2$ | mice regimen | weight beginning | end | tumor weight beginning | end | inhibition ratio $\overline{x}\pm$SD | % |
|---|---|---|---|---|---|---|---|---|---|
| invented composition | 10 | 30 | ip×10qd | 10 | 10 | 20.1 | 23.1 | 1.36±0.20 | 49. 91*** |
| invented composition | 5 | 15 | ip×10qd | 10 | 10 | 20.3 | 23.9 | 1.70±0.37 | 37. 98** |
| invented composition | 2.5 | 7.5 | ip×10qd | 10 | 10 | 20.4 | 23.4 | 2.12±0.47 | 21. 92 |
| positive control cyclophosphamide | 30mg/kg | | ipx7qd | 10 | 10 | 20.2 | 21.0 | 0.22±0.06 | 91.90*** |
| negative control | | solvent | ip×10qd | 20 | 20 | 20. 4 | 24.8 | 2.75±0.45 | |

table 7. Therapeutic effects test of invented composition on mice C-26 colon carcinoma (pedis inoculation) via po

| sample group | dosage ml/kg | administration ml/M$^2$ | mice number regimen | weight beginning | end | tumor weight beginning | end | inhibition ratio $\overline{x}\pm$SD | % |
|---|---|---|---|---|---|---|---|---|---|
| invented composition | 25 | 75 | po×10qd | 10 | 10 | 19.3 | 21.0 | 0.41±0.07 | 54.95*** |
| invented composition | 12.5 | 37.5 | po×10qd | 10 | 10 | 19.2 | 21.7 | 0.55±0.11 | 39.56*** |
| invented composition | 6.25 | 18.75 | po×10qd | 10 | 10 | 19.1 | 22.3 | 0.70±0.15 | 23.08 |
| positive control | | 30mg/kg | | | | | | | |
| cyclophosphamide | | | ipx7qd | 10 | 10 | 19.1 | 20.4 | 0.19±0.06 | 79.12*** |
| negative control | | solvent | po×10qd | 20 | 20 | 19.3 | 24.9 | 0.91±0.18 | |

table 8. Therapeutic effects test of invented composition on mice C-26 colon carcinoma (pedis inoculation) via peritoneal injection

| sample group | dosage ml/kg | administration ml/M$^2$ | mice number regimen | weight beginning | end | tumor weight beginning | end | inhibition ratio $\overline{x}\pm$SD | % |
|---|---|---|---|---|---|---|---|---|---|
| invented composition | 10 | 30 | ip×10qd | 10 | 10 | 19.0 | 21. 7 | 0.42±0.06 | 56.48*** |
| invented composition | 5 | 15 | ip×10qd | 10 | 10 | 18.9 | 21. 4 | 0.58±0.11 | 39.90*** |
| invented composition | 2.5 | 7.5 | ip×10qd | 10 | 10 | 18.7 | 22.3 | 0.75±0.20 | 22.28 |
| positive control | | 30mg/kg | | | | | | | |
| cyclophosphamide | | | ipx7qd | 10 | 10 | 18.7 | 20.0 | 0.18±0.06 | 81.35*** |
| negative control | | solvent | ip×10qd | 20 | 20 | 18.8 | 23. 1 | 0.965±0.18 | |

table 9. Therapeutic effects test of invented composition on mice Lewis lung solid tumor via po

| sample group | dosage ml/kg | administration ml/M$^2$ | mice number regimen | weight beginning | end | tumor weight beginning | end | inhibition ratio $\overline{x}\pm$SD | % |
|---|---|---|---|---|---|---|---|---|---|
| invented composition | 25 | 75 | po×10qd | 10 | 9 | 18.4 | 19.4 | 1.51±0.90 | 40. 08*** |
| invented composition | 12.5 | 37.5 | po×10qd | 10 | 10 | 18. 7 | 20.0 | 1.72±0.38 | 31. 75** |
| invented composition | 6.25 | 18.75 | po×10qd | 10 | 10 | 18.5 | 20.8 | 1.92±0.27 | 23.81 |
| positive control cyclophosphamide | 30mg/kg | | ipx7qd | 10 | 10 | 18.8 | 19.3 | 0.29±0.07 | 88.49*** |
| negative control | solvent | | po×10qd | 20 | 20 | 18. 5 | 22.0 | 2.52±0.61 | |

table 10. Therapeutic effects test of invented composition on mice Lewis lung solid tumor via peritoneal injection

| sample group | dosage ml/kg | administration ml/M$^2$ | mice number regimen | weight beginning | end | tumor weight beginning | end | inhibition ratio $\overline{x}\pm$SD | % |
|---|---|---|---|---|---|---|---|---|---|
| invented composition | 10 | 30 | ip×10qd | 10 | 10 | 18.3 | 19.9 | 1.18±0.27 | 44. 86*** |
| invented composition | 5 | 15 | ip×10qd | 10 | 10 | 18.3 | 20.4 | 1.41±0.43 | 34.11** |
| invented composition | 2.5 | 7.5 | ip×10qd | 10 | 10 | 18.5 | 20.7 | 1.57±0.53 | 26.64 |
| positive control | | 30mg/kg | | | | | | | |
| cyclophosphamide | | | ipx7qd | 10 | 10 | 18.3 | 19.4 | 0.25±0.07 | 88.32*** |
| negative control | | solvent | ip×10qd | 20 | 20 | 18.6 | 22.7 | 2.14±0.27 | |

Through vivo anti- tumor therapeutic effect study, it demonstrates that the Traditional Chinese Medicine composition of the invention has a relatively high tumor inhibition rate when used at a high dosage of 25ml/kg po×10 and 10ml/kg ip×10 on xenogeneic graft mice models of human gastric cancer cell line MKN, the average inhibition rate is 79.75% and 81.54% respectively, which is 2.7 times higher than the level of goverment regulation(30%), middle dosage 12.5ml/kgpo× 10 and 5ml/kg ip×10 also have a moderate tumor inhibition rate, the average inhibition rate is 53.89% and 58.80% respectively.For other animal-transplanted tumor such as colon carcinoma C-26 and Lewis lung carcinoma, xenogeneic graft mice models of human liver cancer cell line QGY, high dosage of invented composition via po or ip both have a moderate anti-tumor effect. Of which, effect of peritoneal injection is better than that of po. Main pharmacodynamics study shows that the invented Traditional Chinese Medicine composition has obvious eliminating pathogen functions.

2.Strengthening body resistance function study

1) Influence of invented Traditional Chinese Medicine composition on phagocytosis function of macrophages exists in the Kunming mice abdominal cavities: Randomly dividing male Kunming mice into several groups, 10 mice each group. Giving composition via po for consecutive 10 days, once each day.Peritoneal injection 1.5 ml of 0.5 % aminopeptodrate for each group of mouse after the last administration of composition, then 24 hours later, peritoneal injection 0.2 ml of chicken red blood cells suspension at a concentration of $1\times10^6$/ml,40 minutes later, washing and collecting mice peritoneal fluid with physiologic saline, centrifugated, collecting cell sediments and making into smear, mehanol fixation, Giemsa staining, mounting. Counting 100 macrophages using immersion objective, Counting the number of macrophage that phagocytized chicken red blood cells and counting total number of phagocytized chicken red blood cells. Calculating phagocytosis percentage and phagocytosis index according to following formula, and the results are shown in table 11.

$$\text{phagocytosis percentage} = \frac{\text{The Number of macrophage that phagocytized chicken RBC amomg 100 macrophages}}{100 \text{ macrophages}} \times 100\%$$

$$\text{phagocytosis index} = \frac{\text{total number of phagocytized chicken RBC among 100 macrophages}}{100 \text{ macrophage}}$$

Table 11 Influence of invented composition on phagocytosis function of macrophages exists in the Kunming mice abdominal cavities

| sample group | dosage ml/kg | administration | mice number | phagocytosis percentage | phagocytosis index |
|---|---|---|---|---|---|
| | | regimen | | $\overline{x}\pm$SD% | |
| composition | 12.5 | po×10qd | 10 | 38.4±6.48** | 0.77±0.06*** |
| control | solvent | po×10qd | 10 | 26.7±7.32 | 0.53±0.08 |
| composition | 12.5 | po×10qd | 10 | 39.6±5.10** | 0.58±0.05*** |
| control | solvent | po×10qd | 10 | 29.2±4.6 | 0.33±0.07 |

2) Influence of invented Traditional Chinese Medicine composition on NK cell activity of the Lewis lung carcinoma bearing mice: hypodermic inoculating $1\times10^6$ Lewis lung carcinoma cell suspensions on C57BL/6 right pedis, regimen 12.5, 6.25 and 3.125ml/kg po×7qd was implemented on the following day with invented composition .after all administration was finished, killing all the mice, taking out spleen and collecting spleen cells , making into effector cells at a concentration of $1\times10^7$/ml. using cultured Yac-1 cells as target cells, concentration is $1\times10^6$/ml, taking out these two cells 100ul respectively and adding into 96 well plate, adding $1.75 \times 104$Bq/well of $^3$H-TdR and culturing for 24 hours, collecting cells, assaying cpm value of each well with Liquid Scintillation Counters and calculating the obvious difference between test groups and control groups(shown in table 12)

Table 12. Influence of invented composition on NK cell activity of cancer bearing mice

| sample group | dosage ml/kg | administration regimen | CPM value $\overline{x} \pm$SD |
|---|---|---|---|
| invented composition | 12.5 | po$\times$10qd | 4213$\pm$728 ** |
| invented composition | 6.25 | pox10qd | 3746$\pm$835 ** |
| invented composition | 3.125 | po$\times$10qd | 4306$\pm$663 ** |
| control | | po$\times$10qd | 5996$\pm$908 |
| invented composition | 12.5 | po$\times$10qd | 3628$\pm$551 ** |
| invented composition | 6.25 | po$\times$10qd | 3150$\pm$908 ** |
| invented composition | 3.125 | po$\times$10qd | 3726$\pm$1141 ** |
| control | | po$\times$10qd | 4938$\pm$871 |

The invented Traditional Chinese Medicine composition can obviously enhance the phagocytosis functions of macrophages in the mice abdominal cavities; at the same time, it can enhance NK cell activity of Lewis lung carcinoma bearing mice to some extent.

3.Synergistic effect study

Hypodermic inoculating S180 sarcomas homogenate on Kunming mice armpit, dividing mice into different groups next day, solely used group: the invented Traditional Chinese Medicine composition, 25.0, 12.5, 6.25ml/kg po$\times$10; combinely used group: 25.0, 12.5, 6.25ml/kg po$\times$10 plus 15mg/kg cyclophosphamide ip$\times$7, 12days after inoculation, dissecting tumors, measuring average tumor weight of each group and calculating standard deviation, comparing test group with control group and calculating tumor inhibition rate, and the results demonstrate that high dosage of invented Traditional Chinese Medicine composition, combined with low dosage of cyclophosphamide, produces certain synergism effects on the treatment of S180 sarcomas(shown in table 13).

Table 13. Therapeutic effects of the invented composition combined with cyclophosphamide on S$_{180}$

| sample group | dosage ml/kg | administration regimen | tumor weight (g) $\overline{x} \pm$SD | inhibition rate % |
|---|---|---|---|---|
| invented composition | 25.0 | po$\times$10 | 1.19$\pm$0.36 | 61.73*** |
| invented composition | 12.5 | pox 10 | 1.69$\pm$0.29 | 45.66*** |
| invented composition | 6.25 | po$\times$10 | 2.15$\pm$0.27 | 30.87*** |
| invented composition | 25.0 | po$\times$10 | 0.88$\pm$0.31 | 71.70*** |
| CTX | 15.0mg/kg | ipx7 | | |
| invented composition | 12.5 | po$\times$10 | 1.55$\pm$0.40 | 50.16*** |
| CTX | 15.0mg/kg | ipx7 | | |
| invented composition | 6.25 | po$\times$10 | 1.84$\pm$0.24 | 40.84*** |
| CTX | 15.0mg/kg | ipx7 | | |
| CTX | 15.0mg/kg | ipx7 | 1.71$\pm$0.25 | 45.02*** |
| CTX | 30.0mg/kg | ipx7 | 0.49$\pm$0.12 | 84.24*** |
| control | solvent | po$\times$10 | 3.10$\pm$0.46 | |
| control | solvent | po$\times$10 | | |

4. Attenuation function study

Cyclophosphamide 100mg/kg ipx2 was given to F1 mice, then randomly divided into three different groups, treated with 25. 0,12. 5,6. 25ml/kg po$\times$10qd of the invented Traditional Chinese Medicine composition, counting white blood cells every four days, getting average number and standard deviation, setting the WBC number of 0 day as 100%, calculating WBC percentage of each timepoint, it shows that the invented composition has no significant functions of rising WBC, at the same time, it also has no effects of enhancing WBC inhibition(shown in table14).

Table 14. Attenuation function of the invented composition on WBC inhibition caused by cyclophosphamide

| sample group | dosage ml/kg | administration regimen | WBC percentage | | | | | |
|---|---|---|---|---|---|---|---|---|
| | | | 0d | 3d | 6d | 9d | 12d | 15d |
| invented composition | 25.0 | po×10qd | 100 | 39.4 | 45.6 | 57.2 | 68.1 | 98.9 |
| invented composition | 12.5 | po×10qd | 100 | 36.7 | 42.1 | 54.8 | 64.0 | 99.5 |
| invented composition | 6.25 | po×10qd | 100 | 35.9 | 44.2 | 51.4 | 62.1 | 103.3 |
| solvent | | po×10qd | 100 | 33.1 | 42.8 | 48.3 | 59.4 | 92.6 |

**Test example2 In vitro anti-tumor cell proliferation test of the invented Traditional Chinese Medicine composition**

I. Tested drugs

1.Content potency: 1ml containing 0.55g of crude drug.
2. Preparative methods: dissolved in the culture medium containing fetal bovine serum, preparing each time when used.

II.Cell strains

Human lung cancer cell line(Al).
Human cervical carcinoma cell line(Hela).
Source: cell bank of Shanghai Institute of Cell Biology.

III.Main experiment steps

1.Seeding tumor cells into culture bottle, 130 thousand unit each bottle.
2.Dividing into different dosage groups
3.Adding culture medium containing specified dosage of tested composition and control drug respectively.
4. Counting the number of cells in different group within in defined time.

IV. Specification of index and time

Observing inhibition rate of different dosage composition on tumor cells and drug concentration on inhibiting concentration (IC50), observing time is 4 days.

V.Dosage setting

Five dosage, they are 0.55,2.75,5.5,13.75,27.5mg/ml respectively.

VI. Administration manner

Composition is added into culture medium and used to culture cells directly.

VII. Controls

chosing canelim capsule as control, ground into powder and steriled, dissolved into medium and centrifugated, divided into 0.3, 1.5,3,7.5,15mg/ml five dosage, administration manner is the same as above.

VIII Results:

The invented composition can inhibit human tumor cells Hela and A1 proliferation in a manner of dosage dependence, while the tumor cells in the group without adding the invented composition can proliferate infinitely, entering exponential phase of growth, half inhibiting concentration of test group and control group are shown in table15.

Table 15. Half inhibiting concentration of the invented composition and canelim capsule on tumor cell growth ($IC_{50}$, $\overline{x}\pm SD$ n=3)

| Cell line | Invented composition (mg/ml) | Canelim capsule (mg/ml) |
|---|---|---|
| Hela | 4.06±1.92 | 5.35±2.19 |
| A1 | 2.12±0.41 | 5.51±2.47 |

The invented composition has inhibition effects on vitro cultured human tumor cells (Hela, Al), half inhibiting concentration ($IC_{50}$) is about 2-5mg/ml.

**Test example3 Acute toxicity test of the invented Traditional Chinese Medicine composition**

I .Tested drugs

1.Preparation labeled dosage: 1ml containing 0.55g of crude drug.
2. Preparative methods: stock solution was diluted to demanding concentration with sterile purified water.
3. Solvent: sterile purified water

II .Animal :mice

1. Kunming mice, supplied by animal center, Shanghai institute of pharmaceutical industry
2 Weight: 20±1g, 6-7weeks old.
3.Mice number:20 mice each group(10 male mice and 10 female mice)

III. Test Methods

1. Administration manner: ip
2.Dosage group:5 groups, calculation unit ml/kg.
3.Volume: 0.5ml per mouse.
4.Solvent: sterile purified water
5.Mice abnormal reactions: Mice immediately appear abdomen intense contraction, body stretching and twist, rebound, short of breath, then mice show action retardation, hair looseness, death appears 1 hour after administration, death climax appears 6 hours after administration. Individual mouse dies 3 days after administration(shown in table16), dissecting dead mice, only mesentery congestion, lots of drug residues in abdominal cavities can be observed by naked eyes. Observing for 3 weeks, calculating LD50 with Bliss method.
6.Results
Ip×1 LD50 of the invented Traditional Chinese Medicine composition is shown in table16. It demonstrates that $LD_{50}$ of the invented Traditional Chinese Medicine composition on Kunming mice has no significant difference between male and female mice (P>0.05) (See tablel7) .

Table 16. Animal death distribution of acute toxicity of the invented composition via ip

| sex | dosage confidence limit) ml/kg | mice number | death distribution (date) | | | | | | | death rate % | $LD_{50}$ (95% confidence limit) ml/kg |
|---|---|---|---|---|---|---|---|---|---|---|---|
| | | | 1 | 2 | 3 | 4 | 5 | 6 | 7......21 | | |
| male | 25 | 10 | 9 | 1 | 0 | 0 | 0 | 0 | 0 | 100 | |
| | 20 | 10 | 5 | 3 | 0 | 0 | 0 | 0 | 0 | 80 | |
| | 16 | 10 | 2 | 1 | 0 | 1 | 0 | 0 | 0 | 40 | 16.7 (15.2-18.36) |
| | 12.8 | 10 | 0 | 1 | 0 | 0 | 0 | 0 | 0 | 10 | |
| | 10.24 | 10 | 0 | 0 | 0 | 0 | 0 | 0 | 0 | 0 | |
| female | 25 | 10 | 10 | 0 | 0 | 0 | 0 | 0 | 0 | 100 | |
| | 20 | 10 | 5 | 2 | 0 | 0 | 0 | 0 | 0 | 70 | |
| | 16 | 10 | 3 | 1 | 0 | 0 | 0 | 0 | 0 | 40 | 17.08 (15.47-18.86) |
| | 12.8 | 10 | 0 | 1 | 0 | 0 | 0 | 0 | 0 | 10 | |
| | 10.24 | 10 | 0 | 0 | 0 | 0 | 0 | 0 | 0 | 0 | |

Table 17. Acute toxicity test results of the invented composition on mice via ip administration

| sex | $LD_{50}$ | $LD_5$ | $LD_{95}$ (95 %confidence limit) |
|---|---|---|---|
| | ml/kg | | |
| male | 16.7 (15.2-18.36) | 12. 27 (10.34-14.56) | 22. 71 (19.12-26.97) |
| female | 17.08 (15.47-18.86) | 12.19 (10.17-14.62) | 23.91 (19.87-28.77) |
| male and female | 16.89 (15.77-18.09) | 12.23 (10.79-13.85) | 23.32 (20.55-26.46) |

6.Conclusion

Acute toxicity LD50 of the invented composition on mice via ip administration is 16.89ml/kg, which is equal to 9.29g/kg of crude drug.

**Application example1 Summary of clinical trail of the invented composition on the treatment of primary hepatic carcinoma and gastric cancer**

Object and method

I . Choice of eligible tested object

1. Chinese medicine syndrome diagnosis symptom of stagnation of poison Gastric cavity full, hard lump, Stabbing pain, loss of appetite, fatigue, vomit, haematemesis,hemafecia, dark , dark red, purple, cyanochroia or ecchymosis texture of tongue, white or yellow coated tongue, extenuated, deep or astringent pulse.
2.Western medical diagosis criterion:
Primary hepatic carcinoma

1) pathologic diagnosis

(1)Liver histological examination proved to be primary hepatic carcinoma
(2) Histologic examination of extra-hepatic tissue proved to be primary hepatic carcinoma

2) Clinical diagnosis

(1) If there is no other evidence of hepatic carcinoma, AFP positive by convection method check or AFP ≥400ng/ml by radioimmunity method check, and persistent more than 4 weeks, excluding pregnancy, reactive hepatopathy, gonadal embryonal tumor and metastatic hepatic carcinoma.

(2) Have or have no clinical manifestation, B ultrasonic check and CT examination show definite intrahepatic parenchymatous occupying lesion, excluding hemangiomas of liver and metastatic hepatic carcinoma., plus any one of the followings:

① AFP≥200ng/ml or obviously high γ-GT.
② Imageological manifestation of typical primary hepatic carcinoma.
③ No jaundice, but obviously high AKP or γ -GT.
④ Obvious metastasis in distant area, bloody ascites or finding cancer cells in the ascites.
⑤ Definite cirrhosis with type B hepatitis marker positive.

3) Clinical stage criterion

I : No definite symptoms and signs of hepatic carcinoma, CT, B ultrasonic examination finds single node, size less than 5cm.
II : Mild symptom, good general condition, exceeds criterion of stage I , while there is no evidence of stage III: any one of obvious cachexia, jaundice, ascites or extrahepatic metastasis Gastric carcinoma

(1) Medical history and symptom: no symptom at earlier period, male, >40, epigast discomfort for unknown reasons, pain, progressing anemia and emaciation, or regularity of ulcer changed, loss of appetite, vomit, haematemesis or hemafecia.
(2) Sign: epigast tenderness or lump palpable, at advantaged stage, superficial lymphadenectasis can be palpable, hard, ascites, anemia.
(3) Fecal occult blood test: Fecal occult blood test shows positive for consecutive 3 days.
(4) Gastric fluid analysis: gastric fluid decrease or hypchlorhydia.
(5) Upper gastrointestinal opacification: dysperistalsis, destroy of gastric mucos, changing of gastric emptying time (acceleration or retardation), abnormality of gastric contour, niche sign of irregular margin and filling defect.
(6) Gastric endoscope examination: tumor or large irregular ulcer can be visible.
(7) Exfoliative cytometer examination of gastric fluid: finding out typical cancer cells.
(8) Operation pathologic sample, biopsy of superficial lymph nodes, endoscope pathologic sample substantiated cases.
Clinical stage criterion of gastric carcinoma

I : Superficial carcinoma with no lymph nodes metastasis and tumor infiltrated into less than 1/2 sector of the muscular layer.
II: Superficial carcinoma with first station lymph nodes metastasis and carcinoma infiltrated into muscular layer, exceeded 1 sector, and $T_3$ tumor without or only with adjacent lymph nodes metastasis.
III: Regardless of tumor size, tumor with distant superficial lymph nodes metastasis or with adjacent deep lymph nodes metastasis, or tumor only with adjacent superficial lymph nodes metastasis, even without lymph nodes metastasis, but the size of the tumor exceeded 1 sector of muscular layer or infiltrated into surrounding tissues.
IV: Regardless of tumor size, tumor with distant metastasis or metastasis of hepatic hilum lymph nodes,para- arteria coeliaca lymph nodes, paraaortic lymoh nodes, para- arteria colica media lymph nodes or lymph nodes of root of mesentery.

1. Inclusion criterion

(1) Stage I , II patients are reluctant to receive other treatments, participating clinical trails voluntarily.
(2) Those patients who have received anti-cancer therapy(including total chemotherapy, arterial cannula chemotherapy and embolotherapy), partly radiotherapy, operations(excluding patients who relapsed after surgical radical correction), cryotherapy or injecting with absolute alcohol, need stop therapy for over 3 months.
(3) Age>18years
(4) Predicted survival time>2months, survival quality Karnofsky score≥50

2. Excluding Case Criterion

(1) Age<18years
(2) Pregnant or breast-feeding women.
(3) Patients with esophageal stenosis, polypi or tumor; gastroduodenal ulcer; reactive gastritis, atrophic gastritis, bile reflux gastritis; bowel obstruction; structural diseases of liver, cholecyst, pancreas, colon; patients can not receive medication via po.
(4) Signs of gastric perforation or bleeding.
(5) Combined heart, liver, kidney and hematopoietic system, immune system severe primary diseases, psychotic patients.
(6) Those who refuse therapy.
(7) Be considered as those are unsuitable for participating in clinical trail by researchers.

II. Methods of clinical trails

1. Trail design:

According to Investigational New Drug Application(IND), performing clinical trails with the invented Traditional Chinese Medicine composition on treatment of primary hepatic carcinoma and gastric carcinoma, primary hepatic carcinoma cases are no less than 30 cases, gastric carcinoma cases are no less than 30 cases, without setting controls, in order to substantiate its anti-cancer effects.

2. Adiministration manner and dosage:

The invented Traditional Chinese Medicine composition, taken orally, twice each day, 15ml each time(1 ml containing crude drugs 0.75g), taken in the early morning and evening, taken with warm water.

3. Course of treatment: 2 months.
4. 0bserved items and methods

(1) Safety detection: blood, urine, faeces routine check; liver, kidney function, ECG are checked before and after therapy. In clinical trails, observe carefully the adverse reactions which may be caused by the invented composition, such as symptoms of digest, respiration, circulation,nerve and blood systems.
(2) Estimation of therapeutic efficacy:

① Tumor foci: performing B ultrasonic, CT or/and MRI examination before and after treatment.

a Measurement of tumor foci size: multiply the two perpendicular maximum diameter.
b Multiple tumor foci are measured with sum of all products of multiplication (refer to product of two perpendicular maximum diameter).
c Diffused nodular tumor should be explained particularly.
d Recording with or without portal vein cancer cell embolism.

② Clinical symptom observations:

a Main symptoms of primary heparic and gastric carcinoma:
Hepatic region pain, lump in superior belly, fatigue, emaciation, jaundice and fever.
b Main symptoms of stagnation of poison:
Lump below the costal region, discomfortableness and pain, fever, dry mouth and bitter mouth, dry stool, constipation, body or eyestained yellow, dark red, purple or cyanochroia texture of tongue, yellow coated tongue, astrigent pulse.
c Survival quality: Karnofsky grade
d Other examination items: AFP, AKP, $\gamma$-GT, CD3, CD4, CD8 etc.

Observing methods:

Observing methods:observing and recording symptoms, Karnofsky score, tongue and pulse regularly; Laboratory examination items including blood routine test, bleeding time and clotting time are checked once a week during or after treatment; urine routine test, faeces routine test, AFP, $\gamma$-GT,LDH, liver function and kidney function are checked once two weeks during or after treatment; Immunology index, ECG, heart function, chest X-Ray are checked once four weeks during or after treatment; the Examination of imageology is carried on once eight weeks during or after treatment, the examination can be carried on at any time when needed.

III Therapeutic efficacy assessment criterions

1.Therapeutic efficacy assessment criterions of the tumor foci:

(1)Complete Remission(CR): tumor disappeared and maintained for more than 1 month.
(2)Partial Reinission(PR): product of two niaximum diameters of tumor minimized more than 50%, and maintained for more than 1 month.
(3)Stable disease (SD) : product of two maximum diameters minimized less than 50%, increased by no more than 25%, maintained for more than 1 month.
(4) Progression disease (PD): product of two maximum diameters increased by more than 25%.

Total remission rate=CR+PR
1. survival quality assessment criterions:
According to Karnofsky score criterions, it is compared before and after treatment.

| Karnofsky score criterions: | |
| --- | --- |
| Normal,no discomfort or sign of disease | 100 |
| Normal activity, mild sign of disease | 90 |
| Nearly normal activity, certain symptoms or signs | 80 |
| Self care, can not maintain normal activity and work | 70 |
| Life need help occasionally, but can meet most individual demands | 60 |
| Need many help and medical care | 50 |
| Losing living ablities, need special help and care | 40 |
| Losing living ablities sevely, need treatment in hospital,no death threat temporarily | 30 |
| Badly ill, need to be kept in hospital and given powerful Supportive treatment | 20 |
| In great danger | 10 |
| Death | 0 |

IV. Handling and summarizing of clinical trail data

Collecting all data, inputing the medical history into computers, constructing database using EPI INF06 software, making statistical treatment and analysis, writing summary of clinical trails, making objective assessment about clinical therapeutic effects and safety of the invented Traditional Chinese Medicine composition treatment on primary hepatic carcinoma and gastric carcinoma.

**Results**

100 eligible cases, all belong to group treated with the invented composition solely;Of which 41 cases of primary hepatic carcinoma, 59 cases of gastric carcinoma diagnosed by western medicine, while being considered as the symptom of stagnation of poison

I .General condition

1. Sex

Table 1 sex construction

|  | male | female | total |
|---|---|---|---|
| Primary hepatic carcinoma | 33 | 8 | 41 |
| Gastric carcinoma | 44 | 15 | 59 |

2. Age

Table 2 age block

|  | 34-40 | 41-50 | 51-60 | 61-78 | $x\pm$SD |
|---|---|---|---|---|---|
| Primary hepatic carcinoma | 6 | 10 | 12 | 13 | 53.9$\pm$10.2 |
| Gastric carcinoma | 4 | 14 | 13 | 28 | 56.5$\pm$9.5 |

3. course of disease

Table 3 course of disease (month)

|  | case* | 1-3 | 4-6 | 7-12 | 13-50 |
|---|---|---|---|---|---|
| Primary hepatic carcinoma | 36 | 26 | 3 | 4 | 3 |
| Gastric carcinoma | 57 | 21 | 10 | 12 | 14 |

● data of 5 cases of primary hepatic carcinoma and 2 cases of gastric carcinoma are lost.

4. Past treatment

Table 4 past treatment *

|  | case* | untreated | operation | TCM | TAI |
|---|---|---|---|---|---|
| Primary hepatic carcinoma | 40 | 27 | 6 | 2 | 5 |
| gastric carcinoma | 58 | 26 | 17 | 3 | 12 |

* data of 1 case of primary hepatic carcinoma and 1 case of gastric carcinoma are lost

5. Foci type and location

Among 41 cases of primary hepatic carcinoma, massive type, nodular type and diffuse type are 17 cases (41.5%),17 cases (41.5%) and 7 cases(17.1%) respectively, 3 cases (7.3%) of tumors located in left hepatic lobe, 29 cases (70.7%) of tumors located in right hepatic lobe, 9 cases (22%) of tumors located in both lobes.

Among 59 cases of gastric carcinoma, carcinoma located in upper region, middle region , lower region or other regions of stomach are 4 cases (6.8%), 8 cases (13.6%),18 cases (30.5%), 21cases(35.6%)respectively, 7 cases(11.9 %) of carcinoma located in multiple regions of stomach.

6. Clinical stage

Table 5 clinical stage

|  | cases | I | II | III | IV |
|---|---|---|---|---|---|
| Primary hepatic carcinoma | 41 | 2 | 20 | 19 | 0 |
| Gastric carcinoma | 59 | 1 | 1 | 19 | 37 |

7. Karnofsky score before treatment

Table 6 Karnofsky score before treatment

|  | cases | 50-69 | 70-79 | 80-90 | $\overline{x}\pm s$ |
|---|---|---|---|---|---|
| Primary hepatic carcinoma | 41 | 9 | 21 | 11 | 69.8±13.9 |
| Gastric carcinoma | 59 | 24 | 27 | 8 | 65.9±8.9 |

8. Body weight before treatment

Table 7 Body weight(kg) before treatment

|  | cases | 36-50 | 51-60 | 61-70 | 71-76 | $\overline{x}\pm s$ |
|---|---|---|---|---|---|---|
| Primary hepatic carcinoma | 41 | 3 | 15 | 20 | 3 | 61.0±7.9 |
| Gastric carcinoma | 59 | 13 | 26 | 13 | 4 | 56.4±8.8 |

9. Appetite before treatment

Table 8 appetite before treatment (taels/day)

|  | cases | 1-4 | 4-5.9 | 6-7.9 | 8-12 | $\overline{x}\pm$ |
|---|---|---|---|---|---|---|
| Primary hepatic carcinoma | 41 | 9 | 12 | 13 | 7 | 5.7±1.6 |
| Gastric carcinoma | 59 | 19 | 8 | 24 | 6 | 5.3±1.9 |

* data of 1 case of gastric carcinoma are lost.

10. AFP test before treatment of primary hepatic carcinoma

Table 9 AFP (ug/ml) test before treatment of primary hepatic carcinoma

|  | cases | <30 | 30-399 | ≥400 | $\overline{x}\pm s$ |
|---|---|---|---|---|---|
| primary hepatic carcinoma | 39 | 2 | 3 | 34 | 381.69±126.13 |

11. γ-GT test before treatment of primary hepatic carcinoma

Table 10 γ-GT test before treatment of primary hepatic carcinoma

|  | cases | $\overline{x}\pm s$(n) |
|---|---|---|
| primary hepatic carcinoma | 22 | 200.2±103.7 (22) |

II. Therapeutic efficacy

1. Total efficacy

Table 11 total therapeutic efficacy

| disease | cases* | CR(%) | PR(%) | SD(%) | PD(%) |
|---|---|---|---|---|---|
| primary hepatic carcinoma | 41 | 0(0.0%) | 1(2.4%) | 34(82.9%) | 6(14.6%) |
| Gastric carcinoma | 59 | 0(0.0%) | 6(10.2%) | 49(83.0%) | 4(6.8%) |

CR,PR,SD,PD of primary hepatic carcinoma, after treatment are 0, 2.4%, 82.9%, 14.6% respectively.
CR,PR,SD,PD of gastric carcinoma after treatment are 0, 10.2%, 83.0%, 6.8% respectively.

2. Follow-up life span, survival rate after treatment

Table 12 Deaths 8 weeks after treatment

| disease | cases | survival | death (cause) | | | | |
|---|---|---|---|---|---|---|---|
| | | | hepatic coma | hepatorrhe xis | upper gastrointestinal bleeding | failure | others |
| primary hepatic carcinoma | 41 | 39 | 0 | 0 | 0 | 1 | 1 |
| gastric carcinoma | 59 | 45 | 0 | 0 | 1 | 12 | 1 |

Table 13 Deaths 1.5 year after treatment

| disease | cases | survival | death (cause) | | | | | |
|---|---|---|---|---|---|---|---|---|
| | | | hepatic coma | Hepatorrhe -xis | upper gastrointestinal bleeding | failure | hepatorenal syndrome | others |
| primary hepatic carcinoma | 41 | 8 | 5 | 4 | 8 | 10 | 2 | 4 |
| Gastric carcinoma | 59 | 25 | 0 | 0 | 3 | 28 | 0 | 3 |

Table 14 life span(month), survival rate 1.5 years after treatment (I)

| disease | cases | Complete data cases | censored | %censored |
|---|---|---|---|---|
| primary hepatic carcinoma | 41 | 33 | 8 | 19.5% |
| Gastric carcinoma | 59 | 34 | 25 | 42.4% |

Table 15 life span(month), survival rate 1.5 years after treatment (II)

| disease | cases | Average survival time (month) | Median Survival Time (month) | 1 year survival rate | |
|---|---|---|---|---|---|
| | | $\overline{x} \pm se$ | $\overline{x} \pm se$ | % | standard error |
| primary hepatic carcinoma | 41 | 7.7±0.9 | 5.0±1.3 | 16.5 | 6.0 |
| Gastric carcinoma | 59 | 10.7±0.8 | 11.0±1.5 | 33.0 | 7.7 |

3. Comparison of changes of tumor foci size before and after treatment

Table 16 Comparison of changes of tumor foci size[$] before and after treatment

| disease | before treatment | after treatment | difference (after-before) |
|---|---|---|---|
| | $\overline{x} \pm s$(n) | $\overline{x} \pm s$(n) | $\overline{x} \pm s$ (n) |
| primary hepatic carcinoma | 39.4±42.9(37) | 46.5±53.1(36) | 6.4±29.6(36)* |
| Gastric carcinoma | 19.2±21.1(59) | 15.8±14.4(59) | -3.4±12.0(59)[#] |

$ Size of tumor foci: product of two perpendicular maximum diameters or sum of products of multiple foci (cm×cm).
*Primary hepatic carcinoma, t=1.30, P=0.203
# Gastric carcinoma, t=2.17, P=0.034

For Primary hepatic carcinoma patients, there is no notable significance in the difference of tumor foci size before and after treatment

For Gastric carcinoma patients, there is notable significance in the difference of tumor foci size before and after treatment.

4. Changes of Karnofsky score after treatment

Table 17 Changes of Karnofsky score after treatment

| disease | before treatment | after treatment | difference (after-before) |
|---|---|---|---|
| | $\overline{x}\pm s$(n) | $\overline{x}\pm s$(n) | $\overline{x}\pm s$(n) |
| Primary hepatic carcinoma | 69.8±13.9(41) | 78.1±8.7(31) | 7.7±16.1(31)* |
| Gastric carcinoma | 65.9±8.9(59) | 77.1±9.9(56) | 11.6±8.0(56)# |

* Primary hepatic carcinoma ,t=2.68, P=0.012
# Gastric carcinoma, t=10.80, P=0.000

For Primary hepatic carcinoma patients, there is notable significance in the difference of Karnofsky score before and after treatment

For Gastric carcinoma patients, there is notable significance in the difference of Karnofsky score before and after treatment

5. Changes of body weight after treatment

Table 18 Changes of body weight after treatment (Kg)

| disease | before treatment | after treatment | difference (after-before) |
|---|---|---|---|
| | $\overline{x}\pm s$(n) | $\overline{x}\pm s$(n) | $\overline{x}\pm s$(n) |
| Primary hepatic carcinoma | 61.0±7.9(41) | 63.1±7.3(31) | 0.9±1.4(31)* |
| Gastric carcinoma | 56.4±8.8(59) | 56.9±8.3(55) | 1.0±3.6(52)# |

*Primary hepatic carcinoma ,t=3.50, P=0.001
# Gastric carcinoma, t=2.10, P=0.040

For Primary hepatic carcinoma patients, there is notable significance in the difference.of body weight before and after treatment

For Gastric carcinoma patients,there is notable significance in the difference.of body weight before and after treatment,

6. Changes of appetite after treatment

Table 19 Changes of appetite after treatment (taels/day)

| disease | before treatment | after treatment | difference (after-before) |
|---|---|---|---|
| | $\overline{x}\pm s$(n) | $\overline{x}\pm s$(n) | $\overline{x}\pm s$(n) |
| primary hepatic carcinoma | 5.7±1.6(41) | 6.7±1.5(31) | 1.2±1.4(31)* |
| gastric carcinoma | 5.3±1.9(59) | 6.7±2.7(58) | 1.5±1.7(56)# |

* Primary hepatic carcinoma, t=4.77, P=0.000
# Gastric carcinoma, t=6.47, P=0.000

For Primary hepatic carcinoma patients, there is notable significance in the difference of appetite before and after treatment.

For Gastric carcinoma patients, there is notable significance in the difference of appetite before and after treatment.

7.Improvements of symptoms and signs after treatment

Table 20 Improvement of fatigue after treatment*

| disease | cases | aggravation | no change | Improved 1 grade | Improved 2 grades | Improved 3 grades |
|---|---|---|---|---|---|---|
| Primary hepatic carcinoma | 30 | 1 | 14 | 7 | 8 | 0 |

(continued)

| disease | cases | aggravation | no change | Improved 1 grade | Improved 2 grades | Improved 3 grades |
|---|---|---|---|---|---|---|
| Gastric carcinoma | 58 | 2 | 20 | 19 | 10 | 7 |

\* Data of 11 cases of primary hepatic carcinoma and 1 case of gastric carcinoma are lost

Improved 1 grade: lowered 1 grade after treatment compared with before treatment, for example, fatigue is difficult to recover after activities, while it is improved to be easy to recover post-treatment.

Improved 2 grades : lowered 2 grades after treatment compared with before treatment,for example, feeling fatigue when rest, while post-treatment fatigue is easy to recover after activities.

Improved 3 grades: lowered 3 grades after treatment compared with before treatment,for example, Lying in bed, while post-treatment fatigue is easy to recover after activities.

For primary hepatic carcinoma patients, improvement rate of fatigue is 50.0%.

For gastric carcinoma patients, improvement rate of fatigue is 62.1%.

8. Improvements of symptoms and signs after treatment

Table 21 improvement of epigastralgia (gastric carcinoma) after treatment

| disease | cases | aggravation | no change | Improved 1 grade | Improved 2 grades | Improved 3 grades |
|---|---|---|---|---|---|---|
| gastric carcinoma | 55 | 3 | 14 | 18 | 16 | 4 |

\* Data of 4 cases of gastric carcinoma are lost.

Improvement rate of epigastralgia is 69.1%

Table 22 improvement of appetite after treatment (gastric carcinoma patients)\*

| disease | cases | aggravation | no change | Improved 1 grade | Improved 2 grades | Improved 3 grades |
|---|---|---|---|---|---|---|
| gastric carcinoma | 55 | 3 | 21 | 16 | 12 | 3 |

\* Data of 4 cases of gastric carcinoma are lost.

Improvement rate of appetite after treatment is 56.4% .

Table 23 improvement of dry mouth and thirsty after treatment\*

| disease | cases | aggravation | no change | Improved 1 grade | Improved 2 grades | Improved 3 grades |
|---|---|---|---|---|---|---|
| primary hepatic carcinoma | 31 | 0 | 20 | 8 | 2 | 1 |
| gastric carcinoma | 58 | 1 | 44 | 9 | 4 | 0 |

\*Data of 10 cases of primary hepatic carcinoma and 1 case of gastric carcinoma are lost

Improved 1 grade: thirst lowered 1 grade after treatment, for example, dry mouth and throat,no desire for drink, improved to only dry mouth after treatment.

Improved 2 grades: thirst lowered 2 grades after treatment, for example, dry mouth and throat, desire for drink, improved to only dry mouth.

Improved 3 grades: thirst lowered 3 grades after treatment, for example,dry mouth and sorethroat, improved to only dry mouth.

For primary hepatic carcinoma patients, improvement rate of thirst is 35.5%.
For gastric carcinoma patients, improvement rate of thirst is 22.4%.

Table 24 Improvement of bitter mouth after treatment

| disease | cases | aggravation | no change | Improved 1 grade | Improved 2 rades | Improved 3 grades |
|---|---|---|---|---|---|---|
| primary hepatic carcinoma | 31 | 0 | 20 | 10 | 1 | 0 |
| gastric carcinoma | 57 | 0 | 45 | 9 | 3 | 0 |

\* Data of 10 primary hepatic carcinoma patients and 2 cases of gastric carcinoma are lost

For primary hepatic carcinoma patients, improvement rate of bitter mouth is 35.5%.
For gastric carcinoma patients, improvement rate of bitter mouth is 21.1%.

Table 25 Improvement of spontaneous perspiration after treatment*

| disease | cases | aggravation | no change | Improved 1 grade | Improved 2 grades | Improved 3 grades |
|---|---|---|---|---|---|---|
| primary hepatic carcinoma | 31 | 0 | 24 | 5 | 1 | 1 |
| gastric carcinoma | 57 | 2 | 42 | 8 | 5 | 0 |

*Data of 10 cases of primary hepatic carcinoma and 2 cases of gastric carcinoma are lost.

Improved 1 grade: spontaneous perspiration lowered 1 grade after treatment, for example,perspire immediately after activities, improved to perspire occasionally after activities.
Improved 2 grades: spontaneous perspiration lowered 2 grades after treatment, for example,perspire when rest, improved to perspire occasionally after activities.
Improved 3 grades: spontaneous perspiration lowered 3 grades after treatment, for example,perspire vehemently , improved to perspire occasionally after activities.

For primary hepatic carcinoma patients, improvement rate of spontaneous perspiration is 22.6%.
For gastric carcinoma patients, improvement rate of spontaneous perspiration is 22.8%.

Table 26 Improvement of night sweat after treatment*

| disease | cases | aggravation | no change | Improved 1 grade | Improved 2 grades | Improved 3 grades |
|---|---|---|---|---|---|---|
| primary hepatic carcinoma | 31 | 0 | 24 | 7 | 0 | 0 |
| gastric carcinoma | 57 | 3 | 44 | 5 | 4 | 1 |

*Data of 10 cases of primary hepatic carcinoma and 2 cases of gastric carcinoma are lost.

Improved 1 grade: night sweat lowered 1 grade after treatment, for example, night sweat usuaally, improved to night sweat occasionally.
Improved 2 grades: night sweat lowered 2 grades after treatment, for example, night sweat every night, improved to night sweat occasionally.
Improved 3 grades: night sweat lowered 3 grades after treatment, for example, night sweat can wet cloth, improved to night sweat occasionally.

For primary hepatic carcinoma patients, improvement rate of night sweat is 22.6%.
For gastric carcinoma patients, improvement rate of night sweat is 15.8%.

Table 27 Improvement of upset and tantrum after treament*

| disease | cases | aggravation | no change | Improved 1 grade | Improved 2 grades | Improved 3 grades |
|---|---|---|---|---|---|---|
| primary hepatic carcinoma | 31 | 0 | 26 | 5 | 0 | 0 |
| gastric carcinoma | 57 | 2 | 37 | 15 | 3 | 0 |

* Data of 10 cases of primary hepatic carcinoma and 2 cases of gastric carcinoma are lost.

Improved 1 grade: upset and tantrum lowered 1 grade after treatment, for example, upset and self-uncontrollable, improved to be upset and self-controllable.
Improved 2 grades: upset and tantrum lowered 2 grades after treatment, for example, burning sensation of five centres and tantrum, improved to be upset and self-controllable.

For primary hepatic carcinoma patients, improvement rate of upset and tantrum is 16.1 %.
For gastric carcinoma patients, improvement rate of upset and tantrum is 31.6%.

Table 28 Improvement of dizziness after treatment *

| disease | cases | aggravation | no change | Improved 1 grade | Improved 2 grades | Improved 3 grades |
|---|---|---|---|---|---|---|
| primary hepatic carcinoma | 31 | 1 | 26 | 4 | 0 | 0 |
| gastric carcinoma | 57 | 3 | 36 | 14 | 4 | 0 |

* Data of 10 cases of primary hepatic carcinoma and 2 cases of gastric carcinoma are lost.

Improved 1 grade: dizziness lowered 1 grade after treatment, for example, dizzy usually , improved to dizzy occasionally.
Improved 2 grades: dizziness lowered 2 grades after treatment, for example, dizzy persistently , improved to dizzy occasionally.

For primary hepatic carcinoma patients, improvement rate of dizziness is 12.9%.
For gastric carcinoma patients, improvement rate of dizziness is 31.6%.

Table 29 Improvement of jaundice after treatment*

| disease | cases | aggravation | no change | Improved 1 grade | Improved 2 grades | Improved 3 gradea |
|---|---|---|---|---|---|---|
| primary hepatic carcinoma | 31 | 2 | 28 | 1 | 0 | 0 |
| gastric carcinoma | 57 | 0 | 57 | 0 | 0 | 0 |

* Data of 10 cases of primary hepatic carcinoma and 2 cases of gastric carcinoma are lost.

Improved 1 grade: jaundice lowered 1 grade after treatment, for example, albuginea oculi and skin stained yellow, improved to only albuginea oculi mildly stained yellow.

For primary hepatic carcinoma patients, improvement rate of jaundice is 3.2%.
For gastric carcinoma patients, improvement rate of jaundice is 0%.

Table 30 Improvement of cancer pain after treatment*

| disease | cases | aggravation | no change | Improved 1 grade | Improved 2 grades | Improved 3 grades |
|---|---|---|---|---|---|---|
| primary hepatic carcinoma | 31 | 1 | 14 | 8 | 7 | 1 |

(continued)

| disease | cases | aggravation | no change | Improved 1 grade | Improved 2 grades | Improved 3 grades |
|---|---|---|---|---|---|---|
| gastric carcinoma | 57 | 1 | 35 | 12 | 8 | 1 |

*Data of 10 cases of primary hepatic carcinoma and 2 cases of gastric carcinoma are lost.

Improved 1 grade : cancer pain lowered 1 grade after treatment, for example,moderate cancer pain(bearable, no upset), improved to mild cancer pain(discomfort, no upset)

Improved 2 grades: cancer pain lowered 2 grades after treatment, for example, severe cancer pain(active body position,demand for painkiller), improved to mild cancer pain(discomfort, no upset)

Improved 3 grades: cancer pain lowered 3 grades after treatment, for example, uncontrollable pain(upset, passive position, need painkiller to relieve),. improved to mild cancer pain(discomfort, no upset)

For primary hepatic carcinoma patients, improvement rate of cancer pain is 51.6%.
For gastric carcinoma patients, improvement rate of cancer pain is 36.8%.

Table 31 Improvement of abdominal distension after treatment*

| disease | cases | aggravation | no change | Improved 1 grade | Improved 2 grades | Improved 3 grades |
|---|---|---|---|---|---|---|
| primary hepatic carcinoma | 31 | 1 | 16 | 10 | 3 | 1 |
| gastric carcinoma | 57 | 0 | 26 | 19 | 10 | 2 |

* Data of 10 cases of primary hepatic carcinoma and 2 cases of gastric carcinoma are lost.

Improved 1 grade: abdominal distension lowered 1 grade after treatment, for example, abdominal distension, unrelieved after passage of gas by anus, improved to relief after passage of gas by anus after treatment, no ascites.

Improved 2 grades: abdominal distension lowered 2 grades after treatment, for example, obvious abdominal distension with small or moderate volume of ascites, improved to abdominal distension , relief after passage of gas by anus, no ascites after treatment.

Improved 3 grades: abdominal distension lowered 3 grades after treatment, for example, obvious abdominal distension with large volume of ascites, improved to abdominal distension , relief after passage of gas by anus, no ascites after treatment.

For primary hepatic carcinoma patients, improvement rate of abdominal distension is 45.2%.
For gastric carcinoma patients, improvement rate of abdominal distension is54.4%.

Table 32 Improvement of diarrhea after treatment*

| disease | cases | aggravation | no change | Improved 1 grade | Improved 2 grades | Improved 2 grades |
|---|---|---|---|---|---|---|
| primary hepatic carcinoma | 31 | 0 | 26 | 2 | 3 | 0 |
| gastric carcinoma | 57 | 2 | 54 | 1 | 0 | 0 |

*Data of 10 cases of primary hepatic carcinoma and 2 cases of gastric carcinoma are lost.

Improved 1 grade: diarrhea lowered 1 grade after treatment, for example, bearable (>2d), improved to diarrhea transiently (<2d) after treatment.

Improved 2 grades: diarrhea lowered 2 grades after treatment, for example, unbearable diarrhea , improved to diarrhea transiently (<2d) after treatment.

For primary hepatic carcinoma patients, improvement rate of diarrhea is 16.1%.
For gastric carcinoma patients, improvement rate of diarrhea is 1.8%.

9. Disappearance of main symptoms and signs after treatment

Table 33 Disappearance of main symptoms after treatment

| symptoms | disease | total cases | beginning cases | disappeared cases | disappearance rate |
|---|---|---|---|---|---|
| fatigue | primary hepatic carcinoma | 31 | 26 | 10 | 61.5% |
| | gastric carcinoma | 58 | 50 | 18 | 36.0% |
| dry mouth and thirst bitter mouth | primary hepatic carcinoma | 31 | 20 | 7 | 35.0% |
| | gastric carcinoma | 58 | 17 | 11 | 64.7% |
| | primary hepatic carcinoma | 31 | 15 | 9 | 60.0% |
| | gastric carcinoma | 58 | 13 | 9 | 69.2% |
| spontaneous perspiration night sweat | primary hepatic carcinoma | 30 | 12 | 6 | 50.0% |
| | gastric carcinoma | 58 | 17 | 10 | 58.8% |
| | primary hepatic carcinoma | 31 | 13 | 7 | 53.8% |
| | gastric carcinoma | 58 | 12 | 10 | 83.3% |
| upset and tantrum | primary hepatic carcinoma | 31 | 10 | 5 | 50.0% |
| dizziness | gastric carcinoma | 58 | 20 | 15 | 75.0% |
| | primary hepatic carcinoma | 31 | 7 | 3 | 42.9% |
| | gastric carcinoma | 58 | 24 | 14 | 58.3% |
| jaundice | primary hepatic carcinoma | 31 | 10 | 1 | 10.0% |
| | gastric carcinoma | 58 | 2 | 0 | 0% |
| cancer pain | primary hepatic carcinoma | 31 | 23 | 13 | 56.5% |
| | gastric carcinoma | 58 | 30 | 14 | 46.7% |
| abdominal distension | primary hepatic carcinoma | 31 | 27 | 10 | 37.0% |
| diarrhea | gastric carcinoma | 57 | 41 | 16 | 39.0% |
| | primary hepatic carcinoma | 31 | 5 | 5 | 100% |
| | gastric carcinoma | 58 | 3 | 1 | 33.3% |

\* Data of 10 or 11 cases of primary hepatic carcinoma and 1 or 2 cases of gastric carcinoma are lost.

10. Changes of White Blood Cells(WBC) after treatment

Table 52 Changes of White Blood Cells(WBC) after treatment*

| disease | cases | aggravation | no change | Improved 1 grade | Improved 2 grades | Improved 3 grades |
|---|---|---|---|---|---|---|
| primary hepatic carcinoma | 29 | 3 | 25 | 1 | 0 | 0 |
| gastric carcinoma | 55 | 2 | 50 | 1 | 0 | 2 |

\* Grade WBC accurately according to "WHOgrade crterions for acute and subacute toxicity reactions of the anti-caner drugs"..

Aggravation: WBC grade increased more than 1 grade after treatment compared with before treatment

Improved 1 grade: WBC grade lowered 1 grade after treatment compared with before treatment.
Improved 2 grades: WBC grade lowered 2 grades after treatment compared with before treatment.
Improved 3 grades: WBC grade lowered 3 grades after treatment compared with before treatment.

For primary hepatic carcinoma patients, improvement rate of WBC is 3.5%, aggravation rate of WBC is 10.3%.
For gastric carcinoma patients, improvement rate of WBC is 5.5%, aggravation rate of WBC is 3.6%.
11. Changes of granulocytes after treatment

Table 35 Changes of granulocytes number after treatment*

| disease | cases | aggravation | no change | Improved 1 grade | Improved 2 grades | Improved 3 grades |
|---|---|---|---|---|---|---|
| primary hepatic carcinoma | 17 | 0 | 15 | 2 | 0 | 0 |
| gastric carcinoma | 53 | 1 | 52 | 0 | 0 | 0 |

*Gradie granulocytes number accurately according to "WHO grade criteriions for acute and subacute toxicity reactions of the anti-cancer drugs".

Aggravation: granulocytes number grade increased more than 1 grade after treatment compared with before treatment

Improved 1 grade: granulocytes number grade lowered 1 grade after treatment compared with before treatment.

Improved 2 grades: granulocytes number grade lowered 2 grades after treatment compared with before treatment.

Improved 3 grades: granulocytes number grade lowered 3 grades after treatment compared with before treatment.

For primary hepatic carcinoma patients, improvement rate of granulocytes number is 11.8%, aggravation rate of granulocytes number is 0%.
For gastric carcinoma patients, improvement rate of granulocytes is 0%, aggravation rate of granulocytes number is 1.9%.
12. Changes of hemoglobin number after treatment

Table 36 Changes of hemoglobin number after treatment *

| disease | cases | aggravation | no change | Improved 1 grade | Improved 2 grades | Improved 3 grades |
|---|---|---|---|---|---|---|
| primary hepatic carcinoma | 31 | 5 | 24 | 2 | 0 | 0 |
| gastric carcinoma | 55 | 9 | 36 | 8 | 1 | 1 |

*Grade hemoglobin number accurately according to "WHOgrade criterions for acute and subacute toxicity reactions of the anti-cancer drugs".

Aggravation: hemoglobin number grade increased more than 1 grade after treatment compared with before treatment

Improved 1 grade: hemoglobin number grade lowered 1 grade after treatment compared with before treatment.

Improved 2 grades: hemoglobin number grade lowered 2 grades after treatment compared with before treatment.

Improved 3 grades: hemoglobin number grade lowered 3 grades after treatment compared with before treatment.

For primary hepatic carcinoma patients, improvement rate of hemoglobin number is 6.5% , aggravation rate of hemoglobin number is 16.1 %.
For gastric carcinoma patients, improvement rate of hemoglobin number is 18.2% , aggravation rate of hemoglobin number is 16.4%.
13.Changes of platelets number after treatment

Table 37 Changes of platelets number after treatment *

| disease | cases | aggravation | no change | Improved 1 grade | Improved 2 grades | Improved 3 grades |
|---|---|---|---|---|---|---|
| primary hepatic carcinoma | 29 | 1 | 25 | 3 | 0 | 0 |
| gastric carcinoma | 55 | 1 | 51 | 1 | 1 | 1 |

* Grading platelets number accurately according to "WHOgrade criterions for acute and subacute toxicity reactions of the anti-cancer drugs".

Aggravation: platelets number grade increased more than 1 grade after treatment compared with before treatment

Improved 1 grade: platelets number grade lowered 1 grade after treatment compared with before treatment.

Improved 2 grades: platelets number grade lowered 2 grades after treatment compared with before treatment.

Improved 3 grades: platelets number grade lowered 3 grades after treatment compared with before treatment.

For primary hepatic carcinoma patients, improvement rate of platelets number is 10.3%, aggravation rate of platelets number is 3.5%.

For gastric carcinoma patients, improvement rate of platelets number is 5.5%, aggravation rate of platelets number is 1.8%.

14. Comparison of immunologic index CD3 before and after treatment

Table 38 Comparison of immunologic index CD3 before and after treatment

| disease | before treatment | after treatment | difference (after-before) |
|---|---|---|---|
| | $\overline{x}\pm s$(n) | $\overline{x}\pm s$(n) | $\overline{x}\pm s$(n) |
| primary hepatic carcinoma | 53.5$\pm$9.7(35) | 55.9$\pm$9.8(25) | 1.8$\pm$2.1(25)* |
| gastric carcinoma | 60.2$\pm$12.7(29) | 61.6$\pm$14.0(29) | 1.3$\pm$9.2(29)[#] |

* Primary hepatic carcinoma, t=4.18, P=0.000.
# Gastric carcinoma, t=0.76, P=0.452.

For Primary hepatic carcinoma patients, there is notable significance in the difference of immunologic index CD3 before and after treatment.

For Gastric carcinoma patients, there is no notable significance in the difference.of immunologic index CD3 before and after treatment.

15. Comparison of immunologic index CD4 before and after treatment

Table 39 Comparison of immunologic index CD4 before and after treatment

| disease | before treatment | after treatment | difference(after-before) |
|---|---|---|---|
| | $\overline{x}\pm s$(n) | $\overline{x}\pm s$(n) | $\overline{x}\pm s$(n) |
| primary hepatic carcinoma | 34.7$\pm$5.0(35) | 35.1$\pm$3.9(25) | 1.4$\pm$1.4(25)* |
| gastric carcinoma | 40.5$\pm$9.5(29) | 37.9$\pm$8.4(29) | -2.6$\pm$5.4(29)[#] |

*Primary hepatic carcinoma, t=5.23, P=0.000.
# Gastric carcinoma, t=2.65, P=0.013.

For Primary hepatic carcinoma patients, there is notable significance in the difference of immunologic index CD4 before and after treatment.

For Gastric carcinoma patients, there is notable significance in the difference of immunologic index CD4 before and after treatment.

16. Comparison of immunologic index CD8 before and after treatment

Table 40 Comparasion of immunologic index CD8 before and after treatment

|  | before treatment | after treatment | difference (after-before) |
|---|---|---|---|
|  | $\overline{x}\pm s$(n) | $\overline{x}\pm s$(n) | $\overline{x}\pm s$(n) |
| primary hepatic carcinoma | 30.2±5.8(35) | 29.2±4.5(25) | -1.1±3.9(25)* |
| gastric carcinoma | 31.2±8.1(29) | 28.9±8.3(29) | -2.3±4.4(29)# |

* Primary hepatic carcinoma, t=1.47, P=0.154.
# Gastric carcinoma, t=2.75, P=0.010.

For Primary hepatic carcinoma patients, there is no notable significance in the difference of immunologic index CD8 before and after treatment.

For Gastric carcinoma patients, there is notable significance in the difference of immunologic index CD8 before and after treatment.

17. Comparison of immunologic index CD4/CD8 before and after treatment

Table 41 Comparison of immunologic index CD4/CD8 before and after treatment

|  | before treatment | after treatment | difference (after-before) |
|---|---|---|---|
|  | $\overline{x}\pm s$(n) | $\overline{x}\pm s$(n) | $\overline{x}\pm s$(n) |
| primary hepatic carcinoma | 1.23±0.55(34) | 1.25+0.35(25) | 0.11±0.26(33)* |
| gastric carcinoma | 1.35±0.44(29) | 1.46±0.47(29) | 0.00±0.31(29)# |

* Primary hepatic carcinoma, t=2.20, P=0.037.
# Gastric carcinoma, t=1.51, P=0.145.

For Primary hepatic carcinoma patients, there is notable significance in the difference of immunologic index CD4/CD8 before and after treatment.

For Gastric carcinoma patients, there is no notable significance in the difference of immunologic index CD4/CD8 before and after treatment.

18. Comparison of primary hepatic carcinoma γ-GT before and after treatment

Table 42 Comparison of primary hepatic carcinoma γ-GT before and after treatment

| disease | before treatment | after treatment | difference(after-before) |
|---|---|---|---|
|  | $\overline{x}\pm s$(n) | $\overline{x}\pm s$(n) | $\overline{x}\pm s$(n) |
| primary hepatic carcinoma | 200.2±103.7(22) | 207.6±101.8(58) t=1.437, P=0.165; | -7.4±24.2(22) |

III.Results of imageology examination after treatment:

1. B type ultrasonic examination:

Totally there are 36 cases of primary hepatic carcinoma received B type ultrasonic examination, all show abnormalities, after treatment, rechecked and still show abnormalities. 37 cases of gastric carcinoma patients received B type ultrasonic examination, of which, 17 cases are normal, 20 cases show abnormalities, rechecked after treatment, no obvious changes.

2. CT and MRI examinations

14 cases of primary hepatic carcinoma patients received CT or MRI examinations, 4 cases are normal, 10 cases are abnormal, rechecked after treatment, no obvious changes. 8 cases of gastric carcinoma patients received CT or MRI examinations, all are abnormal, rechecked after treatment, 1 case is normal, 7 cases are abnormal.

IV. Safety Inspection

All safety indexes are normal before treatment, abnormalities appeared after treatment are recorded in following table

| doubtful adverse reaction | positive cases/total cases | incidence rate |
|---|---|---|
| Hemoglobin number reduction | 7/96 | 7.3% |
| RBCnumber reduction | 3/93 | 3.2% |
| WBCnumber reduction | 2/95 | 2.1% |
| Granulocytes number reduction | 1/83 | 1.2% |
| Platelet number reduction | 1/95 | 1.1% |
| Bilirubin increase | 0/91 | 0.0% |
| AKP rising | 2/94 | 2.1% |
| GPT rising | 3/93 | 3.2% |
| BUN rising | 1/94 | 1.1% |
| serum creatinine rising | 0/94 | 0.0% |
| Abnormal urine protein | 2/79 | 2.5% |
| Abnormal urine RBC | 5/79 | 6.3% |
| fecal occult blood | 3/80 | 3.8% |

六. Adverse event observations

During the courses of clinical trails, doubtful adverse reaction occurred to many cases, including fever, alopecie, choking, short of breath, non cancer pain, nausea and vomit. constipation and fecal occult blood, see details in following tables

| doubtful adverse events | cases(n=100) | incidence rate |
|---|---|---|
| fever | 7 | 7.0% |
| alopecie | 1 | 1.0% |
| oral cavity ulcer | 0 | 0.0% |
| cutaneous reaction | 0 | 0.0% |
| choking and short of breath | 2 | 2.0% |
| non cancer pain | 3 | 3.0% |
| hypersensitiveness | 0 | 0.0% |
| nausea and vomit | 11 | 11.0% |
| constipation | 22 | 22.0% |
| bleeding | 3 | 3.0% |

During the courses of treatment, doubtful adverse reaction occurred to some patients, such as fever, alopecie, choking, short of breath, non cancer pain, nausea, vomit. constipation and bleeding, the incidence rate is shown in above table. For 36 cases , at least one doubtful adverse reaction appears , total incidence rate of doubtful adverse reaction is 36.0% (36/100) .

Conclusions

100 suitable cases, of which, 41 cases of primary hepatic carcinoma, 59 cases of gastric carcinoma.are considered as symptoms of stagnation of poison by Traditional Chinese Medicine

CR, PR, SD and PD rate of treatment on primary hepatic carcinoma are 0, 2.4%, 82.9%, 14.6% respectively; CR, PR, SD and PD rate of treatment on gastric carcinoma are 0, 10.2%,83.0%, 6.8% respectively.

Results of 1.5 years follow-up after treatment show that 1 year survival rates of primary hepatic carcinoma patients and gastric carcinoma patients are 16.5% and 31.7% respectively, median life spans of primary hepatic carcinoma patients and gastric carcinoma patients are 5 months and 11 months respectively, average survival time are 7.7 months and 10.7 months respectively.

Gastric carcinoma patients enrolled into this clinical trails are mainly in advanced stage, of which, stage III, 19 cases (32.2%) ;stage IV, 37 cases (62.7%), above therapeutic efficacy on gastric carcinoma demonstrates that the invented Traditional Chinese Medicine composition has relative better effects on gastic carcinoma (considered as symptom of stagnation of poison by Traditional Chinese Medinine). PR rate is 10.2%, SD rate is 83.0%, 1 year survival rate is 31.7%, median life span is 11 months.

Results of solid tumor foci size assessment indicate:

Comparison of tumor foci size of primary hepatic carcinoma before and after treatment, there is notable significance in the difference.

Comparison of tumor foci size of gastric carcinoma before and after treatment, there is notable significance in the difference. Tumor foci size is minimized obviously after treatment, suggesting the invented Traditional Chinese Medicine composition can minimize tumor foci when used to treat gastric carcinoma (considered as symptom of stagnation of poison by Tradtional Chinese medicine)

Therapeutic effects on main symptoms demonstrate:

After treated with the invented Traditional Chinese Medicine composition, Karnofsky scores, body weights, appetites of the primary hepatic carcinoma patients and gatric carcinoma patients increased obviously compared with before treatment.There is notable significance in their differences.

After treated with the invented Traditional Chinese Medicine composition, symptoms such as fatigue, dry mouth, thirst, dizziness, gastric discomfortableness, loss of apptite, bitter taste of mouth, spontaneous perspiration, night sweat, upset, tantrum, choking, short of breath, cancer pain, abdominal distension, are all improved compared with before treatment.

Above results indicate that the invented Traditional Chinese Medicine composition can improve survival qualities of patients and ameliorate clinical symptoms of patients when used to treat primary hepatic carcinoma and gastric carcinoma (considered as ymptom of stagnation of poison by Traditonal Chinese Medicine)

Results of laboratory examination show:

As to immunological index, Comparison results before and after treatment demonstrate that changes of CD3,CD4,CD4/CD8 of the primary hepatic carcinoma patients have significant difference;while CD4,CD8 of the gastric carcinoma patients are lower than those of before treatment, CD4/CD8 has no obvious changes. It suggests that the invented Traditional Chinese Medicine composition can enhance cellular immune functions of primary hepatic carcinoma patients, while it may not enhance cellular immune functions of gastric carcinoma patients.

WBC, granulocytes, platelets of primary hepatic carcinoma patients and gastric patients have no obvious augmentations after treatment.

In summary, results of 100 cases of clinical trails without control demonstrate that the therapeutic effects of the invented Traditional Chinese Medicine composition on primary hepatic carcinoma are not so obvious, PR rate is only 2.4%, however, Karnofsky scores, body weights, appetites of the primary hepatic carcinoma patients increased obviously compared with before treatment, at the same time, symptoms such as fatigue, dry mouth, thirst, cancer pain, dizziness, abdominal distension and cellular immune functions are improved to some extent compared with those of before treatment. The invented Traditional Chinese Medicine composition has better clinical effects on treatment of gastric carcinoma, PR rate is 10.2%, Karnofsky scores, body weights, appetites of the gastric carcinoma patients increased obviously compared with those of before treatment, at the same time, symptoms such as fatigue, gastric discomfort, loss of appetite, dry mouth, thirst, cancer pain, dizziness, abdominal distension are ameliorated compared with before treatment.

During the course of treatment, few patients were damaged in liver, kidney functions and hematopoietic systems, some patients had suspicious adverse reactions including fever, alopecie,choking, short of breath, non cancer pain, nausea and vomit. constipation and bleeding, but they still do not stop using the test composition.

**Application example 2 Clinical trail summary of adjunctive therapy using the invented Traditonal Chinese Medicine composition on primary hepatic carcinoma**

Object and method

I choice of tested object

1. Chinese medicine syndrome diagosis criterion: the same as example 1
2. Western medicine diagnosis criterion: the same as diagnosis criterion of primary hepatic carcinoma mentioned in application example 1.

3. Inclusion criterion

(1) Patients of stage III without taboo of intervention and chemothery.
(2) Others are the same as application example 1

4. Excluding case criterion

The same as application example 1 except condition mentioned in (4)

II. Methods of clinical trails

1.Trail design:

Adopting randomized controlled trial.

Dividing into different groups via simple and random methods, The researchers acquired random number through operating random key (INV,RAN) on Casio(fx-3600p) calculators, making into random allocated cards, sealed in the envelopes, serial number of envelope is the same as that of card, certificated. Suitable cases entered into trail, according to entering time sequence, opened the envelope and divided according to the cards properly.

5.Adiministration manner and dosage:

Treat group

Begin to take the invented Traditional Chinese Medicine composition orally 1 week before the first hepatic artery intervention chemotherapy plus embolism therapy, twice each day, 15ml each time (1 ml containing crude drugs 0.75g), taken once in the early morning and evening, taken with warm water, performing the second hepatic artery intervention chemotherapy plus embolism therapy after 4 weeks, performing twice together. The invented Traditional Chinese Medicine composition is taken for consecutive 2 months.

Control group:

4 weeks after first time hepatic artery intervention chemotherapy plus embolism therapy, performing second hepatic artery intervention chemotherapy plus embolism, performing twice togather.

6. Chemotherapy regimen:

$$
\begin{array}{ll}
\text{DDP} & 60\text{mg/M}^2 \\
\text{ADM} & 40\text{mg/M}^2 \\
\text{5-Fu} & 600\text{mg/M}^2
\end{array}
$$

7. Embolism methods: iodized oil plus gelatin sponge

Dosages of iodized oil and gelatin sponge should be recorded in details in clinical observation label.

8.Course of treatment: 2 months.(the treatment course of Stage II III patients will also be 2 months for the convience of future statistical analysis of data.)

9. Observed items and methods:

The same as application example 1

III. Therapeutic effects assessment criterions.

1. The same as application example 1.
2. Assessments of life spans and survival rates:

life spans after treatment refer to the time from beginning of treatment to death or the last follow-up.

Follow-up need at least 1 year after treatment.

IV. Handling and summarizing of clinical trail data

Collecting all data, inputting into computers, constructing database using EPI INF06 software, making statistical treatment and analysis, summarize clinical trails, making objective assessment about clinical adjunctive therapeutic effects and safety of the invented Traditional Chinese Medicine composition on primary hepatic carcinoma.

Statistical method: enumeration data are analyzed by $X^2$ test, ranked data are analyzed by Wilcoxon rank sum test, two sample means are checked by t-test. Logistic regression analysis is also used to analyze enumeration data. Life span and survival rate are analyzed using life table method and Kaplan-Meier method.

Results

I .General data

183 suitable cases, 124 cases for treat group, 59 cases for control group; all are diagnosed as primary hepatic carcinoma by westen medicine and as symptom of stagnation of poison by Traditional Chinese Medicine. Please refer to the comparable analysis for information about sex, age and disease .

II .Comparable check of two groups

1. Sex construction comparison of two groups

Table 1 sex comparison of two groups

| group | cases | male | female |
|---|---|---|---|
| treat group | 124 | 110 | 14 |
| control | 59 | 51 | 8 |

$X^2=0.195$    P=0.659

Sex comparison of two groups, there is no notable significance in the difference.

2. Age block comparison of two groups

Table 2 Age block (year) comparison of two groups

| group | cases | 21-40 | 41-50 | 51-60 | 61-75 | $\bar{x}\pm s$ |
|---|---|---|---|---|---|---|
| treat group | 124 | 19 | 40 | 42 | 23 | $51.2\pm10.0$ |
| control | 59 | 13 | 19 | 17 | 10 | $50.1\pm10.6$ |

$X^2=1.400$  P=0.705    t=0.650  P=0.516

Age block comparison of two groups, there is no notable significance in the difference.

3. Disease course comparison of two groups

Table 3 Disease course comparison of two groups (month)

| group | cases | 1-3 | 4-6 | 7-12 | 13-64 |
|---|---|---|---|---|---|
| treat group | 118 | 87 | 13 | 6 | 12 |
| Control group | 58 | 40 | 10 | 4 | 4 |

$X^2=1.958$  P=0.581

* 6 cases in treat group and 1 case in control group are not recorded.

Disease course comparison of two groups, there is no notable significance in the difference.

4. Past treatment comparison of two groups

Table 4 Past treatment comparison of two groups

| group | cases* | untreated | operation | TCM | TAI |
|---|---|---|---|---|---|
| treat group | 113 | 85 | 15 | 0 | 13 |
| Control | 58 | 44 | 8 | 3 | 3 |
| | Fisher's exact test | | P=0.07 | | |

* 11 cases in treat group and 1 case in control group are not recorded.

Past treatment comparison of two groups, there is no notable significance in the difference.

5. Foci of Liver Cancer type comparison of two groups

Table 5 Foci of Liver Cancer type comparison of two groups

| group | cases | massive type | nodular type | diffuse type |
|---|---|---|---|---|
| treat group | 124 | 76 | 38 | 10 |
| control | 59 | 35 | 20 | 4 |
| | Fisher's exact test | | P=0.879 | |

Pathological diagnosis comparison of two groups, there is no notable significance in the difference.

6. Clinical stage comparison of two groups

Table 6 Clinical stage comparison of two groups

| group | cases | I | II | III |
|---|---|---|---|---|
| treat group | 124 | 6 | 92 | 26 |
| control | 59 | 1 | 51 | 7 |
| | | Fisher's exact test | | P=0.210 |

Clinical stage comparison of two groups, there is no notable significance in the difference.

7. Tumor foci size comparison of two groups

Table 7 Tumor foci size* comparison of two groups

| group | cases | $\bar{x} \pm s$ |
|---|---|---|
| treat group | 112 | 71.46±63.23 |
| control | 55 | 78.03±57.04 |
| | rank sum test u=1.054 | P=0.292 |

*12 cases in treat group and 4 cases in control groups are massive type, not calculate size. Tumor foci size is measured by product of two perpendicular maximum diameters of tumor or sum of products of multiple foci (cmxcm) .

Tumor foci size comparison of two groups, there is no notable significance in the difference.

Table 8 Portal vein tumor embolism comparison of two groups

| group | cases | negative | positive |
|---|---|---|---|
| treat group | 124 | 104 | 20 |
| control | 59 | 46 | 13 |
| | | $X^2$=0.943 | P=0.331 |

Portal vein tumor embolism comparison of two groups, there is no notable significance in the difference.

8. Karnofsky score, body weight and appetite comparison of two groups

Table 9 Karnofsky score comparison of two groups before treatment

| group | cases | 60-69 | 70-79 | 80-89 | 90-100 | $\bar{x}\pm s$ |
|---|---|---|---|---|---|---|
| treat goup | 124 | 8 | 56 | 55 | 5 | 74.7±7.0 |
| control | 59 | 2 | 22 | 20 | 15 | 78.4±8.4 |
| Fisher's exact test P=0.000 | | | | | | t=3.095 P=0.002 |

Karnofsky score comparison of two groups before treatment, there is notable significance in the difference, Karnofsky score of treat group is lower than that of control group before treatment.

Table 10 Body weight (Kg) comparison of two groups before treatment

| group | cases | 40-50 | 51-60 | 61-70 | 71-87 | $\bar{x}\pm s$ |
|---|---|---|---|---|---|---|
| treat group | 123* | 6 | 27 | 52 | 38 | 66.0±9.1 |
| control | 59 | 1 | 27 | 26 | 5 | 62.6±6.8 |
| Fisher's exact test P=0.000 | | | | | | t'=2.816 P=0.006 |

*Data of 1 case in treat group is lost.

Body weight comparison of two groups before treatment, there is notable significance in the difference, body weight of treat group is higher than that of control group.

Table 11 Appetite (taels/day) comparison of two groups before treatment

| group | cases | 2~4 | 4~5.9 | 6~7.9 | 8~10 | $\bar{x}\pm s$ |
|---|---|---|---|---|---|---|
| treat group | 124 | 14 | 44 | 41 | 25 | 6.0±1.6 |
| control | 59 | 9 | 7 | 31 | 12 | 6.1±1.6 |
| $X^2$=12.358 P=0.006 | | | | | | t=0.464 P=0.643 |

Appetite comparison of two groups before treatment, there is no notable significance in the difference.

9. Main symptoms and signs comparison of two groups before treatment.

Table 12 Fatigue comparison of two groups before treatment*

| group | cases | grade 0 | grade I | grade II | grade III | grade IV |
|---|---|---|---|---|---|---|
| treat group | 124 | 34 | 47 | 27 | 16 | 0 |
| control | 59 | 21 | 30 | 6 | 2 | 0 |
| | | rank sum test | u=2.457 | P=0.014 | | |

*Grade 0: none
Grade I : Be fatigue after activities, easy to recover
Grade II : Be fatigue after activities, difficult to recover
GradeIII: be fatigue during rest
GradeIV : Lying in bed

Fatigue comparison of two groups before treatment, there is notable significance in the difference, fatigue level of treat group is severer than that of control goup.

Table 13 Dry mouth, thirst comparison of two groups before treatment*

| group | cases | grade 0 | grade I | grade II | grade III | grade IV |
|---|---|---|---|---|---|---|
| treat group | 124 | 98 | 20 | 6 | 0 | 0 |
| control | 59 | 41 | 15 | 2 | 1 | 0 |
| | | | | rank sum test | u=1.531 | P=0.177 |

*Grade 0: none
Grade I : dry mouth
Grade II : dry mouth and throat, no desire for drink.
GradeIII: dry mouth and throat, desire for drink.
GradeIV: dry mouth, sore throat.

Dry mouth, thirst comparison of two groups before treatment, there is no significant difference.

Table 14 Bitter mouth comparison of two groups before treatment

| group | cases | grade 0 | grade I | grade II | grade III | grade IV |
|---|---|---|---|---|---|---|
| treat group | 124 | 86 | 28 | 4 | 6 | 0 |
| control | 59 | 50 | 7 | 2 | 0 | 0 |
| | | | rank sum test | u=2.253 | P=0.024 | |

Bitter taste of mouth comparison of two groups before treatment, there is notable significance in the difference, Bitter taste of mouth of treat group is severer than control group.

Table 15 Spontaneous perspiration comparison of two groups before treatment*

| group | cases | grade 0 | grade I | grade II | grade III | grade IV |
|---|---|---|---|---|---|---|
| treat group | 124 | 110 | 13 | 1 | 0 | 0 |
| control | 59 | 57 | 2 | 0 | 0 | 0 |
| | | | rank sum test | u=1.769 | P=0.077 | |

*Grade 0: none
Grade I : sweat occasionally after activities.
Grade II : sweat immediately after activities.
GradeIII: sweat during rest
GradeIV: sweat vehemently.

Spontaneous perspiration comparison of two groups before treatment, there is no notable significance in the difference.

Table 16 Night sweat comparison of two groups before treatment*

| group | cases | grade 0 | grade I | grade II | grade III | grade IV |
|---|---|---|---|---|---|---|
| treat group | 124 | 111 | 10 | 3 | 0 | 0 |
| control | 59 | 55 | 3 | 1 | 0 | 0 |
| | | | rank sum test | u=0.801 | P=0.423 | |

*Grade 0: none
Grade I : occasionally
Grade II : often
GradeIII: every night
GradeIV: wet cloth.

Night sweat comparison of two groups before treatment, there is no notable significance in the difference.

Table 17 Upset and tantrum comparison of two groups before treatment*

| group | cases | grade 0 | grade I | grade II | grade III | grade IV |
|---|---|---|---|---|---|---|
| treat group | 124 | 106 | 9 | 9 | 0 | 0 |
| control | 59 | 52 | 7 | 0 | 0 | 0 |
| | | | rank sum test | u=0.643 | P=0.520 | |

*Grade 0: none
Grade I : upset, self-controllable
Grade II : upset, self-uncontrollable
GradeIII: burning sensation of five centres, irritable
GradeIV: manic

Upset and tantrum comparison of two groups before treatment, there is no notable significance in the difference.

Table 18 Dizziness comparison of two groups before treatment*

| group | cases | grade 0 | grade I | grade II | grade III | grade IV |
|---|---|---|---|---|---|---|
| treat group | 124 | 112 | 7 | 5 | 0 | 0 |
| control | 59 | 51 | 6 | 2 | 0 | 0 |
| | | | rank sum test | u=0.739 | P=0.460 | |

*Grade 0: none
Grade I : dizzy occasionally
Grade II : dizzy regularly
GradeIII: dizzy persistently
GradeIV : need lie in bed

Dizziness comparison of two groups before treatment, there is no notable significance in the difference.

Table 19 Jaundice comparison of two groups before treatment*

| group | cases | grade 0 | grade I | grade II | grade III | grade IV |
|---|---|---|---|---|---|---|
| treat group | 124 | 113 | 7 | 4 | 0 | 0 |
| Control | 59 | 54 | 3 | 2 | 0 | 0 |
| | | | rank sum test | u=0.082 | P=0.934 | |

*Grade 0: none
Grade I : albuginea oculi mildly stained yellow
Grade II : albuginea oculi and skin mildly stained yellow
GradeIII: albuginea oculi and skin stained saffron yellow
GradeIV: albuginea oculi and skin stained deep yellow

Jaundice comparison of two groups before treatment, there is no notable significance in the difference.

Table 20 Cancer pain comparison of two groups before treatment*

| group | cases | grade 0 | grade I | grade II | grade III | grade IV |
|---|---|---|---|---|---|---|
| treat group | 124 | 46 | 48 | 29 | 1 | 0 |
| control | 58 | 23 | 31 | 4 | 0 | 0 |

(continued)

| group | cases | grade 0 | grade I | grade II | grade III | grade IV |
|---|---|---|---|---|---|---|
| | | | rank sum test | u=1.509 | P=0.131 | |

*Grade 0: none
Grade I : mild (discomfort, no upset)
Grade II : moderate (bearable, no upset)
GradeIII: sereve (positive position, demand painkiller)
GradeIV: uncontrollable (upset, passive position, need painkiller to relieve)

Cancer pain comparison of two groups before treatment, there is no notable significance in the difference

Table 21 Abdominal distension comparison of two groups before treatment*

| group | cases | grade 0 | grade I | grade II | grade III | grade IV |
|---|---|---|---|---|---|---|
| treat group | 124 | 47 | 57 | 12 | 8 | 0 |
| control | 59 | 32 | 21 | 3 | 3 | 0 |
| | | | rank sum test | u=2.052 | P=0.040 | |

*Grade 0: none
Grade I : abdominal distension, relieved after passage of gas by anus, no ascites.
Grade II : abdominal distension ,unrelieved after passage of gas by anus, no ascites.
GradeIII: obvious abdominal distension with small or moderate volume of ascites.
GradeIV: obvious abdominal distension with large volume of ascites

Abdominal distension comparison of two groups before treatment, there is notable significance in the difference, abdominal distension of treat group is severer than control group.
10. Ascites and abdomen circumference size comparison of two groups before treatment.

Table 22 Ascites comparison of two groups before treatment.

| group | cases | without | with |
|---|---|---|---|
| treat group | 124 | 113 | 11 |
| control | 59 | 51 | 8 |
| | | $X^2$=0.944 | P=0.331 |

Ascites comparison of two groups before treatment, there is no notable significance in the difference.

Table 23 Abdomen circumference size comparison of two groups before treatment

| group | cases | $\overline{x}\pm s$ |
|---|---|---|
| treat group | 94 | 82.89±8.22 |
| control | 36 | 80.86±9.59 |
| | rank sum test u=1.272 | P=0.203 |

*Data of 30 cases in treat group and 23 cases in control group are lost.

Abdomen circumference size comparison of two groups before treatment, there is no notable significance in the difference.
11. Tongue Picture and pulse tracings comparison of two groups before treatment

Table 24 Tongue Picture comparison of two groups before treatment

| group | cases | thin and white | thin and yellow | yellow and greasy | yellow and thick |
|---|---|---|---|---|---|
| treat group | 123 | 29 | 47 | 41 | 6 |
| control | 59 | 17 | 25 | 13 | 4 |
| | | Fisher's exact test | | P=0.426 | |

Tongue Picture comparison of two groups before treatment, there is no notable significance in the difference.

Table 25 Tongue texture comparison of two groups before treatment

| group | cases | carmoisine | red | dark red/ purple dark/ cyanochroia | petechia/ ecchymosis |
|---|---|---|---|---|---|
| treat group | 123 | 2 | 23 86 | | 12 |
| control | 59 | 0 | 2 | 55 | 2 |
| | | | Fisher's exact test | P=0.0024 | |

Tongue texture comparison of two groups before treatment, there is notable significance in the difference.

Table 26 Pulse tracings comparison of two groups before treatment

| group | cases | even | astringent | string | smooth | week |
|---|---|---|---|---|---|---|
| treat group | 123 | 4 | 35 | 69 | 12 | 3 |
| control | 59 | 0 | 15 | 28 | 11 | 5 |
| | | | | Fisher's exact test | P=0.089 | |

Pulse tracings comparison of two groups before treatment, there is no notable significance in the difference.
12..AFP, γ-GT, and LDH level comparison of two groups before treatment

Table 27 AFP (ug/ml) comparison of two groups before treatment

| group | cases | <30 | 30-399 | ≥400 | $\overline{x}\pm s$ |
|---|---|---|---|---|---|
| treat group | 123 | 15 | 35 | 73 | 380.5±367.0 |
| control | 58 | 2 | 20 | 36 | 408.8±332.7 |
| | $X^2$=3.73 | P=0.155 | | | |

AFP comparison of two groups before treatment, there is no notable significance in the difference.

Table 28 γ-GT comparison of two groups before treatment

| group | cases | $\overline{x}\pm s$ |
|---|---|---|
| treat group | 96 | 179.75±133.86 |
| control | 43 | 170.79±171.69 |
| | t=0.333 | P=0.739 |

γ-GT comparison of two groups before treatment, there is no notable significance in the difference.

Table 29 LDH comparison of two groups before treatment

| group | cases | $\overline{x}\pm s$ |
|---|---|---|
| treat group | 59 | 220.31±126.70 |
| control | 24 | 210.00±135.57 |
| | t=0.334 | P=0.739 |

LDH comparison of two groups before treatment, there is no notable significance in the difference.
Above results of comparability check show that sex, age, disease courses ,past treatment methods, tumor type, clinical stage, coated tongue and pulse tracings of two groups have no significant differences, except Karnofsky scores, body weights, fatigue, bitter taste of mouth, abdominal distension, tongue textures. Though comparison of Karnofsky scores, body weights, fatigue, bitter taste of mouth, abdominal distension, treat group are worse than those of control groups, considering these factors may influence therapeutic effects conparison, so Logistic regression analysis is necessary when comparing therapeutic effects.

III.Therapeutic effects comparison

1.Total therapeutic effects comparison

Table 30 Total therapeutic effects comparison

| group | cases | CR(%) | PR(%) | SD(%) | PD(%) |
|---|---|---|---|---|---|
| treat group | 124 | 0 | 19 | 97 | 8 |
| | | 0% | 15.3% | 78.2% | 6.5% |
| control | 59 | 0 | 4 | 44 | 11 |
| | | 0% | 6.8% | 74.6% | 18.6% |
| | | rank sum test | u=2.723 | P=0.006 | |

Table 31 Logistic regression analysis of total therapeutic effects*

| factor | coefficient | Wald | D/F | P value | RR | 95% confidence interval of RR |
|---|---|---|---|---|---|---|
| group | 1.540 | 8.533 | 1 | 0.003 | 4.66 | 1.66~13.11 |
| Karnofsky scores | 0.102 | 7.705 | 1 | 0.006 | 1.11 | 1.03~1.19 |
| constant | -6.303 | 5.228 | 1 | 0.022 | | |

* Dividing clinical therapeutic effects into two categories, that is, more than stability and progession disease.variable including group, Karnofsky scores, body weight, appetite, bitter taste of mouth, abdominal distension, tongue texture,using backward method to select variables, select results show that
only group and Karnofsky scores have notable significance. Hosmer & Lemeshow test of model, $X^2=0.199,P=0.995$,indicating model fitting is good.

CR, PR, SD and PD rates of treat group are 0%,15.3%,78.2%,6.5% respectively, CR, PR, SD and PD rates of control group are 0%,6.8%,74.6% and 18.6% respectively. There is notable signicance in the difference between two groups. Considering that factors including Karnofsky scores, body weight, fatigue, bitter mouth, abdominal distension may influence therapeutic effects, so Logistic regression analysis is necessary when comparing therapeutic effects. Results indicate that therapeutic effects between two groups have notable significance in their differences, relative risk (RR) of control group to treat group is 4.66, suggesting that the risk of disease progression of control group is 4.66 times of the treat group.
2. Life spans and survival rates comparison of two groups after treatment.

Table 32 Death comparasion of two groups 8 weeks after treatment

| group | cases | survival | death (cause) | | | | |
|---|---|---|---|---|---|---|---|
| | | | hepatic coma | Hepatorrhexis | upper gastrointestinal bleeding | failure | others |
| treat group | 124 | 102 | 3 | 6 | 4 | 7 | 2 |
| control | 59 | 48 | 2 | 1 | 5 | 2 | 1 |

combined death cases $X^2=0.022$ P=0.882
Death comparasion of two groups 8 weeks after treatment, there is no notable significance in the difference.

Talbe33 Death comparasion of two groups 1.5 years after treatment

| group | cases | survival | death (cause) | | | | | |
| --- | --- | --- | --- | --- | --- | --- | --- | --- |
| | | | hepatic coma | Hepatorrhexis | upper gastrointestinal bleeding | failure | hepatorenal syndrome | others |
| Treat group | 124 | 40 | 8 | 5 | 18 | 38 | 3 | 12 |
| Control | 59 | 21 | 3 | 4 | 10 | 11 | 3 | 7 |

combined death cases $X^2$=0.20 P=0.655

Death comparasion of two groups 1.5 years after treatment, there is no notable significance in the difference.

Table 34 Life span and survival rate comparison of two groups 1.5 year after treatment (I)*

| group | cases | complete data | censored | % censored |
| --- | --- | --- | --- | --- |
| Treat group | 122 | 83 | 39 | 32.0 |
| control | 58 | 38 | 20 | 34.5 |

*Data of 2 cases in treat group and 1 case of control group are lost.

Table 35 Life span and survival rate comparison of two groups 1.5 year after treatment (II) *

| group | cases | Average survival time (month) | Median survival time (month) | 1 year | survival rate |
| --- | --- | --- | --- | --- | --- |
| | | $\overline{x}\pm se$ | $\overline{x}\pm se$ | % | se |
| treat group | 122 | 11±1 | 11±2 | 40.25 | 4.48 |
| control | 58 | 11±1 | 10±4 | 45.75 | 6.63 |

short term effect Breslow test, statistic=0.04. P=0.851 Long term effect Log-rank test, statistic =0.00, P=0.983 survival rate Comparison of two groups ,Gehan test, statistic =0.035, P=0.852

Life span and survival rate comparison of two groups 1.5 year after treatment: 1year survival rate of treat group and control group are 40.25% ,45.75% respectively, there is no notable significance in the difference. between two groups.

3. Tumor foci size comparison of two groups after treatment

Table 36 Tumor foci size comparison of two groups after treatment *

| group | cases | before treatment | after treatment | difference (after-before) |
| --- | --- | --- | --- | --- |
| | | $\overline{x}\pm s$ | $\overline{x}\pm s$ | $\overline{x}\pm s$ |
| treat group | 112 | 71.5±63.2 | 53.8±48.4 | -17.6±29.9 |
| control | 55 | 78.0±57.0 | 71.8±56.8 | -6.9±23.7 |

* Tumor foci size is measured by product of two perpendicular maximum diameters of tumor or sum of products of multiple foci(cm×cm). 10 cases in treat group and 4 cases in control groups are diffuse type, Data of 2 cases in treat group are lost.

Comparison of treat group before and after treatment, t=6.24, P=0.000;

Comparison of control group before and after treatment, t=2.14, P=0.037;

Difference (after-before) comparison of two groups, t=2.31, P=0.022.

Tumor foci size comparison of treat group before and after treatment, there is notable significance in the difference.

Tumor foci size comparison of control group before and after treatment, there is notable significance in the difference..

Tumor foci size difference (after-before) comparison of two groups,there is notable significance in the difference.

4. Karnofsky scores comparison of two groups after treatment

Table 37 Karnofsky scores comparison of two groups after treatment

| group | before treatment | after treatment | difference (after-before) |
|---|---|---|---|
| | $\overline{x}\pm s(n)$ | $\overline{x}\pm s(n)$ | $\overline{x}\pm s(n)$ |
| Treat group | 74.7±7.0(124) | 85.7±9.1(108) | 11.8±7.2(108) |
| control | 78.4±8.4(59) | 77.5±11.5(55) | -0.5±8.5(55) |

Comparison of treat group before and after treatment, t=16.87, P=0.000;
Comparison of control group before and after treatment, t=0.396, P=0.694;
Difference (after-before) comparison of two groups, t=9.584, P=0.000;

Karnofsky scores comparison of treat group before and after treatment, there is notable significance in the difference..
Karnofsky scores comparison of control group before and after treatment, there is no notable significance in the difference..
Karnofsky scores difference (after-before) comparison of two groups, there is notable significance in the difference.
5. Body weight comparison of two groups after treatment

Table 38 Body weight (Kg) t comparison of two groups after treatment

| group | before treatment | after treatment | difference (after-before) |
|---|---|---|---|
| | $\overline{x}\pm s(n)$ | $\overline{x}\pm s(n)$ | $\overline{x}\pm s(n)$ |
| Treat group | 66.0±9.1(123) | 66.5±8.5(108) | 1.3±2.8(107) |
| Control | 62.6±6.8(59) | 62.2±7.2(54) | -0.5±2.4(54) |

Comparison of treat group before and after treatment, t=0.04, P=0.965;
Comparison of control group before and after treatment, t=1.73, P=0.108;
Difference (after-before) comparison of two groups, t=9.584, P=0.000;

Body weight comparison of treat group before and after treatment, there is no notable significance in the difference..
Body weight comparison of control group before and after treatment, there is no notable significance in the difference..
Body weight difference (after-before) comparison of two groups,there is notable significance in the difference..
6. Appetite comparison of two groups after treatment

Table 39 Appetite (taels/day) comparison of two groups after treatment

| group | before treatment $\overline{x}\pm s(n)$ | after treatment $\overline{x}\pm s(n)$ | difference (after-before) $\overline{x}\pm s(n)$ |
|---|---|---|---|
| Treat group | 6.0±1.6(124) | 6.9±1.6(112) | 1.1±1.7(112) |
| Control | 6.1±1.6(59) | 6.4±1.6(55) | -0.3±1.6(55) |

Comparison of treat group before and after treatment, t=4.98, P=0.000;
Comparison of control group before and after treatment, t=5.92, P=0.000;
Difference (after-before) comparison of two groups, t=2.61, P=0.010.

Appetite comparison of treat group before and after treatment, there is notable significance in the difference.
Appetite comparison of control group before and after treatment, there is notable significance in the difference.
Appetite difference (after-before) comparison of two groups, there is notable significance in the difference.
7. Main clinical symptoms and signs improvement comparison of two groups after treatment

Table 40 Fatigue comparison of two groups after treatment*

| group | cases | aggravation | no change | Improved 1 grade | Improved 2 grades | Improved 3 grades |
|---|---|---|---|---|---|---|
| Treat group | 113 | 4 | 32 | 42 | 21 | 14 |
| Control | 55 | 3 | 30 | 18 | 4 | 0 |

(continued)

| group | cases | aggravation | no change | Improved 1 grade | Improved 2 grades | Improved 3 grades |
|---|---|---|---|---|---|---|
| rank sum test | | | | u=3.98 | P=0.000 | |

\* Data of 11 cases in treat group and 4 cases of control group are lost.

Meaning of Improved grade is the same as application example 1.
Fatigue comparison of two groups after treatment, there is notable significance in the difference.

Table 42 Dry mouth and thirst improvement comparison of two groups after treatment\*

| group | cases | aggravation | no change | Improved 1 grade | Improved 2 grades | Improved 3 grades |
|---|---|---|---|---|---|---|
| Treat group | 113 | 2 | 88 | 17 | 6 | 0 |
| control | 55 | 0 | 43 | 12 | 0 | 0 |
| | | | rank sum test | u=0.25 | P=0.806 | |

\* Data of 11 cases in treat group and 4 cases in control group are lost.

Meaning of Improved grade is the same as application example 1.
Mouth and thirst improvement comparison of two groups after treatment, There is no notable significance in the difference.

Table 43 Bitter mouth improvement comparison of two groups after treatment

| group | cases | aggravation | no change | Improved 1 grade | Improved 2 grades | Improved 3 grades |
|---|---|---|---|---|---|---|
| treatgroup | 113 | 0 | 77 | 26 | 4 | 6 |
| control | 55 | 1 | 49 | 5 | 0 | 0 |
| rank sum test | | | | u=3.45 | P=0.001 | |

\* Data of 11 cases in treat group and 4 cases in control group are lost.

Bitter mouth improvement comparison of two groups after treatment, there is notable significance in the difference.

Table 44 Spontaneous perspiration improvement comparison of two groups after treatment\*

| group | cases | aggravation | no change | Improved 1 grade | Improved 2 grades | Improved 3 grades |
|---|---|---|---|---|---|---|
| treatgroup | 113 | 1 | 101 | 11 | 0 | 0 |
| control | 55 | 1 | 52 | 2 | 0 | 0 |
| rank sum test | | | | u=1.46 | P=0.143 | |

\* Data of 11 cases in treat group and 4 cases in control group are lost.

Meaning of Improved grade is the same as application example 1.
Spontaneous perspiration improvement of comparison of two groups after treatment, there is no notable significance in the difference.

Table 45 Night sweat improvement comparison of two groups after treatment*

| group | cases | aggravation | no change | Improved 1 grade | Improved 2 grades | Improved 3 grades |
|---|---|---|---|---|---|---|
| treat group | 113 | 1 | 103 | 9 | 0 | 0 |
| control | 55 | 1 | 50 | 4 | 0 | 0 |
| | | | rank sum test | u=0.33 | P=0.743 | |

* Data of 11 cases in treat group and 4 cases in control group are lost.

Meaning of Improved grade is the same as application example 1.
Night sweat improvement comparison of two groups after treatment, there is no notable significance in the difference.

Table 46 Upset and tantrum improvement comparison of two groups after treatment

| group | cases | aggravation | no change | Improved 1 grade | Improved 2 grades | Improved 3 grades |
|---|---|---|---|---|---|---|
| treat group | 114 | 0 | 96 | 9 | 9 | 0 |
| control | 54 | 0 | 48 | 6 | 0 | 0 |
| | | | rank sum test | u=0.96 | P=0.339 | |

* Data of 10 cases in treat group and 5 cases in control group are lost.

Improved 1 grade: Upset and tantrum lowered 1 grade after treatment, for example, upset and self-uncontrollable, improved to be upset and self-controllable.
Improved 2 grades: upset and tantrum lowered 2 grades after treatment, for example, burning sensation of five centres and tantrum, improved to be upset and self-controllable.
Improved 3 grades: upset and tantrum lowered 3 grades after treatment, for example, vesania, improved to be upset and self-controllable.
Upset and tantrum improvement comparison of two groups after treatment, there is no notable significance in the difference.

Table 47 Dizziness improvement comparison of two groups after treatment

| group | cases | aggravation | no change | Improved 1 grade | Improved 2 grades | Improved 3 grades |
|---|---|---|---|---|---|---|
| treat group | 114 | 1 | 101 | 8 | 4 | 0 |
| control | 54 | 3 | 48 | 3 | 0 | 0 |
| | | | rank sum test | u=1.77 | P=0.077 | |

* Data of 10 cases in treat group and 5 cases in control group are lost.

Meaning of Improved 1 grade and 2 grades are the same as application example 1.
Improved 3 grades means dizziness lowered 3 grades compared with before treatment, for example, need lie in bed, or improve to be dizzy occasionally.
Dizziness improvement of comparison of two groups after treatment, there is no notable significance in the difference.

Table 48 Jaundice improvement comparison of two groups after treatment

| group | cases | aggravation | no change | Improved 1 grade | Improved 2 grades | Improved 3 grades |
|---|---|---|---|---|---|---|
| treat group | 114 | 4 | 103 | 5 | 2 | 0 |
| control | 54 | 5 | 47 | 1 | 1 | 0 |

(continued)

| group | cases | aggravation | no change | Improved 1 grade | Improved 2 grades | Improved 3 grades |
|---|---|---|---|---|---|---|
| | | | rank sum test | u=1.52 | P=0.128 | |

\* Data of 10 cases in treat group and 5 cases in control group are lost.

Improved 1 grade: Jaundice lowered 1 grade after treatment, for example, albuginea oculi and skin slightly stained yellow, improved to only albuginea oculi slightly stained yellow.

Improved 2 grades: Jaundice lowered 2 grades after treatment, for example, albuginea oculi and skin stained saffron yellow, improved to only albuginea oculi slightly stained yellow.

Improved 3 grades: Jaundice lowered 3 grades after treatment, for example, albuginea oculi and skin stained deep yellow or dark yellow, improved to only albuginea oculi slightly stained yellow.

Jaundice improvement comparison of two groups after treatment, there is no notable significance in the difference .

Table 49 Cancer pain improvement comparison of two groups after treatment

| group | cases | aggravation | no change | Improved 1 grade | Improved 2 grades | Improved 3 grades |
|---|---|---|---|---|---|---|
| treat group | 114 | 0 | 44 | 47 | 22 | 1 |
| control | 53 | 1 | 26 | 23 | 3 | 0 |
| | | | rank sum test | u=2.20 | P=0.028 | |

\*Data of 10 cases in treat group and 6 cases in control group are lost.

Meaning of Improved grade is the same as application example 1.

Cancer pain improvement comparison of two groups after treatment, there is notable significance in the difference.

Table 50 Abdominal distension improvement comparison of two groups after treatment

| group | cases | aggavation | no change | Improved 1 grade | Improved 2 grades | Improved 3 grades |
|---|---|---|---|---|---|---|
| treat group | 114 | 3 | 43 | 56 | 9 | 3 |
| control | 53 | 5 | 33 | 12 | 1 | 2 |
| | | | rank sum test | u=3.70 | P=0.000 | |

\* Data of 10 cases in treat group and 6 cases in control group are lost.

Meaning of Improved grade is the same as application example 1.

Abdominal distension improvement comparison of two groups after treatment, there is notable significance in the difference.

8. Main symptoms and signs disappearance rates comparison of two groups after treatment

Table 51 Main symptoms and signs disappearance rates comparison of two groups after treatment

| symptoms | group | total cases | original cases | disappearance cases | disappearance rate | |
|---|---|---|---|---|---|---|
| fatigue | treat group control | 113* 55 | 82 35 | 72 22 | 87.8 62.9 | $X^2$=6.35 P=0.009 |

(continued)

| symptoms | group | total cases | original cases | disappearance cases | disappearance rate | |
|---|---|---|---|---|---|---|
| dry mouth thirst | treat | 113* | 24 | 23 | 95.8 | Fisher's exact test P=0.040 |
| | group control | 55 | 15 | 11 | 73.3 | |
| bitter mouth | treat | 113* | 37 | 36 | 97.3 | Fisher's exact test P=0.103 |
| | group control | 55 | 7 | 4 | 57.1 | |
| spontaneous perspiration | treat | 113* | 11 | 11 | 100.0 | |
| | group control | 55 | 2 | 2 | 100.0 | |
| night sweat | treat | 113* | 10 | 9 | 90.0 | Fisher's exact test P=0.549 |
| | group control | 55 | 4 | 3 | 75.0 | |
| upset and tantrum | treat | 114# | 18 | 18 | 100.0 | |
| | group control | 54 | 6 | 6 | 100.0 | |
| dizziness | treat | 114# | 12 | 11 | 91.7 | Fisher's exact test P=0.037 |
| | group control | 54 | 7 | 3 | 42.9 | |
| jaundice | treat | 114# | 10 | 7 | 70.0 | Fisher's exact test P=0.657 |
| | group control | 54 | 3 | 1 | 33.3 | |
| cancer pain | treat | 114+ | 72 | 66 | 91.7 | Fisher's exact test P=0.199 |
| | group control | 53 | 32 | 26 | 81.3 | |
| abdominal distension | treat | 114+ | 70 | 66 | 94.3 | Fisher's exact test P=0.001 |
| | group control | 53 | 25 | 14 | 56.0 | |

\* Data of 11cases in treat group and 4 cases in control group are lost.
\# Data of 10 cases in treat group and 5 cases in control group are lost.
+ Data of 10cases in treat group and 6 cases in control group are lost.

Disappearance rates comparison of fatigue, dry mouth, thirst, dizziness, abdominal distension of two groups after treatment,there is notable significance in the difference. Disappearance rates comparison of bitter mouth spontaneous perspiration, night sweat, upset, tantrum, jaundice, cancer pain of two groups after treatment,there is no notable significance in the difference.

9. WBC counting comparison of two groups after treatment

Table 52 WBC counting comparison of two groups after treatment*

| group | cases | aggravation | no change | Improved 1 grade | Improved 2 grades | Improved 3 grades |
|---|---|---|---|---|---|---|
| treat group | 114 | 6 | 91 | 8 | 8 | 1 |
| control | 54 | 5 | 44 | 5 | 0 | 0 |
| | rank sum test | | u=1.45 | P=0.148 | | |

● Grade WBC accurately according to "WHOgrade criterions for acute and subacute toxicity reactions of the anti-cancer drugs", see details in application example 1.

WBC counting comparison of two groups after treatment, there is no notable significance in the difference.

10. Granulocytes counting comparison of two groups after treatment

**EP 1 707 209 A1**

Table 53 Granulocytes counting comparison of two groups after treatment *

| group | cases | aggravation | no change | Improved 1 grade | Improved 2 grades | Improved 3 grades |
|---|---|---|---|---|---|---|
| treat group | 117 | 2 | 103 | 6 | 6 | 0 |
| control | 59 | 5 | 51 | 1 | 1 | 1 |
| | | rank sum test | u=2.07 | P=0.039 | | |

* Grade granulocytes accurately according to "WHOgrade criterions for acute and subacute toxicity reactions of the anti-cancer drugs", see details in application example 1.

Granulocytes counting comparison of two groups after treatment, there is notable significance in the difference.

11. Hemoglobin comparison of two groups after treatment

Table 54 Hemaglobin comparison of two groups after treatment *

| group | cases | aggravation | no change | Improved 1 grade | Improved 2 grades | Improved 3 grades |
|---|---|---|---|---|---|---|
| treat group | 118 | 16 | 91 | 10 | 1 | 0 |
| control | 59 | 11 | 40 | 6 | 0 | 2 |
| | | rank sum test | u=0.16 | P=0.870 | | |

* Grade hemoglobin accurately according to WHO"grade criterions for acute and subacute toxicity reactions of the anti-cancer drugs", see details in application example 1.

Hemoglobin comparison of two groups after treatment, there is no notable significance in the difference.

12. Platelets counting comparison of two groups after treatment

Table 55 Platelets counting comparison of two groups after treatment*

| group | cases | aggravation | no change | Improved 1 grade | Improved 2 grades | Improved 3 grades |
|---|---|---|---|---|---|---|
| treat group | 117 | 12 | 83 | 12 | 8 | 2 |
| control | 59 | 7 | 47 | 4 | 1 | 0 |
| | | rank sum test | u=1.48 | P=0.139 | | |

● Grade platelets accurately according to "WHOgrade criterions for acute and subacute toxicity reactions of the anti-cancer drugs", see details in application example 1.
● Phatelets counting comparison of two groups after treatment, there is no notable significance in the difference .

13 .Immune function comparison of two groups after treatment

Table 56 CD3 comparison of two groups before and after treatment

| group | cases | Before treatment $\bar{x}\pm s$ | 8 weeks after treatment $\bar{x}\pm s$ | difference(after-before) $\bar{x}\pm s$ |
|---|---|---|---|---|
| treat group | 58 | 46.6±21.0 | 50.8±21.5 | 4.3±8.0 |

(continued)

| group | cases | Before treatment | 8 weeks after treatment | difference(after-before) |
|---|---|---|---|---|
| | | $\overline{x}\pm s$ | $\overline{x}\pm s$ | $\overline{x}\pm s$ |
| control | 22 | 58.9±7.0 | 55.5±7.6 | -3.4±8.2 |

Comparison of treat group before and after treatment, t=4.06, P=0.000:
Comparison of control group before and after treatment, t=1.95, P=0.065;
Difference (after-before) comparison of two groups, t=3.80, P=0.000.
CD3 comparison of treat group before and after treatment, there is notable significance in the difference
CD3 comparison of control group before and after treatment, there is no notable significance in the difference.
CD3 (after-before) comparison of two groups ,there is notable significance in the difference .

Table 57 CD4 comparison of two groups before and after treatment

| group | cases | Before treatment | 8 weeks after treatment | difference(after-before) |
|---|---|---|---|---|
| | | $\overline{x}\pm s$ | $\overline{x}\pm s$ | $\overline{x}\pm s$ |
| treat group | 58 | 31.4±14.2 | 36.0±15.4 | 4.6±5.1 |
| control | 22 | 39.4±7.4 | 40.8±7.6 | 1.4±7.8 |

Comparison of treat group before and after treatment, t=6.90, P=0.000;
Comparison of control group before and after treatment, t=0.83, P=0.416;
Difference (after-before) comparison of two groups, t'=1.81, P=0.080.

CD4 comparison of treat group before and after treatment,there is notable significance in the difference
CD4 comparison of control group before and after treatment,there is no notable significance in the difference.
CD4difference (after-before) comparison of two groups,there is no notable significance in the difference.

Table 58 CD8 comparison of two groups before and after treatment

| group | cases | Before treatment | 8 weeks after treatment | difference(after-before) |
|---|---|---|---|---|
| | | $\overline{x}\pm s$ | $\overline{x}\pm s$ | $\overline{x}\pm s$ |
| treat group | 58 | 28.0±12.7 | 27.7±12.2 | -0.4±3.6 |
| control | 22 | 36.3±7.8 | 35.2±9.1 | -1.0±6.0 |

Comparison of treat group before and after treatment, t=0.83, P=0.412;
Comparison of control group before and after treatment, t=0.82, P=0.422;
Difference (after-before) comparison of two groups, t'=0.49, P=0.632.

CD8 comparison of treat group before and after treatment, there is no notable significance in the difference.
CD8 comparison of control group before and after treatment, there is no notable significance in the difference. CD8
(after-before) comparison of two groups, there is no notable significance in the difference.

Table 59 CD4/CD8 comparison of two groups before and after treatment

| group | cases | Before treatment | 8 weeks after treatment | difference(after-before) |
|---|---|---|---|---|
| | | $\overline{x}\pm s$ | $\overline{x}\pm s$ | $\overline{x}\pm s$ |
| treat group | 56 | 1.12±0.28 | 1.32±0.22 | 0.19±0.18 |
| control | 21 | 1.11±0.23 | 1.23±0.19 | 0.11±0.13 |

Comparison of treat group before and after treatment, t=7.96, P=0.000;
Comparison of control group before and after treatment, t=3.89, P=0.022;
Difference (after-before) comparison of two groups, t=1.77. P=0.080.

CD4/CD8 comparison of treat group before and after treatment, there is notable significance in the difference.
CD4/CD8 comparison of control group before and after treatment, there is notable significance in the difference.
CD4/CD8 difference (after-before) comparison of two groups ,there is no notable significance in the difference.

Table 60 NK cell comparison of two groups before and after treatment

| goup | cases | Before treatment | 8 weeks after treatment | difference(after-before) |
|---|---|---|---|---|
| | | $\overline{x}\pm s$ | $\overline{x}\pm s$ | $\overline{x}\pm s$ |
| treat group | 30 | 32.6±15.1 | 39.7±14.8 | 7.1±10.2 |
| control | 9 | 49.4±13.1 | 39.3±9.5 | -10.1±10.7 |

Comparison of treat group before and after treatment, t=3.83, P=0.001;
Comparison of control group before and after treatment, t=2.84, P=0.022;
Difference (after-before) comparison of two groups, t=4.40, P=0.000.

NK cell comparison of treat group before and after treatment, there is notable significance in the difference.
NK cell comparison of control group before and after treatment, there is notable significance in the difference.
NK cell difference (after-before) comparison of two groups ,there is notable significance in the difference.
14. γ-GT comparison of two groups after treatment

Table 61 γ-GT comparison of two groups after treatment

| group | Before treatment | 8 weeks after treatment | difference (after-before) |
|---|---|---|---|
| | $\overline{x}\pm s$ (n) | $\overline{x}\pm s$ (n) | $\overline{x}\pm s$ (n) |
| treat group | 179.75±133.86(96) | 148.75±111.50(96) | -31.00±112.02(96) |
| control | 170.79±171.69(43) | 164.37±138.24(41) | -8.58±170.05(41) |

Comparison of treat group before and after treatment, t=2.711, P=0.008;
Comparison of control group before and after treatment, t=0.323, P=0.748;
Difference (after-before) comparison of two groups, t=0.911, P=0.364.

γ-GT comparison of treat group before and after treatment ,there is notable significance in the difference.
γ-GT comparison of control group before and after treatment, there is no notable significance in the difference.
γ-GT difference (after-before) comparison of two groups , there is no notable significance in the difference.
15. LDH comparison of two groups after treatment

Table 62 LDH comparison of two groups after treatment

| group | Before treatment | 8 weeks after treatment | difference (after-before) |
|---|---|---|---|
| | $\overline{x}\pm s$ (n) | $\overline{x}\pm s$ (n) | $\overline{x}\pm s$ (n) |
| treat group | 220.31±126.70(59) | 190.05±128.29(58) | -45.54±98.30(57) |
| control | 210.00±135.57(24) | 194.09±130.45(23) | -19.39±84.72(23) |

Comparison of treat group before and after treatment, t=3.498, P=0.001;
Comparison of control group before and after treatment, t=1.098, P=0.284;
Difference (after-before) comparison of two groups, t=1.118, P=0.267.

LDH comparison of treat group before and after treatment, there is notable significance in the difference.
LDH comparison of control group before and after treatment, there is no notable significance in the difference
LDH difference (after-before) comparison of two groups, there is no notable significance in the differencee

IV.Results of imageology examinations after treatment

1.B type ultrasonic examination:

116 cases in treat group and 55 cases in control group are rechecked by B type ultrasonic examination after treatment.

2. CT and MRI examination:

83 cases in treat group and 33 cases in control group are rechecked by CT.

18 patients in treat group and 7 patients in control group are rechecked by MRI.

V. Safety assessment

All safety indexes are normal before treatment, the abnormal cases after treatment are recorded in following table.

| Doubtful adverse reaction | treat group | | control group | | RR | P value |
|---|---|---|---|---|---|---|
| | positive cases/total cases | incidence rate | positive cases/total cases | incidence rate | | |
| Hemoglobin reduction | 16/124 | 12.9% | 8/59 | 13.6% | 0.95 | 0.902 |
| RBC reduction | 0/121 | 0.0% | 0/59 | 0.0% | . | |
| WBC reduction | 6/120 | 5.0% | 5/55 | 9.1% | 0.55 | 0.325 |
| Granulocyte reduction | 1/119 | 0.8% | 4/59 | 6.8% | 0.12 | 0.042 |
| Platelet reduction | 12/124 | 9.7% | 5/59 | 8.5% | 1.14 | 0.793 |
| Bilirubin increase | 4/112 | 3.6% | 7/53 | 13.2% | 0.27 | 0.039 |
| AKP rising | 0/123 | 0.0% | 2/58 | 3.4% | . | |
| GPT rising | 7/116 | 6.0% | 2/58 | 3.4% | 1.75 | 0.720 |
| BUN rising | 0/119 | 0.0% | 0/54 | 0.0% | . | |
| serum creatinine rising | 2/123 | 1.6% | 0/59 | 0.0% | . | |
| Abnormal urine protein | 0/123 | 0.0% | 0/59 | 0.0% | . | |
| Abnornal urine | 0/120 | 0.0% | 0/59 | 0.0% | . | |
| RBC fecal occult blood | 1/120 | 0.8% | 0/59 | 0.0% | . | |

Safety study results of two groups after treatment demonstrate that incidence rates of granulocyte reduction, BUN rising of treat group is lower than those of control group (P<0.05). Iincidence rates, RR of other items of two groups and statistical analysis results , there is no notable significance in the difference. See details in above table.

VI. Adverse event observations

Results of adverse event observations of two groups after treatment are shown in followint table:

| doubtful adverse events | treat group (n=124) | | control group (n=59) | | RR | P value |
|---|---|---|---|---|---|---|
| | positive cases* | incidence rate | positive cases** | incidence rate | | |
| fever | 57 | 45.9% | 26 | 44.1% | 1.04 | 0.810 |
| alopecie | 21 | 16.9% | 14 | 23.7% | 0.71 | 0.275 |
| oral cavity ulcer | 0 | 0.0% | 1 | 1.7% | . | |
| cutaneous reaction | 0 | 0.0% | 1 | 1.7% | . | |
| choking and short of breath | 1 | 0.8% | 8 | 13.6% | 0.06 | 0.000 |

(continued)

| doubtful adverse events | treat group (n=124) | | control group (n=59) | | RR | P value |
|---|---|---|---|---|---|---|
| | positive cases* | incidence rate | positive cases** | incidence rate | | |
| non cancer pain | 31 | 25.0% | 16 | 27.1% | 0.92 | 0.759 |
| hypersensitiveness | 1 | 0.8% | 0 | 0.0% | . | |
| nausea and vomit | 49 | 39.5% | 34 | 57.6% | 0.67 | 0.021 |
| constipation | 2 | 1.6% | 8 | 13.6% | 0.12 | 0.002 |
| bleeding | 0 | 0.0% | 1 | 1.7% | . | |

*positive cases: if one patient had one symptom, recorded as one case, if one patient had two symptoms, recorded as two cases.

During the courses of treatment, some patients in both groups had adverse effects including alopecie, oral cavity ulcer etc, incidence rate and RR of each symptom, statistical analysis results are shown in above table.

During the courses of treatment, incidence rates comparison of choking, short of breath, cancer pain, nausea and vomit, constipation of two groups, there is notable significance in the difference. Judging from incidence rate and RR, the treat group is lower than those of control group. other symptoms comparison of two groups, there is no notable significance in the difference.

There are 90 cases in treat group who had at least one doubtful adverse reaction, total incidence of doubtful adverse reaction is 72.6% (90/124), there are 53 cases in control group who had at least one doubtful adverse reaction, total incidence of doubtful adverse reaction is 89.8% (53/59), the relative risk(RR) of doubtful adverse reaction of treat group is 0.808(95% confidence interval: 0.704~0.928), indicating the risk of doubtful adverse reaction of treat group is lower than that of control group.

Conclusions

183 eligible patients entered into the clinical trails, of which 124 cases for treat group, 59 cases for control group, all were diagnosed as primary hepatic carcinoma by westen medicine, and as symptom of stagnation of poison by Traditioanl Chinese Medicine.

Results of comparability test before treatment show that the comparisonof Karnofsky scores, body weights, fatigue, bitter mouth, abdominal distension, tongue textures of two groups before treatment, there is notable significance in the difference. But the comparison of sex, age, disease courses ,past treatment methods, tumor type, clinical stage, coated tongue and pulse tracings of two groups, there is no notable significance in the difference . Though comparison of Karnmofsky scores, body weights, fatigue, bitter mouth, abdominal distension, treat group are worse than those of control groups, considering that these factors may influence therapeutic effects, so we performed logistic regression analysis when comparing therapeutic effects in the research, to determine the influences of these factors on therapeutic effects comparison of two groups.

CR, PR, SD and PD rates of treat group are 0%,15.3%,78.2%,6.5% respectively, CR, PR, SD and PD rates of control group are 0%,6.8%,74.6% and 18.6% respectively. After comparsion of two groups,there is notable significance in the difference.

Considering that such factors as Karnofsky scores, body weight, fatigue, bitter mouth, abdominal distension the may influence therapeutic effects, so we performed logistic regression analysis when comparing therapeutic effects, introducing the above variables to the model and choosing them by retreating method . Selected Results indicate that there is notable significance in difference of group and Karnofsky scores. There is notable significance in the difference of therapeutic effects of groups.Relative risk scale (RR) of control group to treat group is 4.66, suggesting that the risk of disease progression of control group is 4.6 times of treat group.

Above results indicate that the invented Traditional Chinese Medicine composition, serving as adjunctive therapy on intervention chemotherapy and embolism treatment of primary hepatic carcinoma, has better clinical therapeutic effects , exerting certain synergistic functions.

After 1.5 years of follow-up, 1 year survival rate of treat group and control group are 40.3% ,45.8% respectively, results of survival analysis show that both short term effect(Breslow test) and long term effect(Log-rank test) of two groups, there is no notable significance in the difference of survial time.Indicating that the invented Traditional Chinese Medicine composition adjunctive therapy on primary hepatic carcinoma patients who received intervention and embolism treatments failed to elongate the survival time, compared with treatment of solely intervention and embolism.

Results of solid tomor foci size assessment show:

Tumor foci size difference (after-before) comparison of two groups shows that there is notable significance in the difference, minimized extent of the tumor foci size of treat group is greater than that of control group. Tumor foci size comparison of treat group before and after treatment shows that there is notable significance in the difference, tumor minimized obviously after treatment compared with before treatment; Tumor foci size comparison of control group before and after treatment aslo shows that there is notable significance in the difference, tumor minimized obviously after treatment compared with before treatment. Suggesting that the invented Traditional Chinese Medicine composition, serving as adjunctive therapy on intervention chemotherapy and embolism treatment of primary hepatic carcinoma patients, can minimize tumor foci obviously, which outweighs therapeutic effects of the group received sole intervention chemotherapy and embolism treatment.

Results of main symptoms improvement show:

Karnofsky score of treat group increased obviously after treatment compared with that of before treatment, and the Karnofsky score increase extent of treat group is greater than control group, Karnofsky score of control group has no obvious increase after treatment compared with that of before treatment; Fatigue of patients in both groups improved obviously after treatment, fatigue improvement extent and fatigue disappearance rate of treat group is greater than that of control group.

Appetite of patients in both groups increased obviously after treatment, symptoms including dry mouth, thirst, bitter taste of mouth, spontaneous perspiration, night sweat, upset,tantrum, dizziness, jaundice, cancer pain, abdominal distension are all improved or have high disappearance rates, for improvement extent of bitter mouth, cancer pain and dizziness, treat group is higher than that of control group; for disappearance rates of dry mouth, thirst, dizziness, abdominal distension, treat group is higher than those of control group, other symptoms comparison in improvement extent and disappearance rate of two groups shows that there is no notable significance in the differences.

Above results indicate that the invented Traditional Chinese Medicine composition, serving as adjunctive therapy on intervention chemotherapy and embolism treatment of primary hepatic carcinoma patients, can ameliorate patients survival qualities, improve clinical symptoms, has better adjunctive therapeutical effects.

Laboratory examination results demonstrate:

CD3, CD4 , CD4/CD8 and NK cells of treat group increased obviously after treatment compared with those of before treatment (P<0.05),furthermore, the CD3 and NK cell difference (after-before) of treat group are higher than those of control group (P<0.05), while changes of CD3, CD4, CD4/CD8 and NK cells of control group are not so obvious before treatment, NK cells of control group reduced obviously after treatment compared with that of before treatment.

Above results indicate that the invented Traditional Chinese Medicine composition, serving as adjunctive therapy on intervention chemotherapy and embolism treatment of primary hepatic carcinoma patients, has certain functions of stimulating immune responses, can assist intervention chemotherapy and embolism treatment to inhibit cancer cells.

Results of this randomized controlled trial demonstrate that the invented Traditional Chinese Medicine composition, serving as adjunctive therapy on intervention chemotherapy and embolism treatment of primary hepatic carcinoma patients, has better clinical therapeutic effects, can exert synergistic functions, can ameliorate patients survival qualities, improve clinical symptoms of patients and enhance cellular immune function of patients, can be used as adjunctive therapy on intervention chemotherapy and embolism treatment of patients primary hepatic carcinoma (considered as sympton of stagnation of poison by Traditinal Chinese Medicine), clinical application is quite safe.

**Application example 3 Clinical trail summary of adjunctive therapy using the invented Traditonal Chinese Medicine composition on gastric carcinoma**

Object and method

I Chosing of eligible tested object

1. Chinese medicine syndrome diagnosis criterion: the same as application example 1
2.Western medicine diagosis criterion: same as diagosis criterion of gastric carcinoma and stage criterion mentioned in application example 1.
3. Inclusion criterion

(1) Voluntary patients.

(2) Stage II , III, IV of gastric carcinoma patients unsuitable for surgical operation or reluctant to operation.

(3) Operations researcher of gastric carcinoma.

(4)Relapse patients of gastric carcinoma after operation unsuitable for surgical operation or reluctant to operation.

(5)Those patients who have received anti-cancer therapy, need stop therapy for more than 2 months.

(6)Age > 18years

(7)Predicted life span> 3months, survival quality Karnofsky score≥50

4. Exclusing criterion

(1) Patients with esophageal stenosis, cardia of stomach obstruction, pyloric obstruction, polypi, tumor, bowel obstruction; structural diseases of liver, cholecyst, pancreas, colon; gastric carcinoma like "linitis plastica " patients can not receive medication via po.

(2) Others are the same as(1), (2), (4)~(7) of application example 1.

II. Methods of clinical trails

1.Trail design: The same as application example 2.

2.Adiministration manner and dosage:

Treat group

Taking the invented Traditional Chinese Medicine composition orally at the right time of beginning chemotherapy, twice each day, 15ml each time (1 ml containing crude drugs 0.75g), taken in the early morning and evening, taken with warm water. The invented Traditional Chinese Medicine composition is taken for consecutive 2 months.

Control group: sole chemotherapy of gastric carcinoma

3. Chemotherapy regimen: MF regimen

5-Fu    500mg/M$^2$, V.D., d1-5

MMC    8mg/ M$^2$. i.v., d1

28 days $\times$2

4. Course of treatment: 2 months, observing for 1 month after treatment.

5. Observed items and methods:

(1)safety detection: The same as application example 1

(2)Therapeutic effects assessment:

① Tumor foci: Performing B type ultrasonic, X-Ray barium meal examination, air-barium double contrast examination, gastroscope, CT examination before and after treatment.

    a. Measurement of tumor size: multiply the two perpendicular maximum diameter.

    b. Multiple tumor foci are measured with sum of all products of multiplication (refer to products of two perpendicular maximum diameter).

    c. Diffused nodular tumor, explaining particularly.

② Clinical symptom observations:

    a.Main symptoms of gastric carcinoma

        Lump, abdominal pain, anorexia, hemafecia, nausea, vomit, fatigue, emaciation, ascites, swollen, jaundice etc.

    b. Main symptoms of stagnation of poison:

gastric discomfort, hard lump, pain ,fatigue, progressing emaciation,vomit, haematemesis , hemafecia , dark red, purple or cyanochroia texture of tongue, white or yellow coated tongue, weak ,astringent or deep pulse.

c. Karnofsky grade

③ Laboratory examinations:

a X-Ray barium meal examination, air-barium double contrast examination (obligatory)
b Gastroscope (obligatory)
c fecal occult blood (obligatory)
d CEA (part)
e B type ultrasonic (obligatory)
f CT(done when need)
g Immunologic test: CD3,CD4,CD8,NKcell etc.

Observing methods:

Observing and recording symptoms, picture of the tongue and pulse tracings once every 2 days, observing and recording Karnofsky score and body weight once every week, blood routine test is checked at the time of beginning visit, every week after treatment; urine routine test, faeces routine test, fecal occult blood test, liver function and kidney function are checked at the time of beginning visit, every two weeks after treatment; Immunology index, ECG, heart function are checked at the time of beginning visit, every four weeks after treatment; X-Ray barium meal examination, air-barium double contrast examination, gastroscope, B type ultrasonic, CEA, bleeding time and clotting time are checked at least once at the time of beginning visit and after treatment, performing examination at any time when needed Follow-up once every 1-2months after treatment, Follow-up lasts at least 1 year

III.Therapeutic efficacy assessment criterions

1.Therapeutic efficacy assessment criterions of the tumor foci:

(1) Complete Remission (CR): tumor disappeared.
(2) Partial Remission (PR): products of two maximum diameters minimized more than 50%.
(3) Stable disease (SD): products of two maximum diameters minimized less than 50%, increased no more than 25%.
(4) Progression disease (PD): products of two maximum diameters increased more than 25%. Total remission rate=CR+PR

2. Assessments of life spans and survival rates:

Life spans after treatment refer to the time from beginning of treatment to death or the last follow-up.
Follow-up at least 1 year after treatment.

3. the change of health condition : make a comparison before and after treatment, according to Karnofsky score criterions.As for Karnofsky score criterions ,see details in application example1.

IV.Handling and summarizing of clinical trail data

Collecting all data, inputting medical histroy into computers, constructing database using EPI INF06 software, making statistical treatment and analysis, writing summary of clinical trails, making objective assessment about clinical adjunctive therapeutic effects and safety of the invented Traditional Chinese Medicine composition on gastric carcinoma.
Statistical method: enumeration data are analysed by $X^2$ test, ranked data are analysed by Wilcoxon rank sum test, two sample means are checked by t-test. Life span and survival rate are analysed using life table method and Kaplan-Meier method.

Results

I .General data

129 suitable cases, 87 cases in treat group, 42 cases in control group; all are diagnosed as gastric carcinoma by westen medicine and as symptom of stagnation of poison by Traditonal Chinese medicine. 15 outpatient cases, 114 inpatient cases.

II .Comparability analysis of two groups

1. Sex comparison of two groups

Table 1 sex comparison of two groups

| group | cases | male | female |
|---|---|---|---|
| treat group | 87 | 61 | 26 |
| control | 42 | 26 | 16 |
| | | $X^2$=0.87 | P=0.35 |

Sex comparison of two groups, there is no notable significance in the difference.
2. Age (year) comparison of two groups

Table 2 Age (year) comparison of two groups

| group | cases | 18-29 | 30-49 | 50-65 | 66-70 | $\bar{x}\pm s$ |
|---|---|---|---|---|---|---|
| treat group | 87 | 0 | 14 | 65 | 8 | 56.2±8.0 |
| control | 42 | 1 | 8 | 29 | 4 | 54.6±10.8 |
| | Fisher's exact test | | | | | t=0.95 |
| | P=0.566 | | | | | P=0.35 |

Age (year) comparison of two groups, there is no notable significance in the difference.
3. Disease courses comparison of two groups

Table 3 Disease courses (month) comparison of two groups

| group | cases | ≤3 | 4-6 | 7-12 | 13-24 | >24 |
|---|---|---|---|---|---|---|
| treat group | 87 | 43 | 19 | 12 | 7 | 6 |
| control | 42 | 24 | 7 | 6 | 4 | 1 |
| | Fisher's exact test | | P=0.803 | | | |

Disease courses comparison of two groups, there is no notable significance in the difference.
4. Past treatment comparison of two groups

Table 4 Past treatment comparison of two groups *

| group | cases | untreated | operation | radiotherapy | chemoth erapy | TCM | others |
|---|---|---|---|---|---|---|---|
| treat group | 85 | 56 | 17 | 1 | 2 | 1 | 8 |
| control | 42 | 24 | 10 | 0 | 1 | 3 | 4 |
| | Fisher's exact test | | | P=0.517 | | | |

* 2 cases in treat group are not recorded; "others " refer to combined therapy (including 1 case received intervention chemotherapy)

Past treatment comparison of two groups, there is no notable significance in the difference.

5. Past treatment effects comparison of two groups

Table 5 Past treatment effects comparison of two groups*

| group | cases | effective | ineffective | relapse |
|---|---|---|---|---|
| treat group | 85 | 8 | 60 | 17 |
| control | 42 | 2 | 33 | 7 |
| | | $X^2=1.18$ | P=0.55 | |

* 2 cases in treat group are not recorded.

Past treatment effects comparison of two groups , there is no notable significance in the difference.

6. Gastric carcinoma pathologic diagnosis comparison of two groups

Table 6 Gastric carcinoma pathologic diagnosis comparison of two groups

| group | cases | adencarcinoma | undifferentiated carcinoma |
|---|---|---|---|
| treat group | 87 | 86 | 1 |
| control | 42 | 38 | 4 |
| | Fisher's exact test | P=0.038 | |

Gastric carcinoma pathologic diagnosis comparison of two groups ,there is notable significance in the difference.

7.Gastric carcinoma clinical stage comparison of two groups

Table7 Clinical stage comparison of two groups

| group | cases | stage II | stage III | stage IV |
|---|---|---|---|---|
| treat group | 87 | 2 | 16 | 69 |
| control | 42 | 1 | 13 | 28 |
| | | Fisher's exact test | P=0.278 | |

Clinical stage comparison of two groups, there is no notable significance in the difference.

8.Tumor foci size comparison of two groups before treatment

Table 8 Tumor foci size comparison of two groups before treatment*

| group | cases | $\overline{x}\pm s$ |
|---|---|---|
| treat group | 83** | 27.10±22.29 |
| control | 40 ** | 26.48±48.58 |

(continued)

| group | cases | $\overline{x}\pm s$ |
|---|---|---|
| | t=0.097 | P=0.92 |

*Tumor foci size is measured by product of two perpendicular maximum diameters of tumor or sum of products of multiple foci (cm×cm) .

**4 cases in treat group and 2 cases in control groups tumor foci size can not be measured:

Treat group: 1 gastric carcinomar case widely metastasized in abdominal cavity, 1 case is pathologically diagnosed as "cancer cells in ulcerative necrosis tissue", 1 case is diagnosed as" diffused, ulcerative, infiltrating carcinoma of gastric body and sinus ventriculi", 1 case is diagnosed as "poorly differentiated adenocarcinoma of cardia of stomach, with metastasis of middle and inferior esophagus ".

Control group: 1 case with wide bone metastasis, 1 case is diagnosed as" diffused, ulcerative, infiltrating carcinoma of gastric body and sinus ventriculi" Tumor foci size comparison of two groups before treatment, there is no notable significance in the difference.

Table 9 Tumor foci location comparison of two groups before treatment*

| group | cases | cardia of stomach | gastric body | sinus ventriculi | Gastrojejunal | others |
|---|---|---|---|---|---|---|
| treat group | 87 | 24 | 28 | 13 | 1 | 21 |
| control | 42 | 6 | 12 | 5 | 1 | 18 |
| | | Fisher's exact test | | P=0.197 | | |

*others: two or more than two tumor foci locations, metastasized to other locations after Gastric operation Tumor foci location comparison of two groups before treatment, there is no notable significance in the difference.

Table 10 Metastasis comparison of two groups before treatment

| group | cases | metastasis | no metastasis |
|---|---|---|---|
| treat group | 87 | 25 | 62 |
| control | 42 | 15 | 27 |
| | | $X^2$=0.64 | P=0.42 |

Metastasis comparison of two groups before treatment, there is no notable significance in the difference

Table 11 Numbers of tumor foci comparison of two groups before treatment

| group | cases | 1↑ | 2↑ | >2↑ |
|---|---|---|---|---|
| treat group | 87 | 74 | 4 | 9 |
| control | 41 | 34 | 6 | 1 |
| | Fisher's exact test | | P=0.061 | |

Numbers of tumor foci comparison of two groups before treatment,there is no notable significance in the difference.

9. Karnofsky score comparison of two groups before treatment

Table 12 Karnofsky score comparison of two groups before treatment

| group | cases | 50-59 | 60-69 | 70-79 | 80-89 | $\overline{x}\pm s$ |
|---|---|---|---|---|---|---|
| treat group | 87 | 6 | 34 | 29 | 18 | 66.78$\pm$8.83 |
| control | 42 | 1 | 15 | 15 | 11 | 69.04$\pm$9.32 |
| rank sum test | | u=1.045 P=0.296 | | | | t=1.34 P=0.18 |

Karnofsky score comparison of two groups before treatment, there is no notable significance in the difference.

10. Body weight comparison of two groups before treatment

Table 13 Body weight (Kg) comparison of two groups before treatment

| group | cases | 35-39 | 40-49 | 50-59 | 60-69 | >70 | $\overline{x}\pm s$ |
|---|---|---|---|---|---|---|---|
| treat group | 87 | 2 | 12 | 40 | 26 | 7 | 57.21$\pm$9.01 |
| control | 42 | 1 | 6 | 17 | 15 | 3 | 57.31$\pm$8.48 |
| rank sum test | | u=0.298 P=0.766 | | | | | t=0.06 P=0.95 |

Body weight comparison of two groups before treatment, there is no notable significance in the difference.

11. Appetite comparison of two groups before treatment

Table 14 Appetite comparison of two groups before treatment

| group | cases | <4 | 4-5.9 | 6-7.9 | >7.9 | $\overline{x}\pm s$ |
|---|---|---|---|---|---|---|
| treat group | 87 | 7 | 37 | 33 | 10 | 5.46$\pm$2.02 |
| control | 42 | 2 | 13 | 23 | 4 | 5.81$\pm$1.29 |
| rank sum test | | u=1.253 P=0.210 | | | | t=1.026 P=0.307 |

Appetite comparison of two groups before treatment, there is no notable significance in the difference.

12. Clinical symptoms and signs comparison of two groups before treatment

Table 15 Clinical symptoms comparison of two groups before treatment

| symptoms | group | cases | grade 0 | grade I | grade II | grade III | grade IV | u | P |
|---|---|---|---|---|---|---|---|---|---|
| gastric pain | treat group | 87 | 10 | 12 | 32 | 27 | 6 | 0.88 | 0.38 |
| | control | 42 | 3 | 11 | 16 | 9 | 3 | | |
| anorexia | treat group | 87 | 14 | 21 | 24 | 24 | 4 | 1.22 | 0.22 |
| | control | 42 | 5 | 14 | 18 | 4 | 1 | | |
| fatigue | treat group | 87 | 13 | 17 | 37 | 16 | 4 | 1.25 | 0.08 |
| | control | 42 | 9 | 12 | 15 | 6 | 0 | | |
| dry mouth thirst | treat group | 87 | 53 | 21 | 13 | 0 | 0 | 0.97 | 0.33 |
| | control | 42 | 29 | 9 | 4 | 0 | 0 | | |
| bitter taste of mouth | treat group | 87 | 53 | 24 | 8 | 2 | 0 | 1.64 | 0.10 |
| | control | 42 | 31 | 10 | 1 | 0 | 0 | | |
| spontaneous perspiration | treat group | 87 | 55 | 14 | 13 | 5 | 0 | 1.06 | 0.29 |
| | control | 42 | 30 | 6 | 6 | 0 | 0 | | |
| night sweat | treat group | 87 | 57 | 16 | 11 | 3 | 0 | 1.71 | 0.088 |
| | control | 42 | 33 | 7 | 2 | 0 | 0 | | |
| upset and tantrum | treat group | 87 | 58 | 17 | 10 | 2 | 0 | 1.46 | 0.15 |
| | control | 42 | 32 | 10 | 0 | 0 | 0 | | |

(continued)

| symptoms | group | cases | grade 0 | grade I | grade II | grade III | grade IV | u | P |
|---|---|---|---|---|---|---|---|---|---|
| dizziness | treat group | 87 | 65 | 19 | 2 | 1 | 0 | 0.49 | 0.62 |
|  | control | 42 | 33 | 8 | 1 | 0 | 0 |  |  |
| cancer pain | treat group | 87 | 14 | 18 | 35 | 17 | 3 | 3.25 | 0.001 |
|  | control | 42 | 13 | 13 | 15 | 1 | 0 |  |  |
| nausea and vomit | treat group | 87 | 49 | 17 | 18 | 2 | 1 | 0.66 | 0.51 |
|  | control | 42 | 26 | 9 | 4 | 3 | 0 |  |  |
| abdominal distension | treat group | 87 | 32 | 20 | 28 | 7 | 0 | 0.25 | 0.80 |
|  | control | 42 | 17 | 10 | 10 | 2 | 3 |  |  |

Cancer pain extent of treat group is higher than control group before treatment. other symptoms comparison of two groups before treatment ,there is no notable significance in the difference.

Table16 Abdominal lump size (cm×cm) comparison of two groups before treatment

| group | cases | $\bar{x}\pm s$ |
|---|---|---|
| treat group | 20 | 51.95±40.48 |
| control | 12 | 61.08±82.77 |
| t=0.419 | | P=0.678 |

Abdominal lump size (cm×cm) comparison of two groups before treatment, there is no notable significance in the difference..

Table 17 Abdominal lump texture comparison of two groups before treatment

| group | cases | hard | tenacious | soft |
|---|---|---|---|---|
| treat group | 20 | 12 | 6 | 2 |
| control | 12 | 9 | 3 | 0 |
| rank sum test | | u=0.98 | P=0.33 | |

Abdominal lump texture comparison of two groups before treatment, there is no notable significance in the difference.

Table 18 Abdominal lump tenderness comparison of two groups before treatment

| group | cases | no tenderness | tenderness |
|---|---|---|---|
| treat group | 20 | 2 | 18 |
| control | 12 | 4 | 8 |
| Fisher's exact test | | P=0.165 | |

Abdominal lump tenderness comparison of two groups before treatment, there is no notable significance in the difference.
13. Tongue demonstration comparison of two groups before treatment

Table 19 Tongue demonstration comparison of two groups before treatment

| group | cases | thin and white | thin and yellow | yellow and greasy | yellow and thick | others |
|---|---|---|---|---|---|---|
| treat group | 87 | 9 | 34 | 29 | 14 | 1 |
| control | 42 | 3 | 11 | 19 | 9 | 0 |
| Fisher's exact test | | | P=0.469 | | | |

Tongue demonstration comparison of two groups before treatment, there is no notable significance in the difference.

Table 20 Tongue texture comparison of two groups before treatment

| group | cases | carmoisine | red | dark red | purple black | cyanochroia | petechia,ecchymosis |
|---|---|---|---|---|---|---|---|
| treat group | 87 | 0 | 5 | 43 | 29 | 7 | 3 |
| control | 42 | 2 | 6 | 14 | 13 | 4 | 3 |

Fisher's exact test    P=0.099

Tongue texture comparison of two groups before treatment, there is no notable significance in the difference.

14. Pulse tracings comparison of two groups before treatment

Table 21 Pulse tracings comparison of two groups before treatment

| group | cases | even | astringent | string | smooth | deep | weak |
|---|---|---|---|---|---|---|---|
| treat group | 87 | 0 | 25 | 8 | 6 | 19 | 29 |
| control | 42 | 1 | 16 | 6 | 7 | 4 | 8 |

Fisher's exact test        P=0.048

Pulse tracings comparison of two groups before treatment, there is notable significance in the difference.

15. Hematology examinations of two groups

85 cases in treat group received WBC counting before treatment, 80 cases are normal, 3 cases WBC within $3.0\text{-}3.9\times10^9$/L, 2 cases WBC>$10.0\times10^9$/L.

41 cases in control group received WBC counting before treatment, 35 cases are normal, 4 cases WBC within $3.0\text{-}3.9\times10^9$/L, 2 cases WBC>$10.0\times10^9$/L.

42 cases in treat group received granulocyte counting before treatment, 41 cases are normal, 1 case granulocyte<$2.0\times10^9$/L

25 cases in control group received granulocyte counting before treatment, 23 cases are normal, 2 cases granulocyte<$2.0\times10^9$/L.

85 cases in treat group received hemoglobin test before treatment, 1case<60g/L, 5 cases within 60-79g/L, 31 cases within 80-109g/L, 48cases≥110g/L.

42 cases in control group received hemoglobin test before treatment, 4 cases within 60-79g/L, 18 cases within 80-109g/L, 20 cases≥110g/L.

84 cases in treat group received platelet counting before treatment, 81 cases are normal, 3 case platelets <$10.0\times10^9$/L.

42 cases in control group received platelet counting before treatment, 38 cases are normal, 4 case platelets <$10.0\times10^9$/L.

16. Imageology examinations of two groups before treatment

In treat group, 72 cases reiceived gastroscope examination, 50 cases received X-Raybarium meal examination or air-barium double contrast examination, 65 cases received B type ultrasonic examination,18 cases received CT examination.

In control group, 26 cases reiceived gastroscope examination, 23 cases received X-Raybarium meal examination or air-barium double contrast examination, 28 cases received B type ultrasonic examination, 12 cases received CT examination.

Above results of comparability check show that there is no notable significance in the difference resulted from the comparison of sex, age, disease courses ,past treatment methods, tumor type, tumor location, number and size, main clinical symptoms and signs, tongue demonstration of two groups before treatment, except that cancer pain extent of treat group is higher than that of control group. Suggesting that prognostic factors are uniform between two groups.

III.Therapeutic effects comparison

1.Total therapeutic effects comparison.

Table 22 Total therapeutic effects comparison

| group | cases | CR(%) | PR(%) | SD(%) | PD(%) |
|---|---|---|---|---|---|
| treat group | 87 | 1 | 15 | 63 | 8 |
| | | (1.1) | (17.2) | (72.4) | (9.2) |
| control | 42 | 0 | 1 | 29 | 12 |
| | | (0.0) | (2.4) | (69.0) | (28.6) |

(continued)

| group | cases | CR(%) | PR(%) | SD(%) | PD(%) |
|---|---|---|---|---|---|
| | | rank sum test | u=3.510 | P=0.000 | |

CR, PR, SD and PD rates of treat group are 1.1%,17.2%,72.4%,9.2% respectively, total remission rate is 18.3%; CR, PR, SD and PD rates of control group are 0%,2.4%,69.0% and 28.6% respectively, total remission is 2.4%. There is notable signicance in the difference between two groups.

1 complete remission case in treat group, male, 64 years old, two years before enrollment, was diagnosed as: gastric carcinoma (adenocarcinoma), received subtotal gastrectomy. The patient feel obstruction after meal, and aggravated gradually,then visited Changzhou cancer hospital, no measurable tumor foci, pathologic diagnosis showed: cancer cells are in ulcerative necrosis tissue (maybe adenocarcinoma).According to the trail regimen, treated the patient with the invented Traditional Chinese Medicine composition combined with chemotherapy. After treatment, pathologic examination showed: moderate reactive gastritis. Follow-up 7 months later and rechecked, the pathological examination showed: inflammation changes of mucosa. Therapeutic effects assessed as complete remission. Considering that the therapeutic effects assessment of this patient was not so confirm, so adopting "concessional conservation method" classified the effect as "Stable Disease", then performed clinical therapeutic effects comparison.results is shown in following table.

Table 23 Total therapeutic effects comparison ("concessional conservation method")

| group | cases | CR(%) | PR(%) | SD(%) | PD(%) |
|---|---|---|---|---|---|
| treat group | 87 | 0 | 15 | 64 | 8 |
| | | (0.0) | (17.2) | (73.6) | (9.2) |
| control | 42 | 0 | 1 | 29 | 12 |
| | | (0.0) | (2.4) | (69) | (28.6) |
| | | rank sum test | u=3.445 | P=0.001 | |

Above results show that there is notable significance in the difference of total therapeutic effects resulted from comparison of two groups (u=3.445, P=0.001), after adding 1 CR case into SD case via"concessional conservation method".

2. Therapeutic effects comparison of gastric carcinoma without metastasis between two groups

Table24 Therapeutic effects comparison of gastric carcinoma without metastasis between two groups

| group | cases | CR (%) | PR (%) | SD (%) | PD (%) |
|---|---|---|---|---|---|
| treat group | 62 | 1 | 10 | 45 | 6 |
| | | (1.6) | (16.1) | (72.6) | (9.7) |
| control | 27 | 0 | 1 | 18 | 8 |
| | | (0.0) | (3.7) | (66.7) | (29.6) |
| | | rank sum test | u=2.723 | P=0.006 | |

CR, PR, SD and PD rates of treat group are 1.6%,16.1%,72.6%,9.7% respectively, total remission rate is 17.7%; CR, PR, SD and PD rates of control group are 0%,3.7%,66.7% and 29.6% respectively, total remission is 3.7%. There is notable signicance in the difference between two groups.

3. Therapeutic effects comparison of gastric carcinoma with metastasis between two groups

Table 25 Therapeutic effects comparison of gastric carcinoma with metastasis between two groups

| group | cases | CR(%) | PR(%) | SD(%) | PR(%) |
|---|---|---|---|---|---|
| treat group | 25 | 0 | 5 | 18 | 2 |
| | | (0.0) | (20) | (72) | (8) |
| control | 15 | 0 | 0 | 11 | 4 |
| | | (0.0) | (0.0) | (73.3) | (26.7) |
| | | rank sum test | u=2.228 | P=0.026 | |

CR, PR, SD and PD rates of treat group are 0.0%,20.0%,72.0%,8.0% respectively, total remission rate is 20.0%; CR, PR, SD and PD rates of control group are 0%,0.0%,73.3% and 26.7% respectively, total remission is 0.0% . There is notable signicance in the difference between two groups.

4. Follow-up life span and survival rate comparison of two groups

Table 26 Death comparison of two groups 1.5 years after treatment

| group | cases | survival | death (cause) | | |
|---|---|---|---|---|---|
| | | | upper gastrointestinal bleeding | failure | others |
| treat group | 87 | 16 | 10 | 60 | 1 |
| control | 42 | 6 | 11 | 21 | 4 |
| Combine death cases | $X^2$=0.34 | | P=0.56 | | |

Death comparison of two groups 1.5 years after treatment, there is no notable significance in the difference.

Table 27 Life span and survival comparison of two groups 1.5 year after treatment (I)*

| group | cases | complete data cases | censored | %cencored |
|---|---|---|---|---|
| treat group | 87 | 71 | 16 | (18.4) |
| control | 41 | 35 | 6 | (14.6) |
| *1 case in control group did not finish treatment course, died 1 month after treatment. | | | | |

Table 28 Life span and survival comparison of two groups 1.5 year after treatment (II) *

| group | cases | Average live time(month) | Median live time(month) | 1 year survival rate | |
|---|---|---|---|---|---|
| | | $\overline{x}\pm s$ | $\overline{x}\pm s$ | % | se |
| treat group | 87 | 10±1 | 10±1 | 32.83 | 5.1 |
| control | 41 | 8±1 | 6±1 | 24.39 | 6.7 |
| short term effect: Breslow test, statistic=4.22 | | | P=0.040 | | |
| Long term effect:Log-rank test, statistic =1.98 | | | P=0.1597 | | |

  * 1 case in control group did not finish treatment course, died 1 month after treatment.

Life span and survival comparison of two groups 1.5 year after treatment:survival rate of treat group and control group are 32.83%, 24.39% respectively, there is notable significance in the difference of short term effect resulted from comparison of two groups ; but there is no notable significance in the difference of Long term effect resulted from comparison of two groups . Indicating short term effect of treat group is better than that of control group.

5. Life span and survival rate comparison of gastric carcinoma without metastasis between two groups after treatment

Table 29 Life span and survival rate comparison of gastric carcinoma without metastasis between two groups after treatment (I)

| group | cases | complete data cases | censored | % censored |
|---|---|---|---|---|
| treat group | 62 | 49 | 13 | (21) |
| control | 27 | 21 | 6 | (22.2) |

Table 30 Life span and survival rate comparison of gastric carcinoma without metastasis between two groups after treatment (II)

| group | cases | Average live time (month) | Median live time (month) | 1 year survival rate | |
|---|---|---|---|---|---|
| | | $\overline{x}\pm s$ | $\overline{x}\pm s$ | % | se |
| treat group | 62 | 10±1 | 10±1 | 31.27 | 0.061 |
| control | 27 | 10±1 | 7±2 | 33.33 | 0.091 |
| short term effect: Breslow test , statistic=4.22=0.05 | | | P=0.824 | | |
| Long term effect:Log-rank test, statistic =1.98=0.56 | | | P=0.454 | | |

Life span and survival rate comparison of two groups 1.5 year after treatment: survival rate of treat group and control group are 31.27%, 33.33% respectively, There is no notable significance in the difference of short term effect and long term effect resulted from comparison of two groups. Short term effect and long term effects on gastric carcinoma without metastasis, treat group are equal to control group after treatment.

6.Life span and survival rate comparison of gastric carcinoma with metastasis between two groups after treatment

Table 31 Life span and survival rate comparison of gastric carcinoma with metastasis between two groups after treatment (I)*

| group | cases | complete data cases | censored | % censored |
|---|---|---|---|---|
| treat group | 25 | 22 | 3 | (12) |
| control | 14 | 14 | 0 | (0.0) |

\* 1 case in control group did not finish therapy course, died 1 month after treatment.

Table 32 Life span and survival rate comparison of gastric carcinoma with metastasis between two groups after treatment (II)*

| group | cases | Average live time(month) | Median live time (month) | 1year survival rate | |
|---|---|---|---|---|---|
| | | $\overline{x}\pm s$ | $\overline{x}\pm s$ | % | se |
| treat group | 25 | 9±1 | 7±2 | 36.0 | 0.096 |
| control | 14 | 5±1 | 4±1 | 7.14 | 0.069 |
| short term effect: Breslow test, statistic =5.72 | | | P=0.0168 | | |
| Long term effect:Log-rank test, statistic =7.56 | | | P=0.0060 | | |

\* 1 case in control group did not finish therapy course, died 1 month after treatment.

Life span and survival rate comparison of two groups 1.5 year after treatment:survival rate of treat group and control group are 36.0%, 7.14% respectively. There is no notable significance in the difference of short term effect and long term effect resulted form comparison of two groups. Short term effects and long term effects on gastric carcinoma with metastasis, treat group are better than control group after treatment.

7. Tumor foci size comparison of two groups after treatment

Table 33 Tumor foci size comparison of two groups after treatment

| group | cases | before treatment $\overline{x}\pm s$ | after treatment $\overline{x}\pm s$ | difference (after-before) $\overline{x}\pm s$ | t | P |
|---|---|---|---|---|---|---|
| treat group | 83 | 27.04±22.32 | 21.92±23.65 | -5.12±8.86 | 5.262 | 0.000 |
| control | 40 | 26.44±48.58 | 26.00±46.44 | -0.44±7.31 | 0.378 | 0.708 |
| | | t=0.095 | t=0.646 | t=2.897 | | |

(continued)

| group | cases | before treatment $\overline{x}\pm s$ | after treatment $\overline{x}\pm s$ | difference (after-before) $\overline{x}\pm s$ | t | P |
|---|---|---|---|---|---|---|
| | | P=0.925 | P=0.519 | P=0.004 | | |

Tumor foci size comparison of treat group before and after treatment, there is notable significance in the difference.

Tumor foci size comparison of control group before and after treatment, there is no notable significance in the difference.

Tumor foci size difference (after-before) comparison of two groups, there is notable significance in the difference.

8. Karnofsky scores comparison of two groups after treatment

Table 34 Karnofsky scores comparison of two groups after treatment

| group | cases | before treatment $\overline{x}\pm s$ | after treatment $\overline{x}\pm s$ | difference (after-before) $\overline{x}\pm s$ | t | P |
|---|---|---|---|---|---|---|
| treat group | 87 | 66.78±8.83 | 81.15±8.13 | 14.37±10.53 | 12.72 | 0.000 |
| control | 42 | 69.05±9.32 | 74.06±11.06 | 5.00±13.84 | 2.34 | 0.024 |
| | | t=1.340 P=0.180 | t=8.434 P=0.000 | t=4.261 P=0.000 | | |

Karnofsky scores comparison of treat group before and after treatment, there is notable significance in the difference .

Karnofsky scores comparison of control group before and after treatment, there is notable significance in the difference .

Karnofsky scores difference (after-before) comparison of two groups, there is notable significance in the difference .

9. Body weight comparison of two groups after treatment

Table 35 Body weight (Kg) comparison of two groups after treatment

| group | cases | before treatment $\overline{x}\pm s$ | after treatment $\overline{x}\pm s$ | difference (after-before) $\overline{x}\pm s$ | t | P |
|---|---|---|---|---|---|---|
| treat group | 87 | 57.21±9.01 | 58.16±9.04 | 0.95±6.66 | 1.34 | 0.19 |
| control | 42 | 57.31±8.48 | 58.36±8.24 | 1.05±7.59 | 0.89 | 0.38 |
| | | t=0.06 P=0.95 | t=0.119 P=0.906 | t=0.071 P=0.943 | | |

Body weight comparison of treat group before and after treatment, there is no notable significance in the difference.

Body weight comparison of control group before and after treatment , there is no notable significance in the difference.

Body weight difference (after-before) comparison of two groups , there is no notable significance in the difference.

10. Appetite comparison of two groups after treatment

Table36 Appetite (taels/day) comparison of two groups after treatment

| group | cases | before treatment $\overline{x}\pm s$ | after treatment $\overline{x}\pm s$ | difference (after-before) $\overline{x}\pm s$ | t | P |
|---|---|---|---|---|---|---|
| treat group | 87 | 5.47±2.01 | 7.93±6.36 | 2.46±5.79 | 3.97 | 0.000 |
| control | 42 | 5.81±1.29 | 6.62±1.81 | 0.81±2.23 | 2.35 | 0.024 |
| | | t=0.995 P=0.322 | t=1.309 P=0.193 | t=1.782 P=0.077 | | |

Appetite comparison of treat group before and after treatment, there is notabe significance in the difference.

Appetite comparison of control group before and after treatment, there is notabe significance in the difference.

Appetite difference (after-before) comparison of two groups, there is no notable significance in the difference.

11. Fatigue improvement comparison of two groups after treatment

Table 37 Fatigue improvement comparison of two groups after treatment

| group | cases | aggravation | no change | Improved 1 grade | Improved 2 grades | Improved 3 grades | Improved 4 grades |
|---|---|---|---|---|---|---|---|
| treat group | 74 | 1 | 7 | 27 | 32 | 5 | 2 |
| control | 33 | 3 | 5 | 16 | 6 | 3 | 0 |
| | | | rank sum test | u=2.42 | P=0.015 | | |

* Improved 1 grade: lowered 1 grade after treatment compared with before treatment.
Improved 2 grades: lowered 2 grades after treatment compared with before treatment.
Improved 3 grades: lowered 3 grades after treatment compared with before treatment.
Improved 4 grades :lowered 4 grades after treatment compared with before treatment.

Fatigue improvement comparison of two groups after treatment, there is notabe significance in the difference.
12. Clinical symptoms and signs improvement comparison of two groups after treatment

Table 38 Clinical symptoms and signs improvement comparison of two groups after treatment*

| symptoms | group | cases | agg rav atio n | no change | Improved 1 grade | Improved 2 grades | Improved 3 grades | Improved 4 grades | u | P |
|---|---|---|---|---|---|---|---|---|---|---|
| gastric pain | treat group | | | | | | | | 2.43 | |
| | | 77 | 0 | 9 | 23 | 32 | 13 | 0 | | 0.02 |
| | control | 39 | 2 | 7 | 16 | 11 | 2 | 1 | | |
| anorexia | treat group | | | | | | | | 2.82 | |
| | | 73 | 0 | 10 | 24 | 29 | 9 | 1 | | 0.01 |
| | control | 37 | 3 | 6 | 19 | 7 | 1 | 1 | | |
| dry mouth thirst | treat group | | | | | | | | 0.61 | |
| | | 34 | 0 | 3 | 24 | 7 | 0 | 0 | | 0.54 |
| | control | 13 | 1 | 2 | 7 | 3 | 0 | 0 | | |
| bitter taste of mouth | treat group | | | | | | | | 2.47 | |
| | | 34 | 0 | 5 | 22 | 5 | 2 | 0 | | 0.01 |
| | control | 11 | 1 | 4 | 6 | 0 | 0 | 0 | | |
| spontaneous perspiration | treat group | | | | | | | | 1.48 | |
| | | 32 | 0 | 1 | 17 | 10 | 4 | 0 | | 0.14 |
| | control | 12 | 1 | 2 | 5 | 4 | 0 | 0 | | |
| night sweat | treat group | | | | | | | | 2.20 | |
| | | 30 | 0 | 3 | 18 | 7 | 2 | 0 | | 0.03 |
| | control | 9 | 2 | 2 | 4 | 1 | 0 | 0 | | |
| upset and tantrum | treat group | | | | | | | | 3.02 | |
| | | 29 | 0 | 2 | 15 | 11 | 1 | 0 | | 0.01 |
| | control | 10 | 1 | 3 | 6 | 0 | 0 | 0 | | |
| dizziness | treat group | | | | | | | | 1.39 | |
| | | 22 | 0 | 2 | 17 | 2 | 1 | 0 | | 0.17 |
| | control | 9 | 2 | 1 | 5 | 1 | 0 | 0 | | |
| cancer pain | treat group | | | | | | | | 2.41 | |
| | | 73 | 1 | 6 | 29 | 23 | 12 | 2 | | 0.02 |
| | control | 29 | 2 | 2 | 17 | 8 | 0 | 0 | | |
| nausea and vomit | treat group | | | | | | | | 2.35 | |
| | | 38 | 0 | 1 | 22 | 12 | 2 | 1 | | 0.02 |
| | control | 16 | 0 | 5 | 8 | 2 | 1 | 0 | | |

| symptoms | group | cases | agg rav atio n | no change | Improved 1 grade | Improved 2 grades | Improved 3 grades | Improved 4 grades | u | P |
|---|---|---|---|---|---|---|---|---|---|---|
| abdominal distension | treat group | | | | | | | | 1.24 | |
| | | 55 | 1 | 5 | 29 | 14 | 6 | 0 | | 0.22 |
| | control | 25 | 2 | 5 | 11 | 4 | 0 | 3 | | |

* Improved 1 grade: lower 1 grade after treatment compared with before treatment, for example,grade IV of anorexia"without appetite, appetite decreased >1/2"; improved to grade III of anorexia"without appetite, appetite decreased within1/3 to1/2", analogized sequentially.

Improvement extent comparison of gastric discomfort, anorexia, bitter mouth, night sweat, upset and tantrum, cancer pain, nausea between two groups after treatment, there is notable significance in the difference.
Improvement extent comparison of dry mouth, thirst, spontaneous perspiration, dizziness and abdominal distension between two groups after treatment, there is no notable significance in the difference

13. Main symptoms and signs disappearance rates comparison of two groups after treatment

Table 39 Main symptoms and signs disappearance rates comparison of two groups after treatment (I)

| group | | gastric discomfort | anorexia | fatigue | dry mouth thirst |
|---|---|---|---|---|---|
| treat group | original cases | 77 | 73 | 74 | 34 |
| | disappearance cases | 30 | 35 | 39 | 26 |
| | disappearance rate | 38.96 | 47.95 | 52.7 | 76.47 |
| control | original cases | 39 | 37 | 33 | 13 |
| | disappearance cases | 16 | 18 | 16 | 10 |
| | disappearance rate | 41.03 | 48.65 | 48.48 | 76.92 |
| | $X^2$ (correction) | 0.05 | 0.00 | 0.16 | Fisher's exact test |
| | p | 0.83 | 0.94 | 0.69 | 1.00 |

Table 40 Main symptoms and signs disappearance rates comparison of two groups after treatment (II)

| group | | bitter mouth | spontaneous perspiration | night sweat | upset and tantrum |
|---|---|---|---|---|---|
| treat group | original cases | 34 | 32 | 30 | 29 |
| | disappearance cases | 27 | 28 | 23 | 26 |
| | disappearance rate | 79.41 | 87.5 | 76.67 | 89.66 |
| control | original cases | 11 | 12 | 9 | 10 |
| | disappearance cases | 6 | 8 | 5 | 6 |
| | disappearance rate | 54.55 | 66.67 | 55.56 | 60 |
| | $X^2$ (correction) | Fisher's exact test | Fisher's exact test | Fisher's exact test | Fisher's exact test |
| | P | 0.24 | 0.18 | 0.23 | 0.057 |

Table 41 Main symptoms and signs disappearance rates comparison of two groups after treatment (III)

| group | | dizziness | Cancer pain | nausea and vomit | abdominal distension |
|---|---|---|---|---|---|
| treat group | original cases | 22 | 73 | 38 | 55 |
| | disappearance cases | 20 | 44 | 31 | 35 |
| | disappearance rate | 90.91 | 60.27 | 81.58 | 63.64 |
| control | original cases | 9 | 29 | 16 | 25 |
| | disappearance cases | 6 | 16 | 8 | 11 |
| | disappearance rate | 66.67 | 55.17 | 50 | 44 |

(continued)

| group | dizziness | Cancer pain | nausea and vomit | abdominal distension |
|---|---|---|---|---|
| $X^2$ (correction) | Fisher's exact test | 0.22 | Fisher's exact test | 2.71 |
| P | 0.13 | 0.64 | 0.043 | 0.099 |

Disappearce rates comparison of nausea and vomit, there is notable significance in the difference.

Disappearce rates comparison of gastric discomfort, anorexia, fatigue, dry mouth, thirst, dizziness, bitter mouth, spontaneous perspiration, night sweat, upset and tantrum, dizziness, cancer pain, abdominal distension of two groups after treatment, there is no notable significance in the difference.

14. WBC counting comparison of two groups after treatment

Table 42 WBC counting comparison of two groups after treatment *

| group | cases | aggravation | no change | Improved 1 grade |
|---|---|---|---|---|
| treat group | 83 | 2 | 80 | 1 |
| control | 39 | 5 | 33 | 1 |
| | rank sum test | u=1.757 | P=0.079 | |

* Grade WBC accurately according to "WHOgrade criterions for acute and subacute toxicity reactions of the anti-cancer drugs", see details in application example 1.

There is no notable significance in the difference of WBC counting.

15. Granulocytes counting comparison of two groups after treatment

Table 43 Granulocytes counting comparison of two groups after treatment*

| group | cases | aggravation | no change | Improved 1 grade | Improved 2 grades | Improved 3 grades |
|---|---|---|---|---|---|---|
| treat group | 41 | 1 | 39 | 1 | 0 | 0 |
| control | 22 | 2 | 18 | 1 | 0 | 1 |
| | rank sum test | | u=0.01 | P=0.99 | | |

* Grade granulocytes accurately according to "WHOgrade criterions for acute and subacute toxicity reactions of the anti-cancer drugs",see details in application example 1.

There is no notable significance in the difference of Granulocytes counting.

16. Hemoglobin comparison of two groups after treatment

Table 44 Hemoglobin comparison of two groups after treatment*

| group | cases | aggravation | no change | Improved 1 grade | Improved 2 grades | Improved 3 grades |
|---|---|---|---|---|---|---|
| treat group | 83 | 8 | 53 | 15 | 5 | 2 |
| control | 40 | 9 | 25 | 2 | 3 | 1 |
| | rank sum test | | u=1.927 | P=0.054 | | |

* Grade hemoglobin accurately according to "WHOgrade criterions for acute and subacute toxicity reactions of the anti-cancer drugs".see details in application example 1.

There is no notable significance in difference of Hemoglobin (P=0.054 close to the clinical value), Hemoglobin of treat group has tend to be increased, compared with control group.

17. Platelets counting comparison of two groups after treatment

Table 45 Platelets counting comparison of two groups after treatment *

| group | cases | aggravation | no change | Improved 1 grade | Improved 2 grades | Improved 3 grades |
|---|---|---|---|---|---|---|
| treat group | 82 | 1 | 79 | 2 | 0 | 0 |
| control | 40 | 3 | 34 | 3 | 0 | 0 |
| | | rank sum test | u=0.223 | P=0.824 | | |

*Grade platelets accurately according to "WHOgrade criterions for acute and subacute toxicity reactions of the anti-cancer drugs", see details in application example 1.

There is no notable significantce in the difference of Platelets counting.

18. .Immune function comparison of two groups after treatment

Table 46 CD3 comparison of two groups before and after treatment

| group | cases | before treatment $\overline{x}\pm s$ | 8 weeks after treatment $\overline{x}\pm s$ | difference (after-before) $\overline{x}\pm s$ | t | p |
|---|---|---|---|---|---|---|
| treat group | 52 | 52.51±8.16 | 57.67±9.04 | 4.84±5.64 | 6.12 | 0.000 |
| control | 21 | 50.44±9.64 | 48.19±9.43 | -1.25±6.19 | 0.808 | 0.432 |
| | | t=0.93 P=0.35 | t=3.62 P=0.00057 | t=3.68 P=0.00047 | | |

CD3 comparison of two groups before treatment, there is no notable significance in the difference..

CD3 comparison of two groups after treatment, there is notable significance in the difference .

CD3 difference (after-before) comparison of two groups,there is notable significance in the difference.

CD3 comparison of treat group before and after treatment,there is notable significance in the difference (t=6.12, P=0.000).

CD3 comparison of control group before and after treatment, there is no notable significance in the difference. (t=0.808, P=0.432).

Table 47 CD4 comparison of two groups before and after treatment

| group | cases | before treatment $\overline{x}\pm s$ | 8 weeks after treatment $\overline{x}\pm s$ | difference (after-before) $\overline{x}\pm s$ | t | p |
|---|---|---|---|---|---|---|
| treat group | 52 | 36.08±7.08 | 40.18±6.87 | 3.78±4.22 | 6.41 | 0.000 |
| control | 21 | 31.94±9.30 | 32.00±9.54 | -0.63±6.44 | 0.39 | 0.703 |
| | | t=2.06 P=0.043 | t=3.77 P=0.00036 | t=3.19 P=0.0022 | | |

CD4 comparison of two groups before treatment, there is notable significance in the difference.

CD4 comparison of two groups after treatment,there is notable significance in the difference.

CD4 after-before) of comparison of two groups,there is notable significance in the difference.

CD4 comparison of treat group before and after treatment. (t=6.41, P=0.000)., there is notable significance in the difference.

CD4comparison of control group before and after treatment (t=0.39, P=0.703),there is no notable significance in the difference..

Table 48 CD8 comparison of two groups before and after treatment

| group | cases | before treatment $\overline{x}\pm s$ | 8 weeks after treatment $\overline{x}\pm s$ | difference (after-before) $\overline{x}\pm s$ | t | p |
|---|---|---|---|---|---|---|
| treat group | 51 | 28.67±8.54 | 25.69±4.35 | -3.00±8.06 | 2.66 | 0.011 |

(continued)

| group | cases | before treatment $\overline{x}\pm s$ | 8 weeks after treatment $\overline{x}\pm s$ | difference (after-before) $\overline{x}\pm s$ | t | p |
|---|---|---|---|---|---|---|
| control | 21 | 28.25±10.32 | 26.06±10.34 | -1.19±3.62 | 1.31 | 0.21 |
| | | t=0.18 P=0.85 | t=0.21 P=0.83 | t=0.87 P=0.39 | | |

CD8 comparison of two groups before treatment, there is no notable significance in the difference .

CD8 comparison of two groups after treatment,there is no notable significance in the difference.

CD8 (alter-before) comparison of two groups,there is no notable significance in the difference.

CD8 of comparison of treat group before and after treatment (t=2.66, P=0.011),there is notable significance in the difference .

CD8 comparison of control group before and after treatment (t=1.31, P=0.21),there is no notable significance in the difference.

Table 49 CD4/CD8 comparison of two groups before and after treatment

| group | cases | before treatment $\overline{x}\pm s$ | 8 weeks after treatment $\overline{x}\pm s$ | difference (after-before) $\overline{x}\pm s$ | t | p |
|---|---|---|---|---|---|---|
| treat group | 51 | 1.31±0.32 | 1.59±0.38 | 0.22±0.42 | 6.85 | 0.000 |
| control | 21 | 1.19±0.26 | 1.28±0.38 | 0.06±0.25 | 0.61 | 0.55 |
| | | t=1.54 P=0.13 | t=2.84 P=0.0059 | t=1.39 P=0.17 | | |

CD4/CD8 comparison of two groups before treatment,there is no notable significance in the difference.

CD4/CD8 comparison of two groups after treatment,there is notable significance in the difference.

CD4/CD8 (after-before) comparison of two groups,there is no notable significance in the difference.

CD4/CD8 comparison of treat group before and after treatment (t=6.85, P=0.000).,there is notable significance in the difference.

CD4/CD8 comparison of control group before and after treatment (t=0.61, P=0.55),there is no notable significance in the difference.

Table 50 NK cell comparison of two groups before and after treatment

| group | cases | before treatment $\overline{x}\pm s$ | 8 weeks after treatment $\overline{x}\pm s$ | difference (after-before) $\overline{x}\pm s$ | t | p |
|---|---|---|---|---|---|---|
| treat group | 12 | 16.17±1.80 | 18.18±3.36 | 2.00±1.71 | 4.05 | 0.002 |
| control | 11 | 16.65±6.22 | 15.73±3.12 | -1.33±1.75 | 1.79 | 0.134 |
| | | t=0.26 P=0.80 | t=1.49 P=0.16 | t=3.88 P=0.0013 | | |

NK cell comparison of two groups before treatment,there is no notable significance in the difference .

NK cell comparison of two groups after treatment,there is no notable significance in the difference

NK cell (after-before) comparison of two groups,there is notable significance in the difference

NK cell comparison of treat group before and after treatment (t=4.05, P=0.002)., there is notable signficance in the difference.

NK cell comparison of control group before and after treatment (t=1.79, P=0.134),there is no notable significance in the difference.

19.CEA comparison of two groups before and after treatment

Table 51 CEA comparison of two groups before and after treatment

| group | cases | before treatment $\overline{x}\pm s$ | 8 weeks after treatment $\overline{x}\pm s$ | difference (after-before) $\overline{x}\pm s$ | t | p |
|---|---|---|---|---|---|---|
| treat group | 68 | 18.69±20.27 | 17.15±18.27 | -1.27±12.41 | 0.69 | 0.49 |
| control | 31 | 17.58±19.79 | 17.04±20.17 | 0.37±4.81 | 0.40 | 0.69 |
| | | t=0.25 P=0.79 | t=0.03 P=0.98 | t=0.37 P=0.72 | | |

CEA comparison of two groups before treatment,there is no notable significance in the difference
CEA comparison of two groups after treatment,there is no notable significance in the difference
CEA (after-before) comparison of two groups,there is no notable significance in the difference.
CEA comparison of treat group before and after treatment (t=0.69, P=0.49),there is no notable significance in the difference.
CEA comparison of control group before and after treatment (t=0.40, P=0.69),there is no notable significance in the difference.
20. Bleeding time and coagulation time comparison of two groups before and after treatment

Table 52 Bleeding time (second) comparison of two groups before and after treatment

| group | cases | before treatment $\overline{x}\pm s$ | 8 weeks after treatment $\overline{x}\pm s$ | difference (after-before) $\overline{x}\pm s$ | t | p |
|---|---|---|---|---|---|---|
| treat group | 40 | 115.08±22.35 | 117.76±20.19 | 2.68±18.49 | 0.88 | 0.38 |
| control | 20 | 107.25±29.36 | 109.17±35.74 | 3.33 ±22.29 | 0.63 | 0.53 |
| | | t=1.15 P=0.26 | t=1.14 P=0.26 | t=0.12 P=0.91 | | |

Bleeding time comparison of two groups before treatment, there is no notable significance in the difference .
Bleeding time comparison of two groups after treatment,there is no notable significance in the difference
Bleeding time (after-before) comparison of two groups,there is no notable significance in the difference
Bleeding time comparison of treat group before and after treatment (t=0.88, P=0.38),there is no notable significance in the difference .
Bleeding time comparison of control group before and after treatment (t=0.63, P=0.53),. there is no notable significance in the difference .

Table 53 Coagulation time (second) comparison of two groups before and after treatment

| group | cases | before treatment $\overline{x}\pm s$ | 8 weeks after treatment $\overline{x}\pm s$ | difference (after-before) $\overline{x}\pm s$ | t | p |
|---|---|---|---|---|---|---|
| treat group | 40 | 156.33±28.09 | 154.51±31.91 | -0.70±15.69 | 0.27 | 0.79 |
| control | 20 | 158.50±40.91 | 154.72±42.51 | 1.94±16.64 | 0.49 | 0.63 |
| | | t=0.24 P=0.81 | t=0.02 P=0.98 | t=0.58 P=0.57 | | |

Coagulation time comparison of two groups before treatment, there is no notable significance in the difference .
Coagulation time comparison of two groups after treatment,there is no notable significance in the difference
Coagulation time difference (after-before) comparison of two groups, there is no notable significance in the difference
Coagulation time comparison of treat group before and after treatment (t=0.27, P=0.79),there is no notable significance in the difference.
Coagulation time comparison of control group before and after treatment (t=0.49, P=0.63),there is no notable significance in the difference.

IV. Safety assessment

All safety indexes are normal before treatment, the safety assessment results of the abnormal after treatment are recorded in following table:

| doubtful adverse reaction | treat group | | control group | | RR | P |
|---|---|---|---|---|---|---|
| | positive cases/total cases | incidence rate | positive cases/total cases | incidence rate | | |
| Hemoglobin reduction | 3/48 | 6.3% | 4/20 | 20.0% | 0.31 | 0.182 |
| WBC reduction | 2/80 | 2.5% | 4/35 | 11.4% | 0.22 | 0.069 |
| Granulocyte reduction | 1/42 | 0.5% | 1/26 | 3.8% | 0.62 | 1.00 |
| Platelet reduction | 1/81 | 1.2% | 3/38 | 7.9% | 0.16 | 0.095 |
| Bilirubin increase | 5/72 | 6.9% | 4/35 | 11.4% | 0.61 | 0.470 |
| AKP rising | 5/56 | 8.9% | 5/36 | 13.9% | 0.64 | 0.505 |
| GPT rising | 2/79 | 2.5% | 1/37 | 2.7% | 0.94 | 1.00 |
| BUN rising | 2/81 | 2.5% | 5/38 | 13.2% | 0.19 | 0.033 |
| serum creatinine rising | 0/85 | 0.0% | 2/42 | 4.8% | | 0.108 |
| Abnormal urine protein | 1/85 | 1.2% | 1/39 | 2.6% | 0.46 | 0.532 |
| Abnomal urine WBC | 0/86 | 0.0% | 1/42 | 2.4% | | 0.328 |
| Abnomal urine | 0/85 | 0.0% | 1/41 | 2.4% | | 0.325 |
| RBC mucous stool | 0/79 | 0.0% | 3/41 | 7.3% | | 0.038 |
| faeces RBC abnormality | 2/79 | 2.5% | 3/41 | 7.3% | 0.35 | 0.337 |
| faeces WBC abnormality | 3/86 | 3.5% | 3/42 | 7.1% | 0.49 | 0.393 |
| fecal occult blood | 0/51 | 0.0% | 1/22 | 4.5% | | 0.301 |

Safety indexes assessment results of two groups after treatment demonstrate that incidence rates of BUN rising , mucous stool of treat group are lower than those of control group (P<0.05) ; The statistical analysis results of incidence rate demonstrate that there is no notable significance in the difference of other items of two groups. See details in above table.

V .Adverse event observations

Table 54 Results of adverse event observations of two groups after treatment

| doubtful adverse reaction | treat group (n=87) | | control group (n=42) | | RR | P |
|---|---|---|---|---|---|---|
| | positive cases* | incidence rate | positivecases | incidence rate | | |
| alopecie | 9 | 10.3% | 4 | 9.5% | 1.09 | 1.000 |
| oral cavity ulcer | 1 | 1.1% | 1 | 2.4% | 0.48 | 0.547 |
| cutaneous reaction | 1 | 1.1% | 0 | 0% | | 1.000 |
| choking and short of breath | 4 | 4.6% | 2 | 4.8% | 0.97 | 1.000 |
| jaundice | 1 | 1.1% | 4 | 9.5% | 0.12 | 0.039 |
| non cancer pain | 4 | 4.6% | 6 | 14.3% | 0.32 | 0.077 |
| hypersensitiveness | 3 | 3.4% | 2 | 4.8% | 0.72 | 0.660 |
| hemafecia | 2 | 2.3% | 3 | 7.1% | 0.32 | 0.329 |

| (continued) | | | | | | |
|---|---|---|---|---|---|---|
| doubtful adverse reaction | treat group (n=87) | | control group (n=42) | | RR | P |
| | positive cases* | incidence rate | positivecases | incidence rate | | |
| constipation | 1 | 1.1% | 2 | 4.8% | 0.24 | 0.247 |
| diarrhea | 5 | 5.7% | 4 | 9.5% | 0.60 | 0.472 |
| haematemesis | 0 | 0% | 0 | 0% | | |

*positive cases: if one patient had one symptom, recorded as one case, if one patient had two symptoms, recorded as two cases.

During the courses of treatment, some patients in both groups had adverse reaction including baldness, oral ulcer. The incidence rates of symptoms and statistical comparison results of two groups demonstrate that jaundice incidence rate of treat group is lower than that of control groups, and there is no notable significance in difference of other adverse reaction of two groups. see details in above table.

There are 25 cases in treat group who has at least one doubtful adverse reaction , and total incidence of doubtful adverse reaction is 28.7%(25/87); there are 17 cases in control group who had at least one doubtful adverse reaction, and total incidence of doubtful adverse reaction is 40.5% (17/42), there is no notable significance in difference of adverse reaction of two groups (RR=0.71, P=0.182).

Conclusions

129 eligible tested object, of which, 87 cases in treat group, 42 cases in control group; all are diagnosed as gastric carcinoma by westen medicine and as symptomof stagnation of poison by Traditional Chinese Medicine. 15 outpatient cases, 114 inpatient cases.

Results of comparability check show that there is no notable significance in differences of sex, age, disease courses ,past treatment methods, tumor type, tumor location, number and size, main clinical symptoms and signs, tongue demonstration of two groups before treatment , except that cancer pain of treat group is more than that of control group. It suggests that prognostic main factors are uniform between two groups with comparability.

Results of total clinical therapeutic effects demonstrate:

CR, PR, SD and PD rates of treat group are 1.1%,17.2%,72.4%,9.2% respectively, total remission rate is 18.3%; CR, PR, SD and PD rates of control group are 0%,2.4%,69.0% and 28.6% respectively, total remission is 2.4%. There are notable significance in difference of two groups.

Clinical therapeutic effects results on gastric carcinoma without metastasis show that CR, PR, SD and PD rates of treat group are 1.6%,16.1%,72.6%,9.7% respectively, total remission rate is 17.7%; CR, PR, SD and PD rates of control group are 0%,3.7%,66.7% and 29.6% respectively, total remission is 3.7% . There are notable significance in difference of two groups.

Clinical therapeutic effects results on gastric carcinoma with metastasis show that CR, PR, SD and PD rates of treat group are 0.0%,20.0%,72.0%,8.0% respectively, total remission rate is 20.0%; CR, PR, SD and PD rates of control group are 0%,0.0%,73.3% and 26.7% respectively, total remission is 0.0% . There are notable significance in difference of two groups.

Therapeutic effect of 1 case in treat group shown as "complete remission", Considering that the therapeutic effects assessment of this patient was not confirmed , so we adopted "concessional conservation method" and classified the effect as "Stable Disease", then performed clinical therapeutic effects comparison.There is notable significance in difference of the two groups (u=3.445, P=0.001), and results are shown in table23.

Above results indicate that the invented Traditional Chinese Medicine composition, combined with chemotherapy on treatment of gastric carcinoma(belonging to the sympton of stagnation of poison), has better clinical therapeutic effects, exerting certain synergistic functions and adjunctive therapeutic functions.

Results of life span and survival rate assessment:

1.5 year follow-up after treatment, survival rate of treat group and control group are 32.83%, 24.39% respectively, there is notable significance in difference of short term effect of two groups ;there is no notable significance in difference of. long term effect of two groups

For gastric carcinoma without metastasis patients, 1.5 year. follow-up after treatment, survival rate of treat group and control group are 31.27%, 33.33% respectively, there is no notable significance in difference of short term effect of two groups ;there is no notable significance in difference of. long term effect of two groups.

For gastric carcinoma metastasis patients, 1.5 year follow-up after treatment, survival rate of treat group and control group are 36.0%, 7.14% respectively, there is notable significance in difference of short term effect of two groups ; there is notable significance in difference of. long term effect of two groups

Indicating that the invented Traditional Chinese Medicine composition, serving as adjunctive therapy on chemotherapy of gastric carcinoma (considered as symptom of stagnation of poison by Traditional Chinese Medicine) short term effect is better than sole chemotherapy, long term effect is equal to sole chemotherapy(for gastric carcinoma patients with metastasis, short term effect and long term effect all are better than sole chemotherapy).

Results of solid tomor foci size assessment show:

There is notable significance in difference of tumor foci size difference (after-before) of two groups , minimized extent of the tumor foci size of treat group is greater than that of control group. There is notable significance in difference of tumor foci size of treat group before and after treatment , tumor minimized obviously after treatment compared with before treatment; There is no notable significance in difference of tumor foci size of control group before and after treatment . Suggesting that the invented Traditional Chinese Medicine composition, serving as adjunctive therapy on chemotherapy of gastric carcinoma (considered as symptom of stagnation of posison by Traditional Chinese Medicine), can minimize tumor foci obviously, which outweighs therapeutic effects of sole chemotherapy.

Results of main symptoms improvement show:

Karnofsky score of both treat group and control group increased obviously after treatment compared with that of before treatment, and the Karnofsky score increased extent of treat group is greater than control group; Fatigue of patients in both groups are improved obviously after treatment, fatigue improvement extent of treat group is greater than that of control group.

Appetite of patients in both groups increased obviously, symptoms including gastric discomfort, anorexia, dry mouth, thirst, bitter mouth, spontaneous perspiration, night sweat, upset,tantrum, cancer pain, nausea and vomit, abdominal distension , are all improved or have high disappearance rates, for improvement extent of gastric discomfort, anorexia, bitter mouth, night sweat, upset,tantrum, cancer pain, nausea and vomit , treat group is higher than that of control group; There is no notable significance in difference of improvement and disappearance rates of other symptoms between two groups .

Above results indicate that the invented Traditional Chinese Medicine composition, serving as adjunctive therapy on chemotherapy of gastric carcinoma, can ameliorate patients survival qualities, improve clinical symptoms and has better adjunctive therapeutical effects.

Laboratory examination results demonstrate:

WBC, RBC, platelet of two groups did not incease obviously after treatment compared with before treatment, hemoglobin of two groups increased obviously after treatment, and there is notable significance in difference of hemoglobin comparison of two groups after treatment ($P=0.054$), Hemoglobin of treat group has tend to be increased, compared with control group.

CD3,CD4 , CD4/CD8 and NK cells of treat group increased obviously after treatment compared with those of before treatment ($P<0.05$),furthermore, the CD3, CD4 and NK cell difference (after-before) of treat group are higher than those of control group($P<0.05$), while changes of CD3, CD4 , CD4/CD8 and NK cells of control group are not so obvious.

Above results indicate that the invented Traditional Chinese Medicine composition, serving as adjunctive therapy on chemotherapy of gastric carcinoma, has certain functions of stimulating immune responses, and can assist intervention chemotherapy to inhibit cancer cells.

Results of safety assessment show:

After treatment, some patients in two groups had hemoglobin reduction, WBC reduction, bilirubin rising, AKP rising, ALT rising, BUN rising(see details in"safety assessment"), incidence rates of BUN rising , mucous stool of treat group are lower than those of control group ($P<0.05$), there is notable significance in difference of other items of two groups .Considering that treat group received the invented Traditional Chinese Medicine composition based on chemotherapy, while control group received sole chemotherapy, it has already reported that the above side effects could also be seen during chemotherapy, so above safety assessment has not determined yet that the invented Traditional Chinese Medicine composition can damage hematopoietic systems and heart, liver functions. We should perform aggregate analysis using safety assessment data of sole treatment of primary hepatic carcinoma and gastric carcinoma with the invented Traditional Chinese Medicine composition (data23-1) .

Results of adverse event demonstrate:

During the courses of treatment, some patients in two groups had adverse reactions including alopecie, dental ulcer, scytitis, choking and short of breath, jaundice, hypersensitivity etc(see details in table54), jaundice incidence rate of treat group is lower than that of control groups, there is no notable significance in differences of other adverse events of two groups There are 25 cases in treat group who had at least one doubtful adverse reaction, total incidence of doubtful adverse reactions is 28.7%(25/87), there are 17 cases in control group who had at least one doubtful adverse reaction, total incidence of doubtful adverse reactions is 40.5% (17/42). There is no notable significance in difference of two groups (RR=0.71, P=0.182).

In summary, results of this randomized controlled trial demonstrate that the invented Traditional Chinese Medicine composition, combined with chemotherapy on treatment of gastric carcinoma, has better clinical therapeutic effects, can exert synergistic functions, can ameliorate patients survival qualities, improve clinical symptoms of patients and enhance cellular immune function of patients, it can be used as adjunctive therapy on chemotherapy of gastric carcinoma patients (considered as sympton of stagnation of poison by Traditional Chinese Medicine) and clinical application is quite safe.

**Claims**

1. A traditional chinese medicine preparation for anti-tumor, **characterized in that** it is composed of following parts by weight of materials: 1 part by weight of hydnocarpus, 0.8-1.4 parts by weight of cochinchina momordica seed, 0.5-1.1 parts by weight of pangolin scales, 0.8-1.3 parts by weight of rhubarb, 1-1.5 parts by weight of licorice root.

2. The traditional chinese medicine preparation according to claim 1, **characterized in that** dosages of all materials are 1 part by weight.

3. Method for producing the traditional chinese medicine preparation according to claim 1, comprising following steps:

   1) weighing each crude herb, grinding to middle size particles;
   2) adding 62% ethyl alocohol in a w/v of 1:2.5~1:3.5 with crude herbs and soaking thoroughly;
   3) heating and recirculating fully;
   4) filtrating, filter liquor acquired is the active ingredient solution of the traditional chinese medicine preparation mentioned in this invention.

4. Method according to claim 3, **characterized in that** the w/v in step 2 is 1:3

5. Method according to claim 3, **characterized in that** adding 62% ethyl alocohol in a w/v of 1:0.8~1:1.5 to the residues and gruffs gotten in step 4, heating again and recirculation thoroughly, filtrated and the filter liquor aquired also is the active ingredient solution of the invented traditional chinese medicine preparation.

6. Method according to claim 5, **characterized in that** the v/w is 1:1.

7. Method according to claim 5, **characterized in that** filter liquor acquired in this step is combined with filter liquor mentioned in claim 3, served as the active ingredient solution of the invented traditional chinese medicine preparation.

8. Method according to claim 3 or 5, **characterized in that** the heating and recirculation time is 0.5-1 hour.

9. Method of any one according to claim 3 to claim 7, **characterized in that** adjusting active ingredient solution with ethyl alocohol and water,changing ethyl alocohol volume percentage to 6.0-8.0%, adjusting pH to 4.0-5.0, then producing the composition of the invented traditional chinese medicine preparation.

10. Method according to claim 10, **characterized in that** the best relative density of this composition of the invented traditional chinese medicine preparation is 1.02-1.08.

**11.** Method of any one according to claim 3 to 7, **characterized in that** drying active ingredient solution of the traditional chinese medicine preparation mentioned above and making granula, filling into blank capsules and making capsules of the invented Traditional Chinese Medicine preparation.

**12.** Method of any one according to claim 3 to 7, **characterized in that** drying the active ingredient solution of the Traditional Chinese Medicine preparation mentioned above and compressing into round lamellar shape, this producing the tablets of the invented Traditional Chinese Medicine preparation.

**13.** Use of the traditional chinese medicine preparation according to claim 1 or 2 for preparing anti-cancer drug.

**14.** Use of the traditional chinese medicine preparation according to claim13, **characterized in that** the mentioned anti-cancer drugs refer to therapeutic drugs of tumors of digestive tract, lung and cervix uteri.

**15.** Use of the traditional chinese medicine preparation according to claim 15, **characterized in that** Anti-cancer drugs on digestive tract tumor mentioned refer to therapeutic drugs of gastric carcinoma, intestinal cancer and liver cancer.

# INTERNATIONAL SEARCH REPORT

International application No.

PCT/CN 2004/001367

**A.    CLASSIFICATION OF SUBJECT MATTER**

IPC(7):  A61K35/78， 35/36， A61P35/00

According to International Patent Classification (IPC) or to both national classification and IPC

**B.FIELDS SEARCHED**

Minimum documentation searched (classification system followed by classification symbols)

IPC(7):  A61K35/78， 35/36， A61P35/00

Documentation searched other than minimum documentation to the extent that such documents are included in the fields searched

Chinese Patent Applications Published Or Announced Since 1985 And Chinese Non-Pantent Documents

Electronic data base consulted during the international search (name of data base and, where practicable, search terms used)

JOPAL  CAPS(US)  PCB(CN)  MIMOSA(JP)  ESPACC—ACCESS  WPI  EPODOC  CPRS( cancer    tumour  *Semen Hydnocarpi , Momordicae Semen , Squama Manitis)*

**C.   DOCUMENTS CONSIDERED TO BE RELEVANT**

| Category* | Citation of document, with indication, where appropriate, of the relevant passages | Relevant to claim No. |
|---|---|---|
| A | CN 1103797A (Li wenzhong) 21 June 1995(21.06.95), entire document | 1-15 |
| A | CN 1095937A (Xiao yunfeng.) 07 Dec 1994(07.12.94) entire document | 1-15 |
| A | CN 1168278A (Shanghai DiKang Fygienical Food Developing CO.,LTD.) 24 Dec 1997(24.12.97), entire document | 1-15 |

☐ Further documents are listed in the continuation of Box C.          ☐ See patent family annex.

| | |
|---|---|
| *    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim (S) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 22 Feb 2005(22.02.05) | 10 · MAR 2005 (1 0 · 0 3 · 2 0 0 5) |

| Name and mailing address of the ISA/ | Authorized officer    Song  Jiangxiu |
|---|---|
| The State Intellectual Property Office 6, Xitucheng Road, Haidian District, Beijing, 100088, China | Telephone No.86-10-62085331 |
| Facsimile No. | |

Form PCT/ISA /210 (second sheet) (July 2004)